(19) Europäisches Patentamt
European Patent Office
Office européen des brevets

(11) Publication number: **0 038 869**
**A1**

(12) **EUROPEAN PATENT APPLICATION**

(21) Application number: 80102338.3

(22) Date of filing: 30.04.80

(51) Int. Cl.³: C 07 D 487/04
//C07D205/08, C07F7/10,
C07C125/065, (C07D487/04, 209/00,
205/00)

(43) Date of publication of application:
04.11.81 Bulletin 81/44

(84) Designated Contracting States:
AT BE CH DE FR GB IT LI LU NL SE

(71) Applicant: MERCK & CO. INC.
126, East Lincoln Avenue P.O. Box 2000
Rahway, New Jersey 07065(US)

(72) Inventor: Christensen, Burton G.
195 Watchung Terrace
Scotch Plains, New Jersey 07076(US)

(72) Inventor: Ratcliffe, Ronald W.
234 Matawan Avenue
Matawan New Jersey 07747(US)

(74) Representative: Abitz, Walter, Dr.-Ing. et al,
Abitz, Morf, Gritschneder P.O. Box 86 01 09
D-8000 Munich 86(DE)

(54) **Process for the preparation of 1-carbapenems, and intermediates for their preparation.**

(57) Processes and intermediates for the total synthesis of 1-carbapenem antibiotics (I) *via* intermediates (III) and (IV):

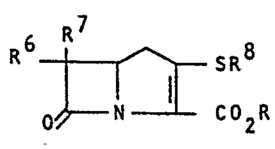

11

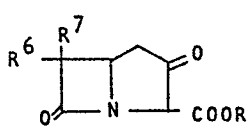

111

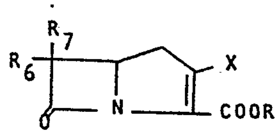

1V

wherein R is hydrogen, a pharmaceutically acceptable ester moiety or salt cation, or a readily removable blocking group; $R^6$, $R^7$ and $R^8$ are, *inter alia*, independently selected from the group consisting of hydrogen, alkyl, alkenyl, aryl and aralkyl, and X is a conventional leaving group.

EP 0 038 869 A1

TITLE MODIFIED
see front page

16101Y



# PROCESS FOR THE PREPARATION OF 1-CARBAPENEMS AND INTERMEDIATES VIA 4-ALLYLAZETIDINONE

## BACKGROUND OF THE INVENTION

This invention relates to the total synthesis of certain 1-carbapenems and their pharmaceutically acceptable salt, ester and amide derivatives which are useful as antibiotics. Such compounds may generically be represented by the following structural formula:

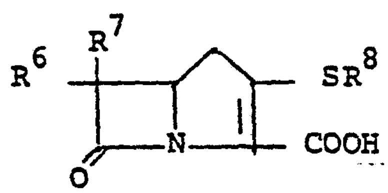

wherein $R^6$, $R^7$, and $R^8$ are independently selected from the group consisting of hydrogen, substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl; wherein the substituent or substituents relative to the above-named radicals are selected from the group consisting of:

$-X^{\circ}$ halo (chloro, bromo, fluoro)

$-OH$ hydroxy

$-OR^1$ alkoxy, aryloxy

$-O\overset{O}{\overset{\|}{C}}NR^1R^2$ carbamoyloxy

$-\overset{O}{\overset{\|}{C}}NR^1R^2$ carbamoyl

$-NR^1R^2$ amino

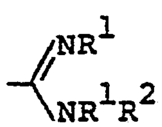 amidino

$-SO_2NR^1R^2$ sulfonamido

$-NH\overset{O}{\overset{\|}{C}}NR^1R^2$ ureido

$R^1\overset{O}{\overset{\|}{C}}NR^2-$ amido

$-CO_2H$ carboxy

$-CO_2R^1$ carboxylate

$-\overset{O}{\overset{\|}{C}}R^1$ acyl

$-O\overset{O}{\overset{\|}{C}}R^1$ acyloxy

-SH mercapto

$-\overset{O}{\overset{\|}{S}}R^1$ alkyl and aryl sulfinyl

$-\underset{\underset{O}{\|}}{\overset{O}{\overset{\|}{S}}}R^1$ alkyl and aryl sulfonyl

-CN cyano

$-N_3$ azido

wherein, relative to the above listed substituents on $R^6$, $R^7$, and $R^8$, the groups $R^1$ and $R^2$ are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; aryl, such as phenyl; aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms.

This invention also relates to the carboxyl derivatives of I which are antibiotics and which may be represented by the following generic structure (I):

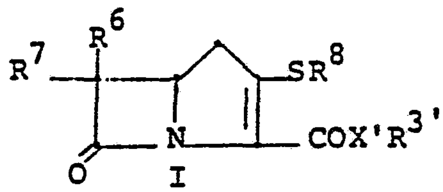

I

wherein X' is oxygen, sulphur or NR' (R' = H or lower alkyl having 1-6 carbon atoms); and $R^{3'}$ is, inter alia, representatively selected from the group consisting of hydrogen, conventional blocking groups such as trialkylsilyl, acyl and the pharmaceutically acceptable salt, ester and amide moieties known in the bicyclic β-lactam antibiotic art; the definition of $R^{3'}$ is given in greater detail below.

Starting from an appropriately substituted 4-allylazetidinone (II), the synthesis proceeds <u>via</u> intermediates III and IV:

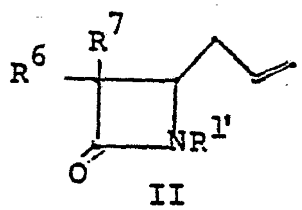

II

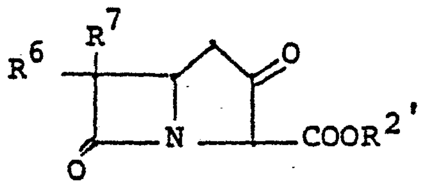

III

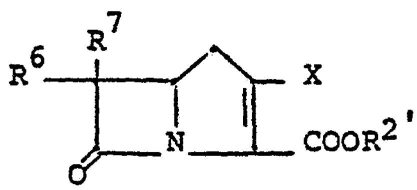

IV

wherein $R^6$ and $R^7$ are as previously defined; X is a conventional leaving group and $R^{2'}$ is hydrogen, a pharmaceutically acceptable ester moiety or a conventional, readily removable protecting group or salt cation. For intermediates III, $R^{2'}$ is as defined but preferably is an ester moiety defined under $R^{2'}$. $R^{1'}$ is hydrogen or a readily removable protecting group such as a triorganosilyl group.

The details of the total synthesis are given below.

The final compounds prepared by the process of this invention are disclosed and claimed in the following co-pending European Patent Application Serial Number 78 101 156 filed October 16, 1978; European Patent Application Serial Number 80 102 076 filed April 18, 1980 and European Patent Application Serial Number 78 101 157 filed October 16, 1978. To the extent that the foregoing Patent Applications describe the antibiotic utility of final compound I and to the extent that they define substituents $R^6$, $R^7$, $R^8$, R', X' and $R^{3'}$ they are hereby incorporated by reference.

Thus, it is an object of the present invention to provide a novel class of antibiotics which are useful in animal and human therapy and in inanimate systems. These antibiotics are active against a broad range of pathogens which representatively include both gram positive bacteria such as S. aureus, Strep. pyogenes, and B. subtilis, and gram negative bacteria such as E. coli, Pseudomonas, Proteus morganii, Serratia, and Klebsiella. Further objects of this invention are to provide chemical processes for the preparation of such antibiotics and their non-toxic pharmaceutically acceptable salts; pharmaceutical compositions comprising such antibiotics; and to provide methods of treatment comprising administering such antibiotics and compositions when an antibiotic effect is indicated.

DETAILED DESCRIPTION OF THE INVENTION

The process of the present invention may conveniently be summarized by the following reaction diagram:

DIAGRAM I

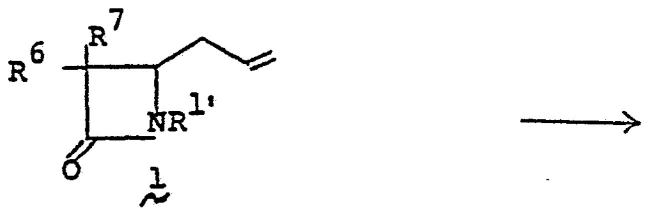

1

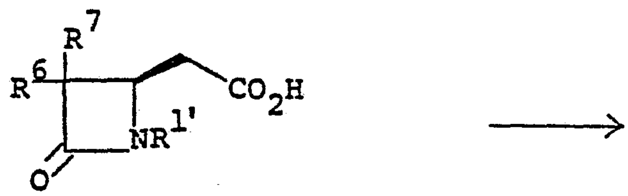

2

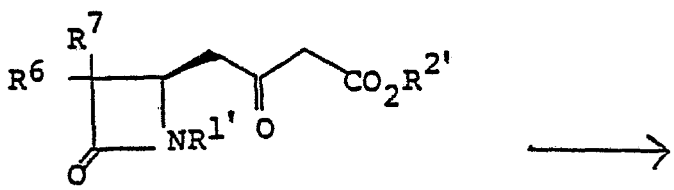

3

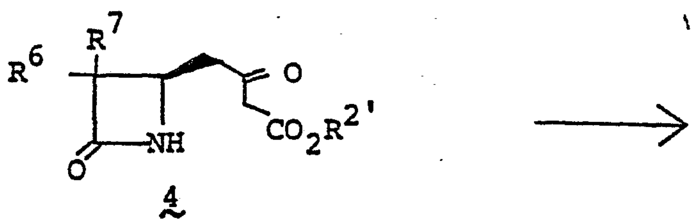

4

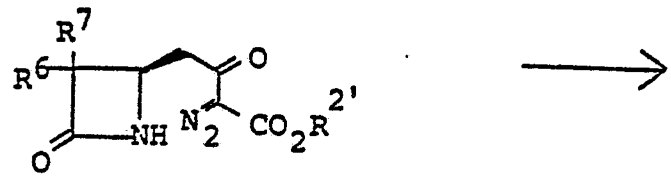

5

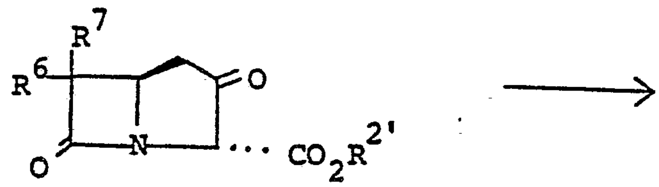

6

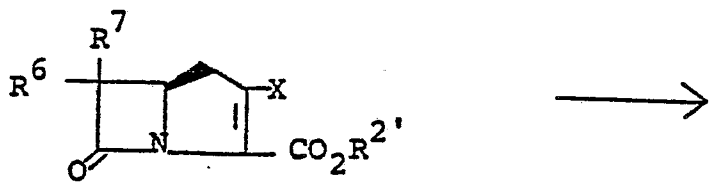

7

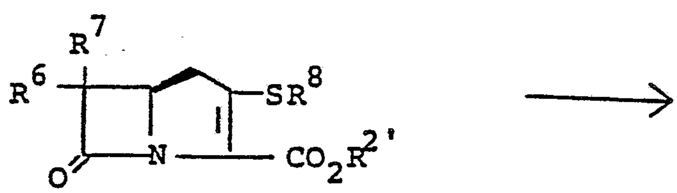

8

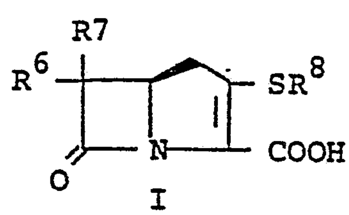

I

In words relative to Diagram I, oxidation of starting material 1 (described below) is accomplished by treating 1 in a solvent such as methylene chloride, methanol, chloroform, dichloroethane, or the like, with an oxidizing agent such as ozone, or the like at a temperature of from -100° to 0°C for from 0.1 to 4 hours, followed by treating the crude product with an oxidizing agent such as m-chloroperbenzoic acid, hydrogen peroxide, peracetic acid, or the like at a temperature of from 0°C to 100°C for from 1 to 100 hours to form 2. Chirality is conveniently introduced at this stage of the synthesis. Resolution of racemic 2 can be accomplished, for example, by fractional crystallization of the carboxylate salt formed with an optically active base such as brucine, N-methylphenethylamine, N,N-dimethylphenethylamine or the like.

The addition 2→3 is accomplished by treating 2 with 1,1'-carbonyldimidazole, or the like, in a solvent such as tetrahydrofuran, dimethoxyethane, DMF, or the like, at a temperature of from 0 to 50°C, followed by the addition of 0.5 to 3.0 equivalents of $(R^{2'}O_2CCH_2CO_2)_2Mg$, at a temperature of from 0 to 50°C for from 1 to 48 hours. $R^{2'}$ is a readily removable carboxyl protecting group such as p-nitrobenzyl, benzyl, or the like. It should also be noted that $R^{2'}$ may be a pharmaceutically acceptable ester moiety; such ester groups are representatively mentioned below. (DMF is dimethylformamide.)

Removal of protecting group $R^{1'}$ (3→4) may be accomplished by a variety of known procedures such as hydrolysis or hydrogenation. When $R^{1'}$ is a triorganosilyl group (for example, $[(CH_3)_3C](CH_3)_2Si-$) removal is typically accomplished by acidic aqueous hydrolysis of 3 in a solvent such as methanol, ethanol,

tetrahydrofuran, dioxane, DMF, or the like in the presence of an acid such as hydrochloric, sulfuric, acetic or the like at a temperature of from 0 to 100°C for from 2 to 18 hours.

It should be noted that an otherwise identical deblocking can occur on 1 or 2. Thus, when $R^{1'}$=H, the chain elongation can proceed directly from 2 to 4.

The diazo species 5 is prepared from 4 by treating 4 in a solvent such as $CH_3CN$, $CH_2Cl_2$, THF, or the like, with an azide such as p-carboxybenzenesulfonylazide, toluenesulfonylazide, methanesulfonylazide, or the like, in the presence of a base such as triethylamine, pyridine, $(C_2H_5)_2NH$, or the like, for from 1 to 50 hours at 0-25°C. (THF is tetrahydrofuran.)

Cyclization (5→6) is accomplished by treating 5 in a solvent such as benzene, toluene, THF, or the like, at a temperature of from 50-110°C for from 1-5 hours in the presence of a catalyst such as bis (acetylacetonato)Cu(II) [Cu(acac)$_2$], $CuSO_4$, Cu powder, $Rh(OAc)_2$, or $Pd(OAC)_2$. Alternatively, the cyclization may be accomplished by irradiating 6 througn a pyrex filter (a wave length greater than 300nm) in a solvent such as benzene, $CCl_4$, diethylether, or the like, at a temperature of from 0-25°C for from 0.5 to 2 hours. ["OAc" = acetate.]

Establishment of leaving group X (6 → 7) is accomplished by acylating the keto ester 6 with an acylating agent R°X such as p-toluenesulfonic acid anhydride, p-nitrophenylsulfonic acid anhydride, 2,4,6-triisopropylphenylsulfonic acid anhydride, methanesulfonic acid anhydride, trifluoromethane sulfonic acid anhydride, diphenyl chlorophosphate, toluenesulfonyl chloride, p-bromophenylsulfonyl chloride, or the like, wherein X is the corresponding leaving group such as toluene sulfonyloxy, p-nitrophenylsulfonyloxy, diphenylphosphoryl, and other leaving groups which are established by conventional procedures and are well known in the art. Typically, the above acylation to establish leaving groups X is conducted in a solvent such as methylene chloride, acetonitrile or dimethylformamide, in the presence of a base such as diisopropylethylamine, triethylamine, 4-dimethylamino-pyridine or the like at a temperature of from -20 to 40°C for from 0.1 to 5 hours. The leaving group X of intermediate 7 can also be halogen. The halogen leaving group is established by treating 7 with a halogenating agent such as $Ø_3PCl_2$, $Ø_3PBr_2$, $(ØO)_3PBr_2$, oxalyl chloride or the like in a solvent such as $CH_2Cl_2$, $CH_3CN$, THF, or the like in the presence of a base such as diisopropylethylamine, triethylamine, or 4-dimethylaminopyridine or the like. [Ø = phenyl.]

The reaction 7 → 8 is accomplished by treating 7 in a solvent such as dioxane, dimethylformamide, dimethylsulfoxide, acetonitrile, hexamethylphosphoramide, or the like in the presence of an approximately equivalent to excess of the mercaptan reagent $HSR^8$, wherein

$R^8$ is as defined above, in the presence of a base such as sodium hydrogen carbonate, potassium carbonate, triethylamine, diisopropylethylamine, or the like at a temperature of from -40 to 25°C for from 1 to 72 hours. When $R^8$ is substituted by a primary or secondary amino group, for example $-CH_2CH_2NH_2$, the mercaptan reagent may be represented as $HSCH_2CH_2NHR°$, for example; wherein R° is a readily removable N-protecting group such as p-nitrobenzyloxycarbonyl, ($-CO_2PNB$), o-nitrobenzyloxycarbonyl, or the like. The specifically illustrated mercaptan reagent, $HSCH_2CH_2NHR°$, is typically prepared by treating aminoethylmercaptan in the presence of the desired acid chloride in the presence of a base such as sodium bicarbonate, sodium hydroxide, or the like in a solvent such as aqueous diethylether, aqueous dioxane, aqueous acetone, or the like at a temperature of from 0 to 25°C for from 0.5 to 4 hours. The foregoing mercaptan reagent, $HSR^8$, and means for its protection, is simply illustrative. The class of suitable $HSR^8$ reagents is representatively described below and in the Examples.

The final deblocking step 8 → I is accomplished by conventional procedures such as solvolysis or hydrogenation. Typically 8 in a solvent such as dioxane-water-ethanol, tetrahydrofuran-aqueous dipotassium hydrogen phosphate-isopropanol or the like is treated under a hydrogen pressure of from 1 to 4 atmospheres in the presence of a hydrogenation catalyst such as palladium on charcoal, palladium hydroxide, platinum oxide, or the like at a temperature of from 0 to 50°C for from 0.25 to 4 hours to provide I. Photolysis, when $R^2$ is a group such as o-nitrobenzyl, for example, may also be used for deblocking.

Introduction of the Thia Side Chain

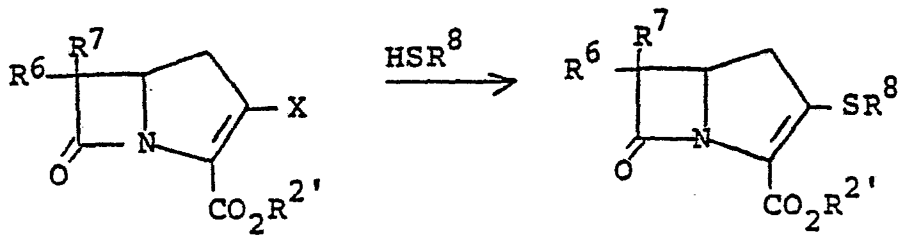

7 8

Relative to the foregoing description of the invention, suitable reagents $HSR^8$ utilized in the transformation 7 → 8 are listed below. The list is arranged according to structural and functional characteristics of the thia side chain $-SR^8$; annotation is provided where necessary. The thia side chain of choice is derived from the corresponding mercaptan reagent $HSR^8$. When the mercaptan contains a functional group which might interfere with the intended course of reaction, the offending group is covered. For example, when a basic nitrogen group is encountered (-NHR or $-NH_2$, for example) it is usually protected by acylation (e.g., $-CO_2PNB$) and when a carboxyl group ($-CO_2H$) is present, it is usually protected by esterification (e.g., PNB ester). Such protection also facilitates in the purification of products 8 by chromatographic means. (PNB is p-nitrobenzyl).

1.) <u>Aliphatic (including carbocyclic) Mercaptans</u>: $HSR^8$ wherein $R^8$ is 1-10 carbon alkyl, cycloalkyl, alkenyl, cycloalkenyl, or alkynyl; $R^8$ may be branched or unbranched,

<u>Examples</u>

$HSCH_3$

$HSCH_2CH_3$

$HSCH_2CH_2CH_3$

$HSCH(CH_3)_2$

$HS(CH_2)_3CH_3$

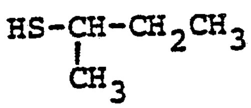

$HSCH_2CH(CH_3)_2$

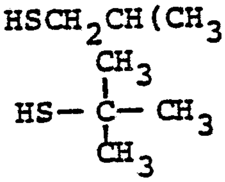

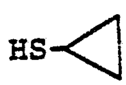

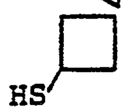

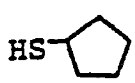

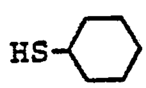

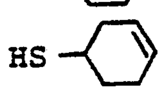

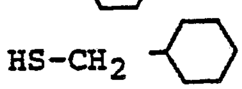

$HS-CH_2-CH=CH_2$

$HS-CH_2-CH=C(CH_3)_2$

$HS-CH_2-C\equiv CH$

$HS-CH_2-C\equiv C-CH_3$

2.) <u>Substituted Aliphatic Mercaptans</u>: $HSR^8$ wherein $R^8$ is a 1-10 carbon branched or unbranched alkyl, cycloalkyl, alkenyl, cycloalkenyl or alkynyl group substituted by one or more halo, OH, $OR^1$, $O\overset{O}{C}R^1$, $O\overset{O}{C}NH_2$, $O\overset{O}{C}NR^1R^2$, $NH_2$, $NHR^1$, $NR^1R^2$, $\overset{O}{C}R^1$, $CO_2H$, $CO_2R^1$, $CONH_2$, $CONHR^1$, $CONR^1R^2$, CN, $SR^1$, $\overset{O}{S}R^1$, $SO_2R^1$, $SO_2NH_2$, $SO_2NHR^1$, $SO_2NR^1R^2$, $NH\overset{O}{C}R^1$, $NH\overset{O}{C}NH_2$, $NH\overset{O}{C}NHR^1$, $NH\overset{O}{C}NR^1R^2$, $NH\overset{O}{C}OR^1$, $\overset{NH}{C}NH_2$, $\overset{NR^1}{C}NHR^2$, wherein $R^1$ and $R^2$ are as previously defined relative to substituents on $R^8$. Preferred substituents are basic nitrogen containing groups.

<u>EXAMPLES</u>

$HS(CH_2)_nOR^1$ $n = 2\text{-}4$, $R^1 = H$, $\overset{O}{C}CH_3$, $CH_3$

$HS(CH_2)_n\overset{O}{C}XR$ $n=1\text{-}3$, X=O, NH, $NR^1$ $R^1$=H, $CH_3$

$HS(CH_2)_nNH_2$ n=2-4

$HS(CH_2)_nNHR^1$ n=2-4, $R^1$=$CH_3$, $CH_2CH_3$, $CH_2CH_2CH_3$, $\overset{O}{C}CH_3$

$HS(CH_2)_nNR^1R^2$ n=2-4, $R^1/R^2$=$CH_3$, $CH_2CH_3$

$HS\text{-}\overset{CH_3}{CH}\text{-}CH_2NH_2$

$HS\text{-}\underset{CH_3}{\overset{CH_3}{C}}\text{-}CH_2NH_2$

$HS\text{-}CH_2\text{-}\overset{CH_3}{CH}\text{-}NH_2$

$HS\text{-}CH_2\text{-}\underset{CH_3}{\overset{CH_3}{C}}\text{-}NH_2$

$HS\text{-}CH_2CH_2SCH_3$

$HS\text{-}CH_2CH_2NHC(CH_3)_3$

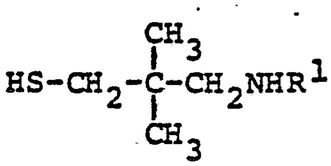 $R^1 = H, CH_3, \overset{O}{\overset{\|}{C}}CH_3$

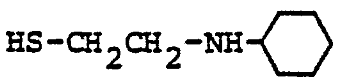

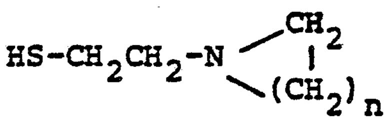 n=3-5

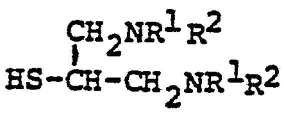 $R^1$=H, $CH_3$ ; $R^2$ = H, $CH_3$

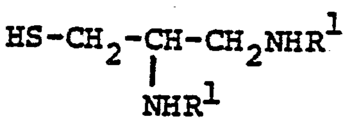 $R^1$=H, $CH_3$

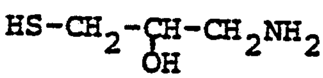

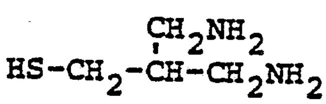

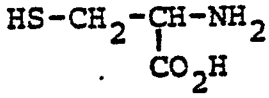

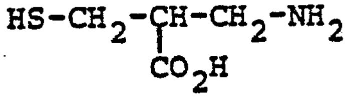

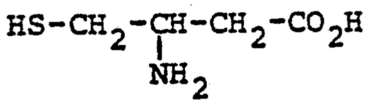

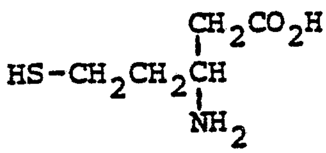

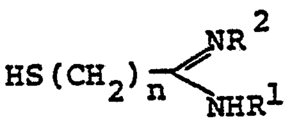 $n=1-3, R^2=H, CH_3, R^1=H, CH_3$

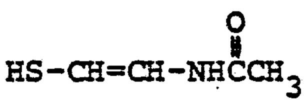

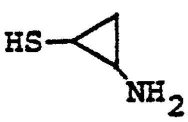

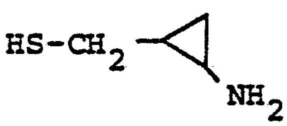

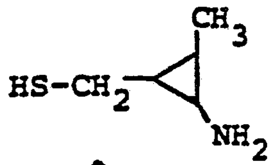

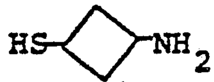

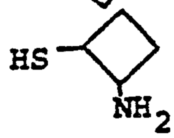

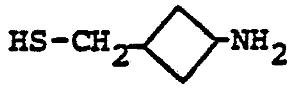

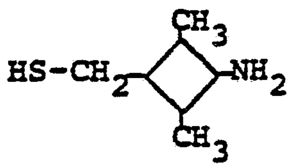

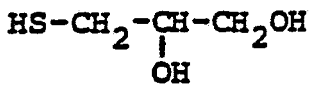

5-thio-D-glucose

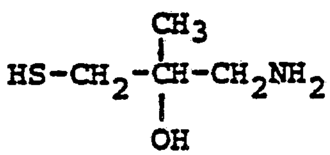

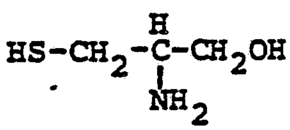

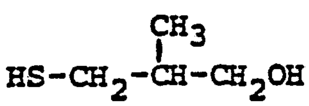

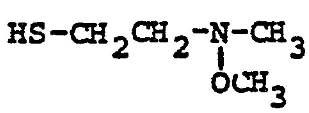

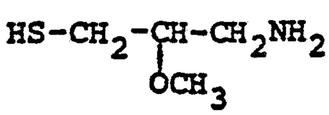

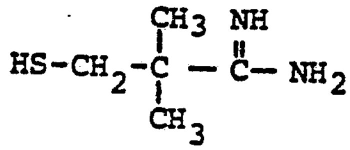

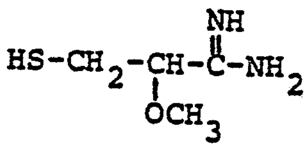

$HS-CH_2-CH=CH-CH_2NH_2$

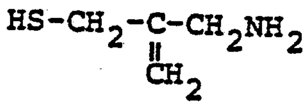

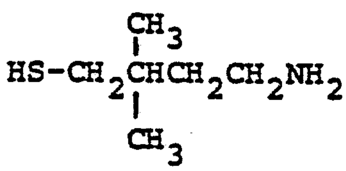

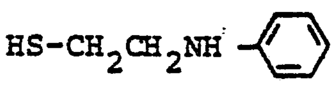

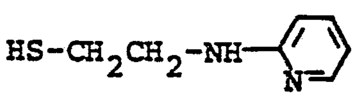

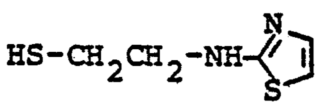

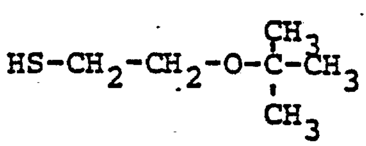

3.) <u>Aryl Mercaptans</u>: $HSR^8$ wherein $R^8$ is phenyl or substituted phenyl. The substituents are independently selected from those previously defined for $R^8$. Especially preferred substituents include alkyl, halo, hydroxy, alkoxy, acyloxy, acyl, carboxy, mercapto, sulfinyl, sulfonyl, amino, substituted amino, aminoalkyl, substituted aminoalkyl, amido, and ureido.

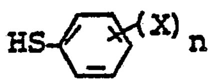

n= 1, 2 or 3,
X= F, Cl, Br, OH, OR, $O\overset{O}{\overset{\|}{C}}R^1$, $NH_2$, $NHR^1$; $NR^1R^2$, $CH_2NH_2$, $CH_2NR^1R^2$, $CO_2H$, $CO_2R^1$, $COR^1$, $CONH_2$, $CONR^1R^2$, $R^1CONH$, $R^1NHCONH$, $SR^1$, $\overset{O}{\overset{\|}{S}}R^1$, $SO_2R^1$, $CH_3$, $CF_3$;
$R^1$ and $R^2$ are as previously defined under $R^8$.

<u>Examples</u>

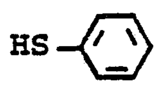

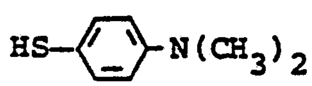

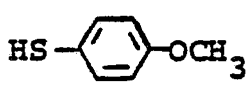

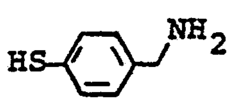

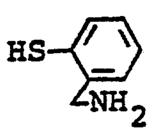

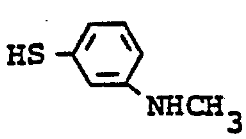

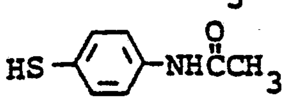

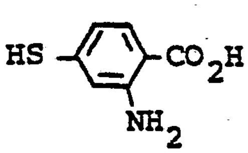

4.) Heteroaryl Mercaptans: $HSR^8$ wherein $R^8$ is a substituted or unsubstituted heteroaryl group containing 1-4 O, N or S atoms. Typical substituents include those mentioned above under "Aryl Mercaptans".

Examples

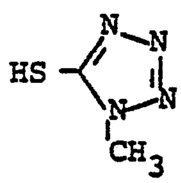

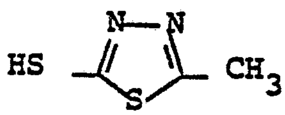

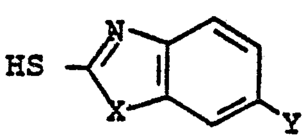

X=N,O Y=H
X=S Y=H, Cl, $OCH_2CH_3$

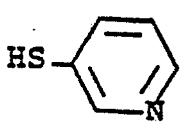

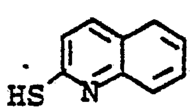

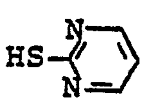

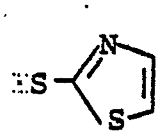

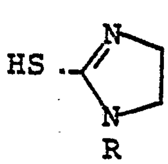

R=H, $CH_3$

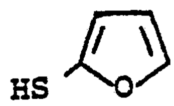

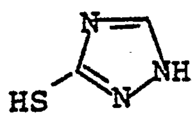

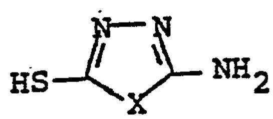

X=NH, S

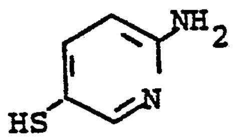

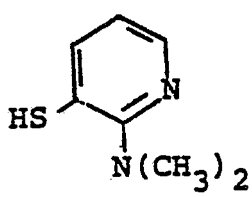

5.) <u>Arylaliphatic Mercaptans:</u> $HSR^8$ where $R^8$ is a 1-6 carbon branched or unbranched alkyl, cycloalkyl, alkenyl, or alkynyl group substituted by a phenyl or substituted phenyl group. Typical phenyl substituents include those mentioned under "Aryl Mercaptans".

<u>Examples</u>

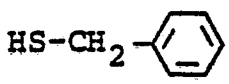

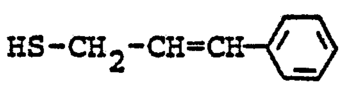

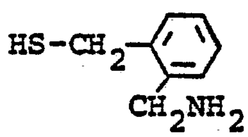

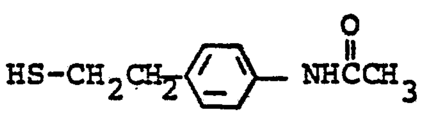

6.) <u>Heteroarylaliphatic and Heterocyclicaliphatic Mercaptans</u>

$HSR^8$ wherein $R^8$ is a 1-6 carbon branched or unbranched alkyl, cycloalkyl, alkenyl, or alkynyl group substituted by a heteroaryl or heterocyclic group containing 1-4, O, N, or S atoms. The heteroaryl or heterocyclic group is unsubstituted or substituted by those substituents mentioned under "Aryl Mercaptans", (No.3, above).

<u>Examples</u>

$HS-(CH_2)_n-$ 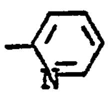

$HS-(CH_2)_n-$ 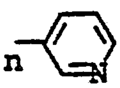

$HS-(CH_2)_n-$ 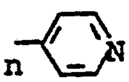 n=1,2

$HS-(CH_2)_n-$ 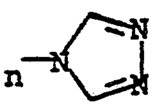

$HS-(CH_2)_n-$ 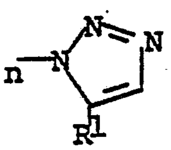 $R^1 = OCH_2CH_3$

$HS-(CH_2)_n-$ 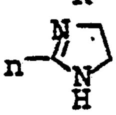

$HS-(CH_2)_n-$ 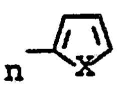

$HS-(CH_2)_n-$ 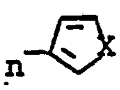 X= O, S, NH

$HS-(CH_2)_n-$ 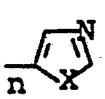 X = O, S, NH

$HS-(CH_2)_n-$ 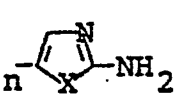 X = O, S, NH

$HS-(CH_2)_n-$ 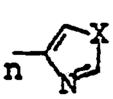 X = O, S, NH

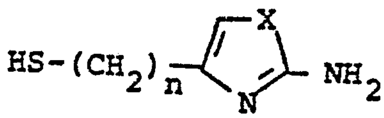

X = O, S, NH

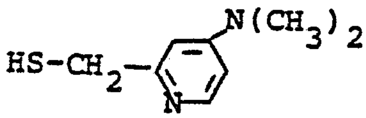

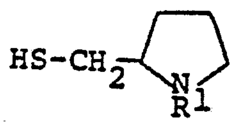

$R^1$ = H, $CH_3$

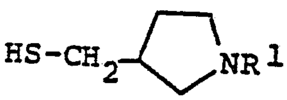

$R^1$=H, $CH_3$

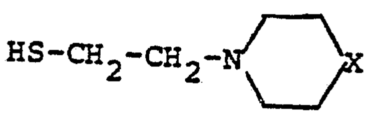

X = O, NH, $NCH_3$

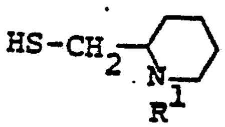

$R^1$ = H, $CH_3$

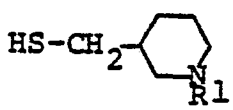

$R^1$ = H, $CH_3$

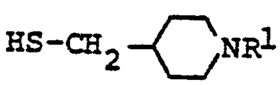

$R^1$=H, $CH_3$

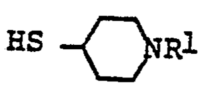

$R^1$ = H, $CH_3$

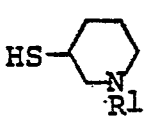

$R^1$=H, $CH_3$

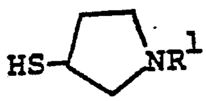

$R^1$=H, $CH_3$

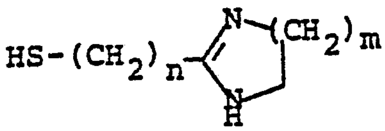

n=1-3, m=1-3

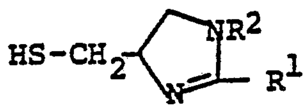

$R^2$=H, $CH_3$, $R^1$=H, $CH_3$, $NH_2$

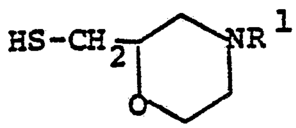

$R^1$=H, $CH_3$.

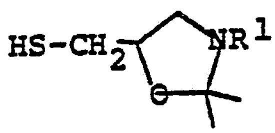

$R^1$=H, $CH_3$

7.) Alkyl-Heteroatom - Alkyl Mercaptans, $HSR^8$

Wherein $R^8$ is

$-(CH_2)_nX(CH_2)_mR^9$

wherein n = 2 to 4, m = 2 to 4; X is NR°, O or S; and wherein R° is H, $CH_3$, $CH_2CH_3$, $CH_2CH_2OH$, or $CH_2CH_2NH_2$ and $R^9$ is OH, $NH_2$, $NHCH_3$, $N(CH_3)_2$, $O\underset{\underset{O}{\|}}{C}CH_3$, $NH\underset{\underset{O}{\|}}{C}CH_3$. Note, in the above representation, the methylene carbons may be branched; for example: 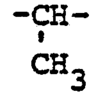 , 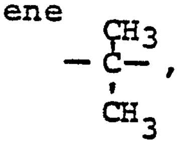 ,

and the like.

The following $HSR^8$ are representative of this class:

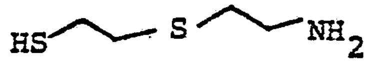

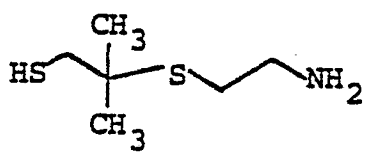

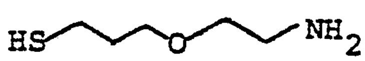

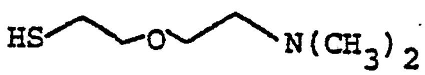

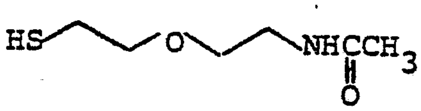

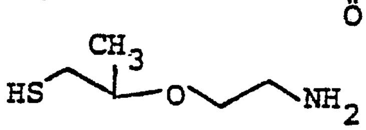

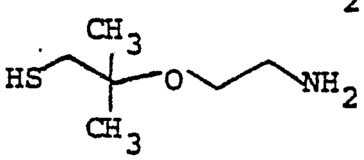

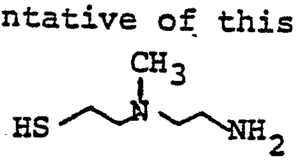

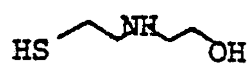

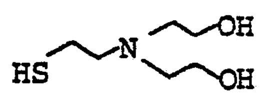

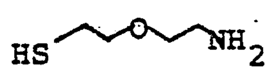

PREPARATION OF THE SUBSTITUTED 4-ALLYLAZETIDINONE, 1

Starting material 1 is conveniently prepared from 4-allylazetidinone according to the following scheme:

SCHEME I

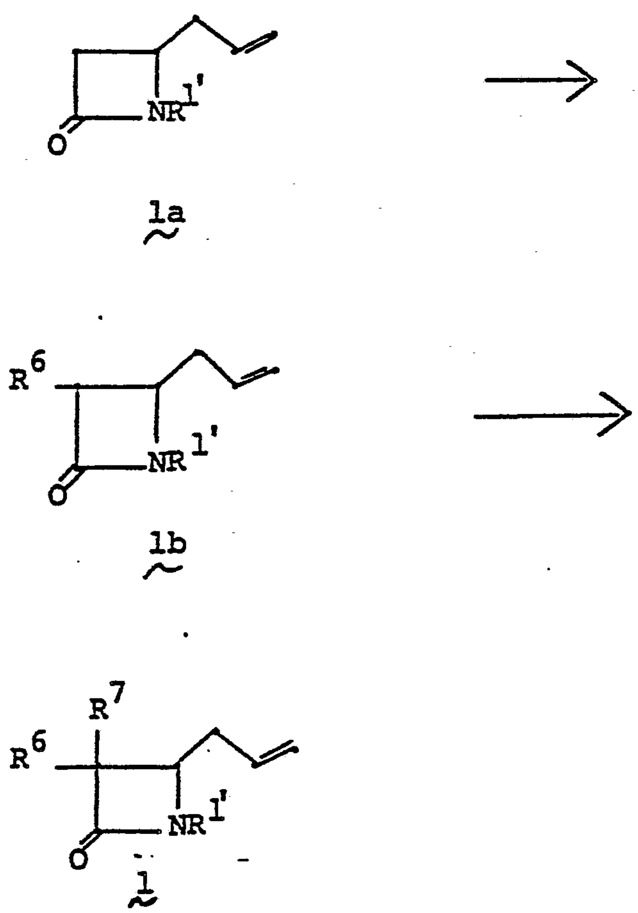

1a

1b

1

It should be noted that starting material 1a is known. Further, in this regard, co-pending, commonly assigned U.S. Patent Application Serial Number 59,842 filed 7/23/79, is incorporated herein by reference for its definition and utilization of starting material 1a. $R^{1'}$ is a removable blocking group such as triorganosilyl, for example, trimethylsilyl, t-butyldimethylsilyl, triphenylsilyl, and the like.

In words relative to the above reaction diagram starting material 1a can be mono-, or dialkylated at ring position 3. Alkylation of 1a provides 1b. Typically, 1a is treated with a strong base such as lithium diisopropylamide, lithium 2,2,6,6-tetramethylpiperidide, potassium hydride, lithium hexamethyldisilazane, phenyllithium or the like in a solvent such as tetrahydrofuran (THF), hexamethylphosphoramide, ether, dimethoxyethane, and the like at a temperature of from -80°C to 0°C whereupon the alkylating agent of choice, $R^6X°$ is added (X° is chloro, iodo or bromo); alternatively the alkylating agent may be $R^6$-tosylate, $R^6$-mesylate or an aldehyde or ketone such as acetaldehyde to provide monoalkylated species 1b. When desired, dialkylated species 1 may be obtained from 1b by repeating the alkylating procedures 1a → 1b. The 6-substituents can also be established by direct acylation using an acylating agent such as N-acyl imidazole or the like. Such N-acyl imidazole acylating reagents are listed below. Also given below is a detailed description of this second approach for establishing, $R^6$ and $R^7$. The following list is representative of useful alkylating agents for establishing $R^6$ and $R^7$, according to the above scheme: 1a → 1b → 1:

Alkylating Agents

$CH_3CHO$

$\not{O}CH_2CHO$ $\not{O}$ = phenyl

$\not{O}CH_2CH_2CHO$

$CH_2O$

$CH_3I$

$\not{O}CH_2Br$

$CH_3COCH_3$

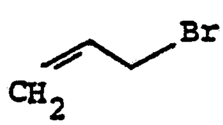

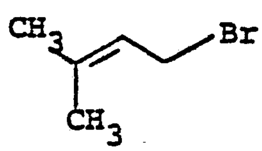

$CH_3OCH_2CHO$

$CH_3CH_2I$

$(CH_3)_2CHI$

$N_3CH_2CHO$

$Me_2NCH_2CHO$

$RO_2CCH_2Br$ R = $CH_3$, benzyl, p-nitrobenzyl

$CF_3CF_2CHO$

$RO_2CCH_2CHO$ R = $CH_3$, benzyl, p-nitrobenzyl

$CH_3CH(CH_3)CHO$,

$CH_3(CH_3)CHCH_2CHO$,

$CH_3CH_2CHO$ ,

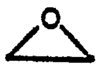 ,

$CF_3CHO$,

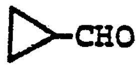

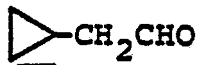

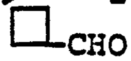

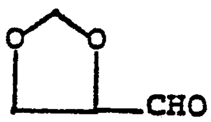

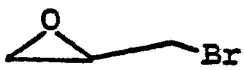

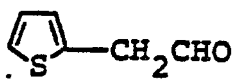 ,

$[(CH_3)_3C](CH_3)_2SiOCH_2\overset{O}{\overset{\|}{C}}H$

$F_2CH\overset{O}{\overset{\|}{C}}H$

$FCH_2\overset{O}{\overset{\|}{C}}H$

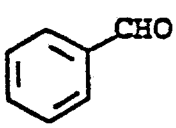

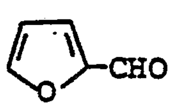

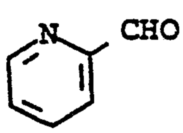

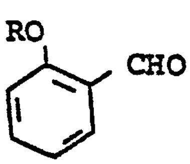

R = protecting group

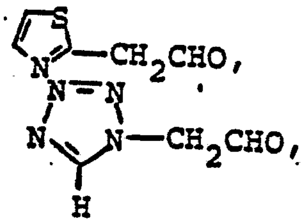

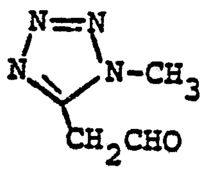
,

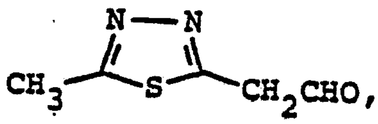

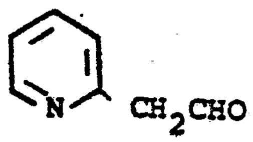

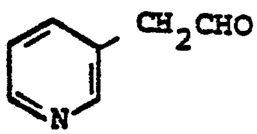

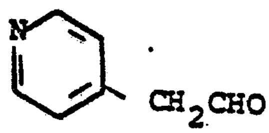

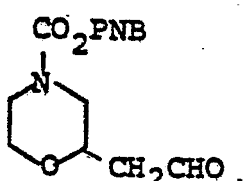

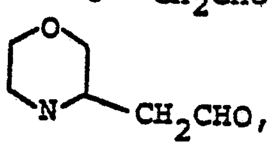

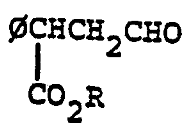

R is removable carboxyl protecting group, such as benzyl.

As mentioned above, the 6-substituents may also be established by acylation. Utilization of such acylating agents may be demonstrated in the following manner with regard to a preferred starting material 1:

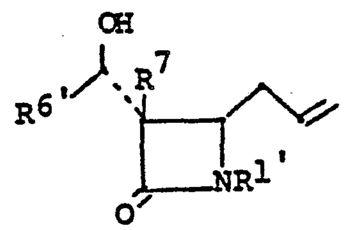

1

wherein $R^7$ and $R^{1'}$ are as defined above. $R^{6'}$ is defined relative to the definition of $R^6$ and in that sense is the balance of the previously identified group $R^6$. In other words, for purposes of this definition $R^{6'}CH(OH)- = R^6$. An especially preferred material 1 is when $R^7$ is hydrogen and $R^{6'}$ is methyl. Such preferred starting materials are described in the following co-pending, commonly assigned U.S. Patent Application Serial Number 59,842 filed July 23, 1979 which is incorporated herein by reference. Basically, such 1'-hydroxy $R^{6'}$ species 1 are prepared according to the following scheme:

SCHEME II

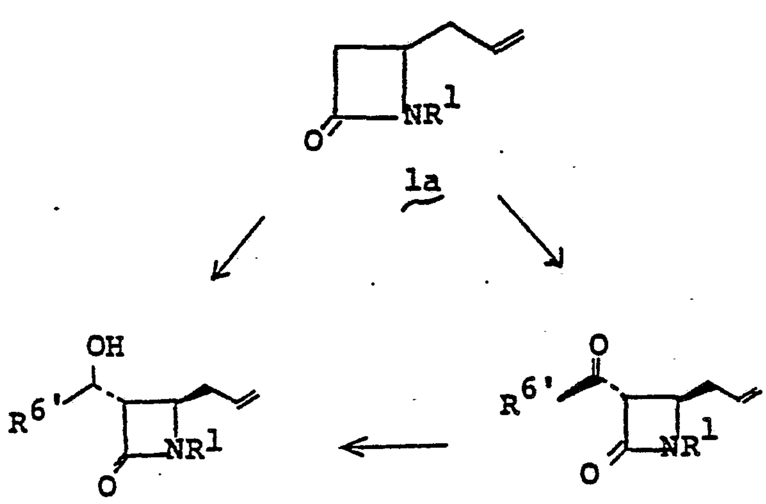

1a

1

1'

The alkylation 1a → 1, Scheme II, is accomplished as previously described, by treating 1a in a solvent such as tetrahydrofuran, dimethoxyethane, diethylether, hexamethylphosphoramide, at a temperature of from -100° to -20°C with a strong base such as lithium diisopropylamide, lithium hexamethyldisilazide, lithium 2,2,6,6-tetramethylpiperidide, potassium hydride or the like followed by the addition of an equivalent to 10 fold excess of an aldehyde. This reaction gives a mixture of isomers from which the desired trans-R form 1 can be conveniently separated by chromatography or crystallization.

Intermediate 1a may proceed directly to 1 as indicated above, or it may take the circuitous path via 1'. The direct acylation, to 1' is accomplished by treating 1a with two or more equivalents of a base such as lithium diisopropylamide, lithium hexamethyldisilazide, lithium 2,2,6,6-tetramethylpiperidide, in a solvent such as tetrahydrofuran, diethylether, or dimethoxyethane, for example, at a temperature of from -100 to -20°C with an acylating agent such as N-acyl imidazole or the like. Addition of the 1a plus base mixture to the acylating agent is preferred.

Representative acylating agents for this scheme 1a → 1' → 1 are listed below.

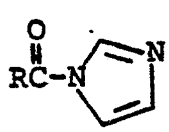 ; R=$CH_3$, $ClCH_2$, $CH_3CH_2$, $N_3CH_2$, $CH_3OCH_2$,

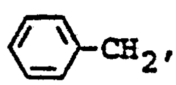, 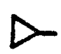, 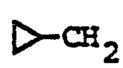, 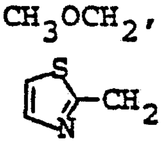,

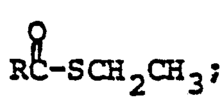; R=$CF_3$, $CF_2H$, $CH_2$=CH, 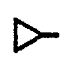,

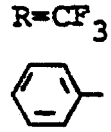, 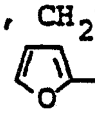

Further with respect to Scheme II, the reduction, 1' → 1 is accomplished by contacting the ketone with a reducing agent such as potassium tri(sec-butyl)borohydride, lithium tri(sec-butyl)borohydride, sodium borohydride, sodium tris(methoxyethoxy)aluminum hydride, lithium aluminum hydride or the like in a solvent such as diethylether, tetrahydrofuran, toluene or the like at a temperature of from -78° to 25°C. The reaction can conveniently be conducted in the presence of an added complexing salt such as potassium iodide, magnesium bromide or the like.

In a similar manner, unresolved 1 (cis and trans) may be oxidized to 1' for reduction to 1 as indicated above:

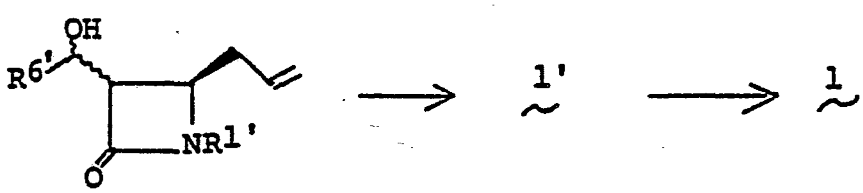

The oxidation is accomplished with an oxidizing agent such as dipyridine chromium (VI) oxide, trifluoroacetic anhydride-dimethylsulfoxide-triethylamine, pyridinium dichromate, acetic anhydride-dimethylsulfoxide in a solvent such as methylene chloride, acetonitrile, or the like at a temperature of from -78 to 25°C for from 5 minutes to 5 hours.

As noted above, the compounds of the present invention may also generally be represented by the following structural formula:

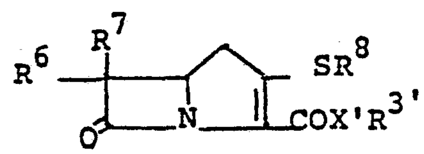

I

wherein X' is oxygen, sulfur or NR' (R' is hydrogen or loweralkyl having from 1 to 6 carbon atoms); and $R^{3'}$ is hydrogen, or, <u>inter alia</u>, is representatively selected to provide the pharmaceutically acceptable salt, ester anhydride ($R^{3'}$ is acyl) and amide moieties known in the bicyclic β-lactam antibiotic art; $R^{3'}$ may also be a readily removable blocking group.

<u>Identification of the Radical $-COX'R^{3'}$</u>

In the generic representation of the compounds of the present invention (I, above), the radical represented by $-COX'R^{3'}$ is, <u>inter alia</u>, -COOH (X' is oxygen and $R^{3'}$ is hydrogen) and all radicals known to be effective as pharmaceutically acceptable ester, anhydride ($R^{3'}$ is acyl) and amide radicals in the bicyclic β-lactam antibiotic art, such as the cephalosporins and penicillins and nuclear analogues thereof.

Suitable blocking esters ($R^{3'}$, X' = O) include those selected from the following list which is representative:

(i) $R^{3'} = CR^aR^bR^c$ wherein at least one of $R^a$, $R^b$ and $R^c$ is an electron-donor, e.g., p-methoxyphenyl. The remaining $R^a$, $R^b$ and $R^c$ groups may be hydrogen or organic substituting groups. Suitable ester groups of this type include p-methoxybenzyloyxcarbonyl.

(ii) $R^{3'} = CR^aR^bR^c$ wherein at least one of $R^a$, $R^b$ and $R^c$ is an electron-attracting group, e.g., p-nitrophenyl, trichloromethyl, and o-nitrophenyl. Suitable esters of this type include p-nitrobenzyloxycarbonyl, and 2,2,2-trichloroethoxycarbonyl.

(iii) $R^{3'} = CR^aR^bR^c$ wherein at least two of $R^a$, $R^b$ and $R^c$ are hydrocarbon such as alkyl, e.g., methyl or ethyl, or aryl, e.g., phenyl and the remaining $R^a$, $R^b$ and $R^c$ group, if there is one, is hydrogen. Suitable esters of this type include t-butyloxycarbonyl, diphenylmethoxycarbonyl and triphenylmethoxycarbonyl.

Silyl esters, under this category of blocking groups, may conveniently be prepared from a halosilane of the formula: $R^4{}_3SiX'$
wherein X' is a halogen such as chloro or bromo and $R^4$ is alkyl, e.g., methyl, ethyl, t-butyl.

Pharmaceutically acceptable carboxyl derivatives of the present invention are those derived by reacting I with alcohols, acylating reagents

and the like. For example, esters and amides of interest are the above-listed starting materials and final products having the $-COX'R^{3'}$ group at the 3-position; wherein X' is oxygen, sulfur or NR' (R' is H or $R^{3'}$), and $R^{3'}$ is alkyl having 1-6 carbon atoms, straight or branched, such as methyl, ethyl, t-butyl, and the like; carbonylmethyl, including phenacyl; aminoalkyl including 2-methylaminoethyl, 2-diethylaminoethyl; alkanoyloxyalkyl wherein the alkanoyloxy portion is straight or branched and has 1-6 carbon atoms and the alkylportion has 1-6 carbon atoms, such as pivaloyloxymethyl; haloalkyl wherein halo is chloro, and the alkyl portion is straight or branched having 1-6 carbon atoms, e.g., 2,2,2-trichloroethyl; alkenyl having 1-4 carbon atoms such, as 2-propenyl, 3-butenyl, and 4-butenyl; aralkyl and lower alkoxyl- and nitro- substituted aralkyl such as benzyl, benzhydryl, o-nitrobenzyl, p-methoxybenzyl, and p-nitrobenzyl; phthalidyl; benzyloxyalkyl having 8-10 carbon atoms such as benzyloxymethyl, and (4-nitro) benzyloxymethyl.

In addition to the esters (and thio esters) listed above, amides are also embraced by the present invention, i.e., wherein X' is the $-\overset{R'}{N}-$ group. Representative of such amides are those wherein R' is selected from the group consisting of hydrogen and lower alkyl such as methyl and ethyl.

The most preferred $-COX'R^{3'}$ radicals of the present invention are those wherein (relative to Structure I above), X' is oxygen and $R^{3'}$ is hydrogen; loweralkyl having 1-4 carbon atoms; lower alkenyl such as 3-methylbutenyl, 4-butenyl and the like; benzyl and substituted benzyl such as p-nitrobenzyl; pivaloyloxymethyl, 3-phthalidyl; and phenacyl.

The compounds of the present invention (I) are valuable antibiotics active against various gram-positive and gram-negative bacteria and accordingly find utility in human and veterinary medicine. Representative pathogens which are sensitive to antibiotics I include: Staphyloccus aureus, Escherichia coli, Klebsiella pneumoniae, Bacillus subtilis, Salmonella typhosa, Psuedomonas and Bacterium proteus. The antibacterials of the invention are not limited to utility as medicaments; they may be used in all manner of industry, for example: additives to animal feed, preservation of food, disinfectants, and in other industrial systems where control of bacterial growth is desired. For example, they may be employed in aqueous compositions in concentrations ranging from 0.1 to 100 parts of antibiotic per million parts of solution in order to destroy and inhibit the growth of harmful bacteria on medical and dental equipment and as bactericides in industrial applications, for example in waterbased paints and in the white water of paper mills to inhibit the growth of harmful bacteria.

The products of this invention may be used in any of a variety of pharmaceutical preparations. They may be employed in capsule, powder form, in liquid solution, or in suspension. They may be administered by a variety of means; those of principal interest include: orally, topically or parenterally by injection (intravenously or intramuscularly).

Such tablets and capsules, designed for oral administration, may be in unit dosage form, and may contain conventional excipients, such as binding agents, for example, syrup, acacia, gelatin, sorbitol, tragacanth, or polyvinylpyrrolidone; fillers, for example, lactose, sugar, cornstarch, calcium phosphate, sorbitol, or glycerine; lubricants, for example, magnesium stearate, talc, polyethylene glycol, silica; disintegrants, for example, potato starch; or acceptable wetting agents such as sodium lauryl sulphate. The tablets may be coated according to methods well known in the art. Oral liquid preparations may be in the form of aqueous or oily suspensions, or solutions, or they may be presented as a dry product for reconstitution with water or other suitable vehicle before use. Such liquid preparations may contain conventional additives such as suspending agents, for example, sorbitol, methyl cellulose, glucose/sugar syrup, gelatin, hydroxyethylcellulose, or carboxymethyl cellulose. Suppositories will contain conventional suppository bases, such as cocoa butter or other glycerides.

Compositions for injection, the preferred route of delivery, may be prepared in unit dosage form in ampules, or in multidose containers. The compositions may take such forms as suspensions, solutions, or emulsions in oily or aqueous vehicles, and may contain formulatory agents such as suspending, stabilizing and/or dispersing agents. Alternatively, the active ingredient may be in powder form for reconstitution, at the time of delivery, with a suitable vehicle, such as sterile water.

The compositions may also be prepared in suitable forms for absorption through the mucous membranes of the nose and throat or bronchial tissues and may conveniently take the form of liquid sprays or inhalants, lozenges, or throat paints. For medication of the eyes or ears, the preparation may be presented in liquid or semi-solid form. Topical applications may be formulated in hydrophobic or hydrophilic bases as ointments, creams, lotions, paints, or powders.

The dosage to be administered depends to a large extent upon the condition and size of the subject being treated as well as the route and frequency of administration -- the parenteral route by injection being preferred for generalized infections. Such matters, however, are left to the routine discretion of the therapist according to principles of treatment well known in the antibiotic art. In general, a daily dosage consists of from about 5 to about 600 mg of active ingredient per kg. of body weight of the subject in one or more treatments per day. A preferred daily dosage for adult humans lies in the range of from about 10 to 240 mg. of active ingredient per kg. of body weight. Another factor influencing the precise dosage regimen, apart from the nature of the infection and peculiar identity of the individual being treated, is the molecular weight of the chosen species of this invention (I).

The compositions for human delivery per unit dosage, whether liquid or solid, may contain from 0.1% to 99% of active material, the preferred range being from about 10-60%. The composition will generally contain from about 15 mg. to about 1500 mg. of the active ingredient; however, in general, it is preferable to employ a dosage amount in the range of from about 250 mg to 1000 mg. In parenteral administration, the unit dosage is usually the pure compound I in sterile water solution or in the form of a soluble powder intended for solution. For zwitterionic species described under Structure I, the pH of such solutions typically will correspond to the zwitterionic point; however, consideration of individual properties of solubility and stability may require such aqueous solutions to have a pH other than that of the zwitterionic point, for example in the range of 5.5 to 8.2.

In the foregoing word description of the above schematic reaction diagram for the total synthesis of the defined carbapenem antibiotics, it is to be understood that there is considerable latitude in selection of precise reaction parameters. Suggestion of this latitude and its breadth is generally indicated by the enumeration of equivalent solvent systems, temperature ranges, protecting groups, and range of identities of involved reagents. Further, it is to be understood that the presentation of the synthetic scheme as comprising distinct steps in a given sequence is more in the nature of a descriptive convenience than as a necessary requirement; for one will recognize that the mechanically dissected scheme represents a unified scheme of synthesis and that certain steps, in actual practice, are capable of being merged, conducted simultaneously, or effected in a reverse sequence without materially altering the progress of synthesis.

The following examples recite a precise scheme of total synthesis. It is to be understood that the purpose of this recitation is to further illustrate the total synthesis and not to impose any limitation. Temperature is given in °C.

EXAMPLE 1

Preparation of 1-(t-Butyldimethylsilyl)-4-(prop-2-ene)-azetidin-2-one

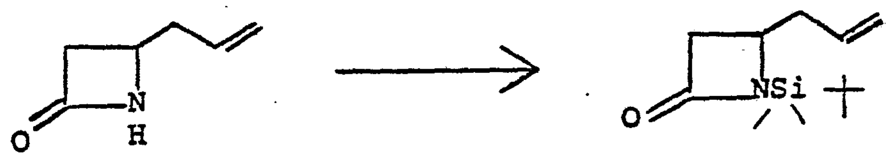

t-Butyldimethylchlorosilane (7.51 g, 49.8 mmol) is added in one portion to an ice-cold, stirring solution of 4-(prop-2-ene)-azetidin-2-one (5.26 g, 47.4 mmol) and triethylamine (5.04 g 49.8 mmol) in anhydrous dimethylformamide (100 ml). A voluminous white precipitate forms almost immediately. The reaction mixture is stirred at 0-5° for 1 hour and then allowed to warm to room temperature. Most of the solvent is removed under vacuum to give a residue which is partitioned between diethyl ether (250 ml) and water. The ethereal phase is washed with 2.5N hydrochloric acid (50 ml), water (3x50 ml), and brine, dried with magnesium sulfate, filtered and evaporated under vacuum to provide an oil which is purified either by vacuum distillation or chromatography on silica gel (20% ether in petroleum ether) to yield 1-(t-Butyldimethylsilyl-4-(prop-2-ene)-azetidin-2-one. n.m.r. ($CnCl_3$) $\delta$ 4.8-6.0(3H, m, olefinic), $\delta$ 3.5(1H, m, H4), $\delta$ 3.03(1H,dd,J=15,5.2H3x), $\delta$ 2.56(1H,dd,J=15, 2.8,H2β), $\delta$ 1.8-2.8(2H, m, allylic), $\delta$ 0.9(9H,S), $\delta$ 0.2(6H,S).

EXAMPLE 2

1-(t-Butyldimethylsilyl)-3-(1-hydroxyethyl)-4-(prop-2-ene)-azetidin-2-one

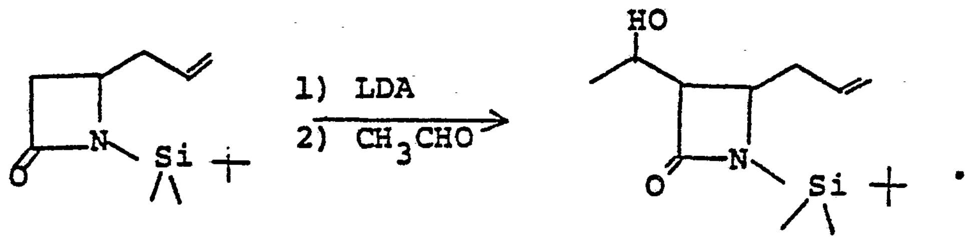


n-Butyllithium in hexane (26.25 mmol) is added slowly by syringe to a solution of diisopropylamine (26.25 mmol) in anhydrous tetrahydrofuran (100 ml) at -78°C. The resulting solution is stirred for 15 min prior to the addition of a solution of 1-(t-butyldimethylsilyl)-4-(prop-2-ene)-azetidin-2-one (25.0 mmol) in anhydrous tetrahydrofuran (25 ml). After stirring for 15 min at -78°C, acetaldehyde (75 mmol) is added by syringe and the resulting solution is stirred at -78°C for 5 min. Saturated aqueous ammonium chloride solution (15 ml) is added by syringe and the reaction mixture is allowed to warm to room temperature, then diluted with ether (250 ml) and washed with 2.5N hydrochloric acid solution (2 x 50 ml), water (100 ml) and brine and dried over magnesium sulfate. Solvents are removed *in vacuo* and the semi-solid residue is chromatographed on silica gel (1:1, ether:petroleum ether). The first product to elute is the cis R* compound (688 mg) n.m.r. ($CDCl_3+D_2O$) $\delta$ 4.8 - 6.2 (3H, m, olefinic), $\delta$ 4.2 (1H, dq, J= 6.5, 3.7, H-8), $\delta$ 3.75 (1H, ddd, J=5.5, 5, 4.8, H-5) $\delta$ 3.28 (H, dd, J=5.5, 3.7, H-6), $\delta$ 2.2-3.0 (2H, m, allyl), $\delta$ 1.35 (3H, d, J=6.5, $CH_3$-CHOH), $\delta$ 1.0 (9H, s, +Si-), $\delta$ 0.3 (6H, s, $(CH_3)_2Si$). The second fraction is a mixture of the trans R* and S* products (5.56 g). Crystallization of this material from petroleum ether gives the pure trans R* material, m.p. 81-82°C.

IR ($CHCl_3$) 3400, 2920, 2850, 1723 $cm^{-1}$; n.m.r. ($CDCl_3$ + $D_2O$) $\delta$ 4.9-6.2 (3H, m, olefinic), $\delta$ 4.1 (1H, dq, J= 7.0, 6.8, H8), $\delta$ 3.66 (1H, ddd, J= 11, 4.5, 3.0, H5), $\delta$ 2.9 (1H, dd, J=6.8, 3.0, H6) $\delta$ 1.8-2.8 (2H, m, allyl ), $\delta$ 1.26 (3H, d, J=7.0, $C\underline{H}_3$-), $\delta$ 1.0 (9H, S, $\underline{+}$Si), $\delta$ 0.28 (6H, 2S, $(C\underline{H}_3)_2Si$).

EXAMPLE 3

(3S*, 4R*)-1-(t-Butyldimethylsilyl)-3-(1-oxoethyl)-4-(prop-2-ene)-azetidin-2-one

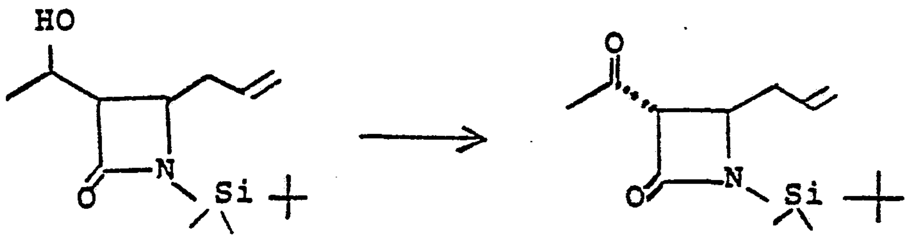

A. Trifluoroacetic anhydride (7.5 mmol) is added dropwise by syringe to a solution of dimethylsulfoxide (10 mmol) in anhydrous methylene chloride (15 ml) at -78°C. The resulting mixture is stirred at -78°C for 20 min. during which time a white precipitate forms. A solution of 1-(t-butyldimethylsilyl)-3-(1-hydroxyethyl)-4-(prop-2-ene)-azetidin-2-one (5.0 mmol) in methylene chloride (15 ml) is added by syringe and the resulting solution is stirred at -78°C for 30 min. Triethylamine (14 mmol) is added by syringe and the cooling bath is removed. After an additional 1 hr., the reaction mixture is diluted with methylene chloride (100 ml), washed with water (50 ml) and brine and dried over magnesium sulfate. Removal of solvents *in vacuo* yields an oil which is chromatographed on silica gel (2:1, petroleum ehter:ether) to yield (3S*,4R*)-1-(t-butyldimethylsilyl-3-(1-oxoethyl)-4-(prop-2-ene)-azetidin-2-one. I.R. ($CHCl_3$) 2925, 2855, 1734, 1705 $cm^{-1}$; n.m.r. ($CDCl_3$) δ 4.8-6.1 (3H, m, olefinic) δ 3.8-4.2 (2H, overlapping multiplets, H5, H6) δ 2.0-2.9 (2H, m, allylic), δ 2.3 (3H, S, $C\underline{H}_3$-C(=O)) δ 0.96 (9H, S, $\underline{+}$Si-), δ 0.25 (6H, 2S, $(C\underline{H}_3)_2Si$). Mass spectrum m/e 267(m+)252,226,210.

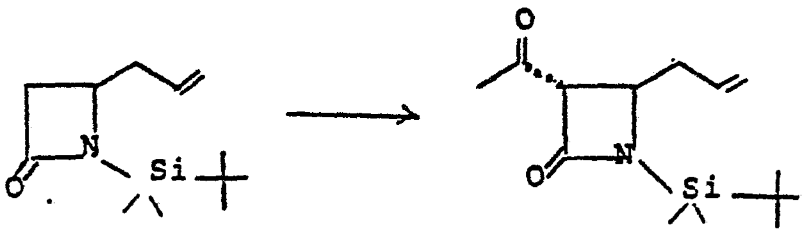

B. n-Butyllithium in hexane (4.10 mmol) is added by syringe to a solution of diisopropylamine (4.10 mmol) in anhydrous tetrahydrofuran (16 ml) at -78°C. The resulting solution is stirred at -78°C for 15 min. prior to the addition of a solution of 1-(t-butyldimethylsilyl)-4-(prop-2-ene)-azetidin-2-one (2.0 mmol) in anhydrous tetrahydrofuran (2 ml). After an additional 15 min. at -78°C, the reaction mixture is added via a Teflon tube to a mixture of N-acetylimidazole (4.1 mmol) in anhydrous tetrahydrofuran (16 ml) at -78°C. The resulting yellow reaction mixture is stirred at -78°C for 15 min, then quenched by addition of saturated aqueous ammonium chloride solution (10 ml). The reaction mixture is diluted with ether (100 ml) and washed with 2.5N hydrochloric acid solution (25 ml) water (25 ml) and brine. The organic phase is dried over magnesium sulfate and concentrated in vacuo to yield an oil. This material is chromatographed on silica gel (2:1 petroleum ether:ether) to yield (3S*,4R*)-1-(t-butyldimethylsilyl)-3-(1-oxoethyl)-4-(prop-2-ene)-azetidin-2-one.

EXAMPLE 4

(3S*, 4R*)-1-(t-Butyldimethylsilyl)-3-[(R*)-1-hydroxyethyl]-4-(prop-2-ene)-azetidin-2-one

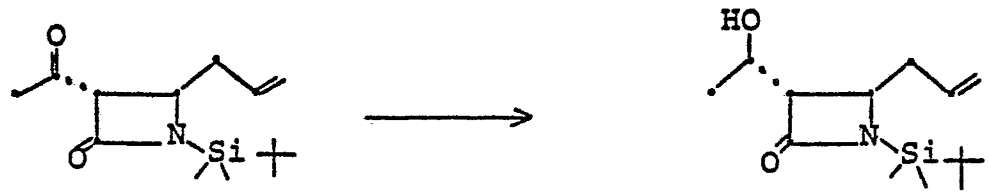

K-Selectride (potassium tri-(sec)-butylborohydride) in tetrahydrofuran (4.8 mmol) is added by syringe to a mixture of potassium iodide (2.0 mmol) and (3S*, 4R*)-1-(t-butyldimethylsilyl)-3-(1-oxoethyl)-4-(prop-2-ene)-azetidin-2-one (2.0 mmol) in anhydrous ether (20 ml) at room temperature. The resulting mixture is stirred at room temperature for 2.5 hours, then quenched by addition of glacial acetic acid (9.6 mmol). The resulting mixture is diluted with ethylacetate (100 ml) and filtered through celite. Removal of solvents *in vacuo* gives an oil which is chromatographed on silica gel (1:1 ether:petroleum ether) to yield 1.90 g (95%) of (3S*, 4R*)-1-(t-Butyldimethylsilyl)-3-(1-hydroxyethyl)-4-(prop-2-ene)-azetidin-2-one as a white solid. NMR examination of this material indicates the R*/S* ratio at the hydroxy center to be >5/1. The R* isomer is isolated by crystallization from petroleum ether.

EXAMPLE 5

(3S*, 4R*)-1-(t-Butyldimethylsilyl)-3-[(R*)-1-hydroxyethyl]-4-(carboxymethyl)-azetidin-2-one

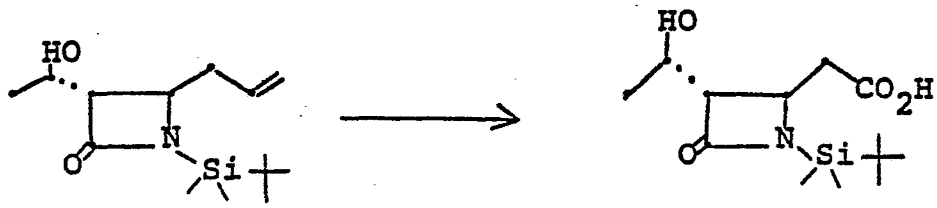

A solution of (3S*, 4R*)-1-(t-Butyldimethylsilyl)-3-[(R*)-1-hydroxyethyl]-4-(prop-2-ene)-azetidin-2-one (3.0 mmol) in dry methylene chloride (30 ml) is cooled to -78°C (dry ice-acetone) and a stream of ozone is bubbled through until the reaction mixture becomes blue. The ozone flow is then stopped and the reaction is purged by bubbling through nitrogen until the blue color disappears. Solid m-chloroperbenzoic acid (3.0 mmol) is added and the cold bath is removed. When the reaction mixture reaches room temperature, the flask is fitted with a reflux condenser and the mixture is heated at reflux for three days. Removal of solvents *in vacuo* gives a white solid which is chromatographed on silica gel (2% glacial acetic acid in methylene chloride) to yield 663 mg (77%) of (3S*, 4R*)-1-(t-Butyldimethylsilyl-3-[(R*)-1-hydroxyethyl]-4-(carboxymethyl)-azetidin-2-one. n.m.r. ($CDCl_3$ + $D_2O$) $\delta$ 3.6-4.3 (2H, m), $\delta$ 2.98 (1H, dd, J=7, 2.1), $\delta$ 2.7 (2H, d of ABq, $-CH_2CO_2H$), $\delta$ 1.29 (3H, d, J=6), $\delta$ 0.95 (9H, S), $\delta$ 0.25 (6H, S).

## EXAMPLE 6

**(3S*, 4R*)-1-(t-Butyldimethylsilyl)-3-[(R*)-1-hydroxyethyl]-4-(3-p-nitrobenzyloxycarbonyl-2-oxopropyl)-azetidin-2-one**

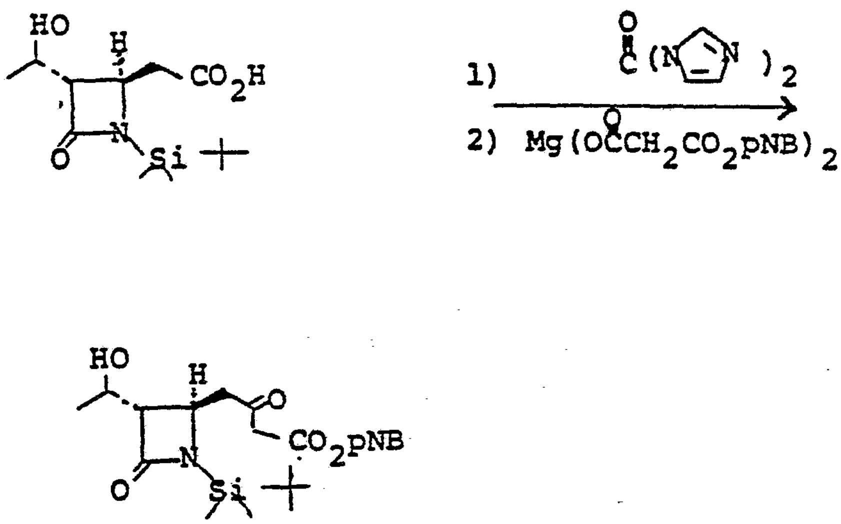


1,1'-Carbonyldimidazole (1.10 mmol) is added in one portion to a solution of (3S*, 4R*)-1-(t-butyldimethylsilyl-3-[(R*)-1-hydroxyethyl]-4-carboxymethyl-azetidin-2-one (1.0 mmol) in anhydrous tetrahydrofuran (5 ml) at room temperature. The resulting solution is stirred at room temperature for 6 hours. In a second flask, magnesium ethoxide (5 mmol) is added in one portion to a solution of the mono-p-nitrobenzyl ester of malonic acid (10 mmol) in anhydrous tetrahydrofuran (25 ml). The resulting mixture is stirred at room temperature for 1 hr, then the tetrahydrofuran is removed at the pump and the gummy residue is triturated with ether to yield the magnesium salt as an off-white solid. (1.1 mmol) of this magnesium salt is then added to the first reaction flask and the resulting mixture is stirred at room temperature for 18 hrs. The reaction mixture is then poured into 50 ml of ether, washed with 0.5N hydrochloric

acid solution (20 ml), water (20 ml), saturated aqueous sodium bicarbonate solution (20 ml), brine and dried over magnesium sulfate. Removal of solvents *in vacuo* gives an oil which is chromatographed on silica gel (ether) to yield (3S*,4R*)-1-(t-butyldimethylsilyl)-3-[(R*)-1-hydroxyethyl]-4-(3-p-nitrobenzyloxycarbonyl-2-oxopropyl)-azetidin-2-one. n.m.r. ($CDCl_3$ - $H_2O$) $\delta$ 8.24, 8.10, 7.52, 7.38(2H, AB, aromatic), $\delta$ 5.26 (2H, S, $-CH_2-Ar$), $\delta$ 3.5-4.2 (2H, m, H-5, H-8), $\delta$ 2.6-3.3 (3H, m, H-6, $-CH_2-\overset{O}{\overset{\|}{C}}-$), $\delta$ 1.3 (3H, d, J=6.6, $CH_3-$) $\delta$ 0.98 (9H, S, $+$Si-) $\delta$ 0.25 (6H, S, $(CH_3)_2Si<$ ).

EXAMPLE 7

(3S*,4R*)-3-[(R*)-1-hydroxyethyl]-4-(3-p-nitrobenzyloxycarbonyl-2-oxopropyl)-azetidin-2-one

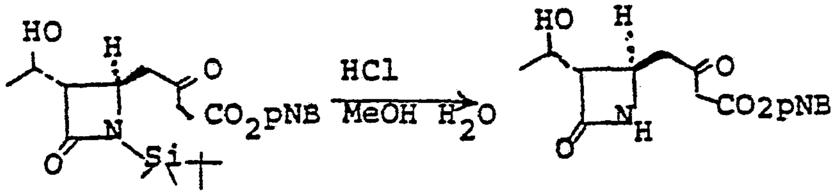


A solution of (3S*, 4R*)-1-(t-butyldimethylsilyl)-3-[(R*)-1-hydroxyethyl]-4-(3-p-nitrobenzyloxycarbonyl-2-oxopropyl)-azetidin-2-one (1.0 mmol) in 20 ml of 9:1 (v/v) methanol-water is cooled to 0°C. Concentrated hydrochloric acid (0.34 ml) is added and the resulting solution is stirred at 0°C for 15 min., then allowed to warm to room temperature. After 2.5 hrs. at room temperature the reaction mixture is diluted with ethyl acetate (25 ml), washed with water (10 ml) and brine, dried over magnesium sulfate and concentrated *in vacuo* to yield (3S*, 4R*)-3-[(R*)-1-hydroxyethyl]-4-(3-p-nitrobenzyloxycarbonyl-2-oxopropyl)-azetidin-2-one.

EXAMPLE 8

Preparation of (3S*,4R*)-3-[(R*)-1-hydroxyethyl)-4-[3-(4-nitrobenzyl)oxycarbonyl-2-oxo-3-diazopropyl]azetidin-2-one

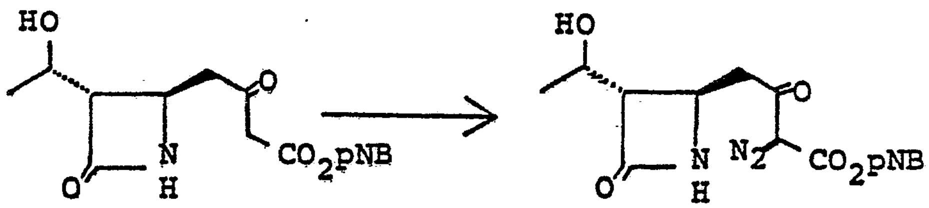

Triethylamine (263 mg, 2.6 mmol) is added by syringe to a mixture of (3S*,4R*)-3-[(R*)-1-hydroxyethyl]-4-[3-(4-nitrobenzyl)oxycarbonyl-2-oxopropyl]azetidin-2-one (253 mg, 0.72 mmol) and p-carboxybenzene sulfonylazide (196 mg, 0.84 mmol) in dry acetonitrile (6 ml) at 0°C. When addition is complete the cooling bath is removed and the reaction mixture is stirred at room temperature for 1 hour. The mixture is then diluted with ethyl acetate (50 ml) and filtered. The filtrate is concentrated *in vacuo* and the residue is chromatographed on a short silica gel column (ethyl acetate) to yield 222 mg, (81% overall from (3S*, 4R*)-1-(t-butyldimethylsilyl)-3-[(R*)-1-(t-butyl dimethylsilyloxy)ethyl]-4-[3-(4-nitrobenzyl)oxycarbonyl-2-oxopropyl]azetidin-2-one) of (3S*,4R*)-3-(R*)-1-hydroxyethyl)-4-[3-(4-nitrobenzyl)oxycarbonyl-2-oxo-3-diazopropyl]azetidin-2-one as a white solid. IR($CHCl_3$, $CM^{-1}$) 3410, 2132, 1756, 1718, 1650, 1350, 1280, 1120; n.m.r.($CDCl_3$) $\delta$7.9(2d-aromatic,4), $\delta$5.4(s,2), $\delta$6.2(brs,1), $\delta$4.1(m,2), $\delta$2.6-3.6(m,4), $\delta$1.32(d,3,J=6.2).

EXAMPLE 9

Preparation of (5R*,6S*)p-Nitrobenzyl 6-[(R*)1-hydroxyethyl]-1-azabicyclo[3.2.0]heptan-3,7-dione-2-carboxylate

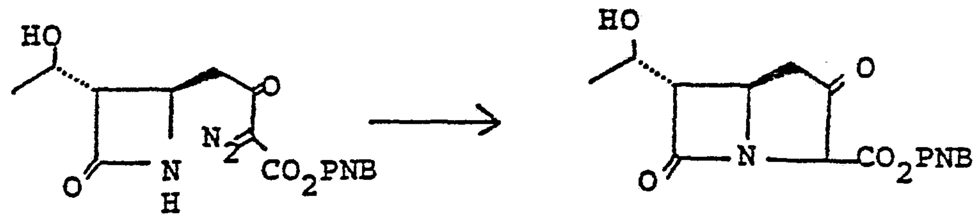

A suspension of (3S*,4R*)-3-[(R*)-1-hydroxyethyl]-4-[3-(4-nitrobenzyl)oxycarbonyl-2-oxo-3-diazopropyl]azetidin-2-one (56.4 mg, 0.15 mmol) and rhodium (II) acetate (0.1 mg) in dry benzene (3ml) is deoxygenated by bubbling through nitrogen for 10 minutes. The mixture is then heated to 78°C for 1 hour. During heating the solid starting material gradually goes into solution. The mixture is then cooled, filtered to remove the catalyst, and the filtrate is concentrated *in vacuo* to yield (5R*, 6S*)p-nitrobenzyl 6-[(R*)-1-hydroxyethyl]-1-azabicyclo[3.2.0]heptan-3,7-dione-2-carboxylate.

Physical Properties:

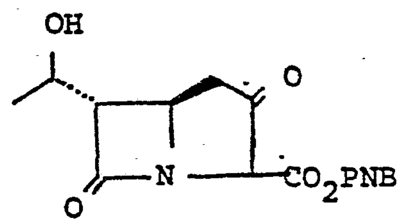

PNB = p-nitrobenzyl

n.m.r.: (300MHz, $CDCl_3$)$\delta$8.26, 7.54(aromatic, 4), 5.29 (AB, 2), 4.77 (s,1), 4.32(dq,1,J=6.6,7), 4.16(ddd,1, J=7,7.5,2.2), 3.21(dd,1,J=7,2.2), 2.94(dd,1,J=19.5,7) 2.50(dd,1,J=19.5,7.5), 2.2(brs,1), 1.37(d,3,J=6.6).

I.R.: ($CHCl_3$,$CM^{-1}$) 1770, 1758, 1610, 1522, 1353

## EXAMPLE 10

### Preparation of p-Nitrobenzyloxycarbonylaminoethanethiol

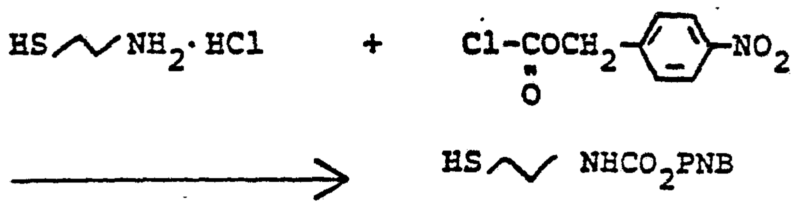

To 600 ml diethyl ether ($Et_2O$) - 75 ml $H_2O$ in an ice bath with stirring is added 3.2 g cysteamine hydrochloride (mw = 114; 28.1 mmole). A solution of 7.14 g $NaHCO_3$ (mw = 84; 85 mmole) in 75 ml $H_2O$ is added. The ice bath is removed, and at room temperature a solution of 6.75 g p-nitrobenzylchloroformate (mw = 216; 31.3 mmole) in 270 ml $Et_2O$ is added dropwise over a period of one hour. After 10 additional minutes, the layers are separated. The ether layer is extracted with 150 ml 0.25 N HCl, and then with 200 ml brine. Each aqueous layer is then backwashed successively with 100 ml $Et_2O$. The combined $Et_2O$ layers are dried over anhydrous $MgSO_4$, filtered, and concentrated under a $N_2$ stream. The crystalline residue is slurried in a small amount of ether, filtered, and the pale yellow crystals are dried under high vacuum to give 4.7 g. p-nitrobenzyloxycarbonylaminoethanethiol (65% yield). NMR ($CDCl_3$): 8.18 (d, J=8Hz, aromatic protons ortho to nitro), 7.47 (d, J=8Hz, aromatic protons meta to nitro), 5.27 (-NH-), 5.20 (s, $_CH_2$-NH-), 2.67 (m, $-CH_2$-SH), 1.35 (t, J=8.5Hz, -SH) in ppm downfield from TMS. IR ($CHCl_3$ solution): carbonyl- 1725 $cm^{-1}$. M.S.: molecular ion-256, (M-47) at 209, (M-136) at 120, $^+CH_2ØpNO_2$ at 136.

EXAMPLE 11

Preparation of (5R*,6S*)p-Nitrobenzyl 3-[2-(p-nitrobenzyloxycarbonyl)aminoethylthio]-6-[(R*)-1-hydroxyethyl]-1-azabicyclo[3.2.0]-hept-2-en-7-one-2-carboxylate

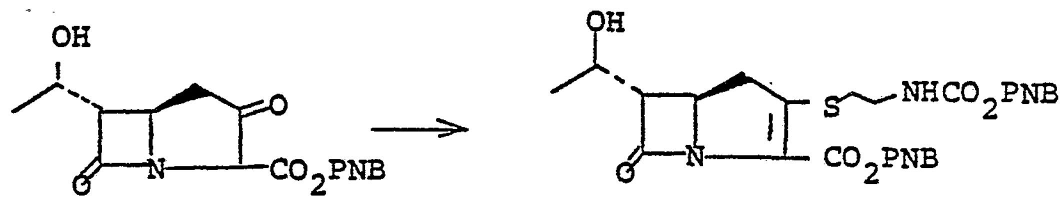

(5R*,5S*)p-Nitrobenzyl 6-[(R*)1-hydroxyethyl]-1-azabicyclo[3.2.0]heptan-3,7-dione-2-carboxylate (51 mg, 0.147 mmol) is dissolved in acetonitrile (3 ml) and the resulting solution is cooled to 0°C. Diisopropylethylamine (22 mg, 0.17 mmol) is added by syringe and the resulting solution is stirred at 0°C for 1 minute prior to the addition of a solution of freshly recrystallized p-toluene sulfonic anhydride (51 mg, 0.156 mmol) in dry acetonitrile (1 ml). The resulting solution is stirred at 0°C for 1 hour to provide (5R*,6S*)p-nitrobenzyl 3-(p-toluenesulfonyloxy)-6-[(R*)-1-hydroxyethyl]-1-azabicyclo[3.2.0]hept-2-en-7-one-2-carboxylate, then cooled to -25°C. Diisopropylethylamine (80.5 mg, 0.624 mmol) is added by syringe followed shortly thereafter by a solution of N-p-nitrobenzyloxycarbonylcysteamine (40 mg, 0.156 mmol) in 1 ml of dry acetonitrile. The reaction mixture is then stored in a refrigerator for 70 hrs. The mixture is diluted with 25 ml of ethylacetate washed with brine and dried over magnesium sulfate. Solvents are removed *in vacuo* to yield a yellow oil which is chromatographed on a silica gel plate (ethyl acetate, $R_f$ = 0.4) to yield (5R*,6S*)-p-nitrobenzyl-3-[2-(p-nitrobenzyloxycarbonyl)-aminoethylthio]-6-[(R*)-1-hydroxyethyl]-1-azabicyclo-

[3.2.0]-hept-2-en-7-dione-2-carboxylate as a yellow solid. IR (Nujol mull) 1773 and 1690 $cm^{-1}$; n.m.r. ($CDCl_3$) δ 7.54-8.26 (overlapping ABq,4), δ 5.40(ABq, 2), δ 5.22(s,2), δ 4.27(m,2), δ 3.47(m), 3.23(dd, 1), δ 3.14(dd, 1), δ 3.40(dd, 1), δ 3.04(m,2), δ 1.37(d,3).

EXAMPLE 12

Preparation of Thienamycin

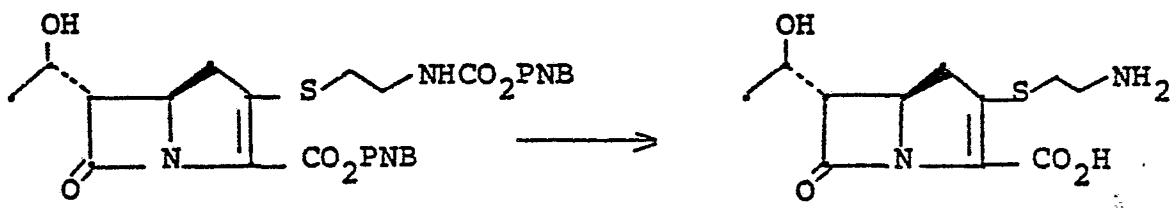

A mixture of N-p-nitrobenzyloxycarbonyl thienamycin p-nitrobenzyl ester (10 mg, 0.017 mmol) and 10% Pd/C-Bolhofer type in tetrahydrofuran (2 ml), 0.1M dipotassium hydrogen phosphate solution (1.4 ml) and 2-propanol (0.2 ml) is hydrogenated at 40 psi on the Parr shaker for 30 minutes. The mixture is then filtered and the catalyst is washed with water (3 x 3 ml). The combined filtrate and washings are extracted with ethyl acetate-ethyl ether then concentrated to ~3ml and lyophilized.

EXAMPLE 13

Preparation of 6-Methylthienamycin

* * *

1-(t-Butyldimethylsilyl)-3-(1-hydroxyethyl)-3α,β-methyl-4-(prop-2-en)-azetidin-2-one

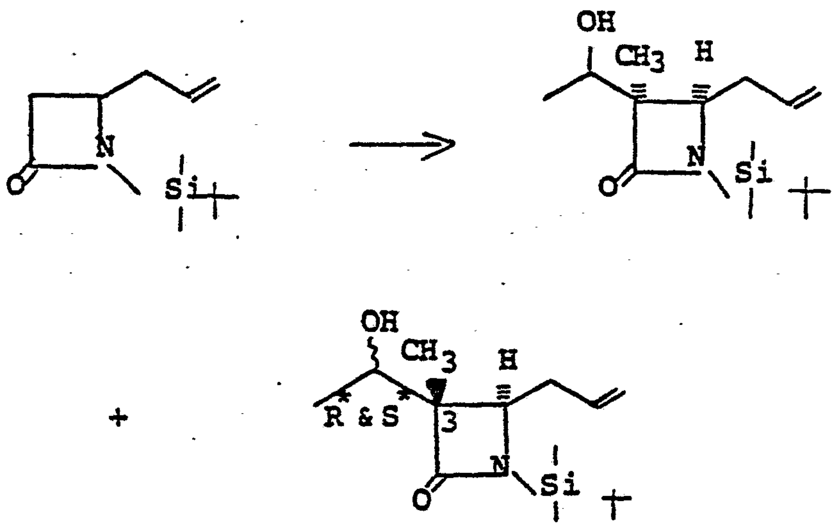

+

To 342μl diisopropylamine (2.44 mmol) in 5 ml anhydrous tetrahydrofuran (THF) at -78°C with stirring under $N_2$ is added 1 ml of 2.45M n-butyllithium (2.45 mmol). After stirring for 10 min, a solution of 500 mg of 1-(t-butyldimethylsilyl)-4-(prop-2-en)-azetidin-2-one in 2 ml anhydrous THF is added. After stirring for 10 min, 153μl methyliodide (2.46 mmol) is added. The cooling bath is removed, and the reaction mixture is stirred for 25 min. Upon re-cooling to -78°C, 1 ml of 2.45M n-butyllithium (2.45 mmol) is added. After 5 min, 250μl distilled acetaldehyde (4.49 mmol) is added, and the cooling bath is removed. After 30 min, the reaction mixture is added to 10 ml 1M $KH_2PO_4$-brine-$Et_2O$. After separation of the layers, the organic layer is washed with 10 ml 1M $KH_2PO_4$-15 ml brine and then with brine. After drying over magnesium sulfate,

filtration, and concentration *in vacuo*, 626 mg of crude reaction product is obtained. Chromatography on silica gel (0-20% ether:petroleum ether) provides 125 mg of the 3α-methyl compound and 234 mg of a slightly more polar mixture of 3β-methyl compounds (R* and S* ).

NMR of 3α methyl component (300MHz, $CDCl_3$) δ 0.24 and 0.26 (2S, $Si(CH_3)_2$), 0.97 (S, $Si-C(CH_3)_3$), 1.22 (d, J=6Hz, $\underline{CH}_3CHOH-$), 1.30 (S, $CH_3$), 2.34 (d, OH), 2.42-2.81 (m, $\underline{CH}_2CH=CH_2$), 3.44 (dd, J=4 and 10Hg, $H_4$), 4.09 - 4.19 (m, $CH_3C\underline{H}OH-$), 5.09-6.02 (m, $-C\underline{H}=C\underline{H}_2$).

NMR of 3β-methyl components (300MHz, $CDCl_3$) δ 0.23-0.25 (s's, $Si(CH_3)_2$'S), 0.95 and 0.96(2S, $Si-C(CH_3)_3$'S), 1.14 - 1.22 (series of peaks for $CH_3$'s and $\underline{CH}_3CHOH$'S) 2.29 - 2.62 (m, $\underline{CH}_2CH=CH_2$), 3.40 (dd, J=3.5 and 11Hz, $H_4$ of S*), 3.70 (dd, J=3.5 and 11 Hz, $H_4$ of R*), 3.86 - 4.00 (m, $CH_3\underline{CH}OH-$), 5.08-5.96 (m, $C\underline{H}-C\underline{H}_2$).

1-(t-Butyldimethylsilyl)-3-(1-oxoethyl)-3β-methyl-4-(prop-2-en)-azetidin-2-one

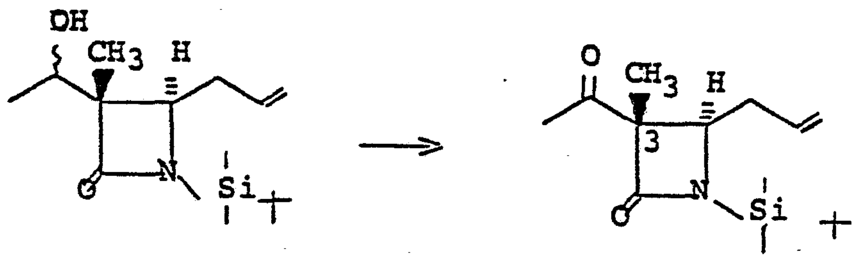

Trifluoroacetic anhydride (180μl,1.28 mmol) is added dropwise by syringe to a solution of dimethylsulfoxide (120μl, 1.69 mmol) in anhydrous methylene chloride (2.5 ml) at -78°C under $N_2$. The resulting mixture is stirred at -78°C for 15 min. A solution of 1-(t-butyldimethylsilyl)-3-(1-hydroxyethyl)-3β-methyl 4-(prop-2-en)-azetidin-2-one (234 mg, 0.83 mmol) in methylene chloride (2 ml) is added, and the resulting solution is stirred at -78°C for 1 hr. Triethylamine (382μl, 2.76 mmol) is added by syringe, and the cooling bath is removed. After an additional 1 hr, the reaction mixture is diluted with methylene chloride, washed with water and brine, dried over magnesium sulfate, filtered. Removal of solvents *in vacuo* yields partially crystalline oil. Chromatography on silica gel (0-10% ether:petroleum ether) provides 191 mg of the title compound.

IR($CHCl_3$)μ 5.76, 5.87

MS m/e $NoM^+$, 266, 240, 224

NMR (60MHz, $CDCl_3$) 0.25(2S, $Si(CH_3)_2$), 0.97(s, Si-$C(CH_3)_3$), 1.45(s, $CH_3$), 2.28(s, $CH_3$-C=O), 2.33-2.62 (m, $C\underline{H}_2CH=CH_2$), 4.15(dd, J=5 and 10Hz, $H_4$), 4.88-6.10 (m, $C\underline{H}=C\underline{H}_2$).

1-(t-Butyldimethylsilyl)-3-(1-hydroxyethyl)-3β-methyl-4-(prop-2-en)-azetidin-2-one

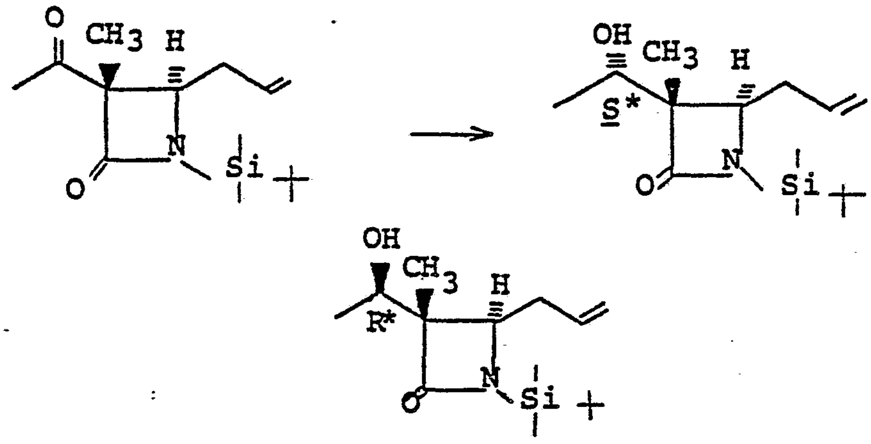

To a stirred solution of 4.26g of 1-(t-butyldimethylsilyl)-3-(1-oxoethyl)-3β-methyl-4-(prop-2-en)-azetidin-2-one (15.2 mmol) in 90 ml i-propanol is added 807 mg sodium borohydride (21.2 mmol). After stirring vigorously under $N_2$ for 45 min, the reaction mixture is poured (carefully) into ~100ml 1M $KH_2PO_4$- 400 ml $H_2O$-500ml $Et_2O$. After phase separation, the aqueous layer is washed 2 x $Et_2O$. The combined organic layers are then washed 2 x brine, dried over magnesium sulfate, filtered, and concentrated in vacuo to 4.1g of an oil. Chromatography on 300g silica gel (0-10% acetone:hexane) provided 3.84g of material containing mainly the S* diastereomer and 1.58g of the R* diastereomer.

Data for the R* diastereomer:

IR($CHCl_3$)μ 5.80

MS m/e 284($M^+$ + 1), 268, 226

NMR (300 MHz, $CDCl_3$) δ 0.24(2S, $Si(CH_3)_2$, 0.96(s, $Si(CH_3)_3$), 1.17(d, partially hidden by sat 1.18, $CH_3CHOH$-) 1.18 (s, $CH_3$)1 1,70(d, OH), 2.32-2.60 (m, $CH_2CH=CH_2$), 3.71(dd, J=4 and 11Hz, $H_4$), 3.89 - 3.98(m, $CH_2CHOH$), 5.12-5.90 (m, $CH=CH_2$).

1-(t-Butyldimethylsilyl)-3-(1-t-Butyldimethylsilyloxyethyl)-3β-methyl-4-(prop-2-en)-azetidin-2-one

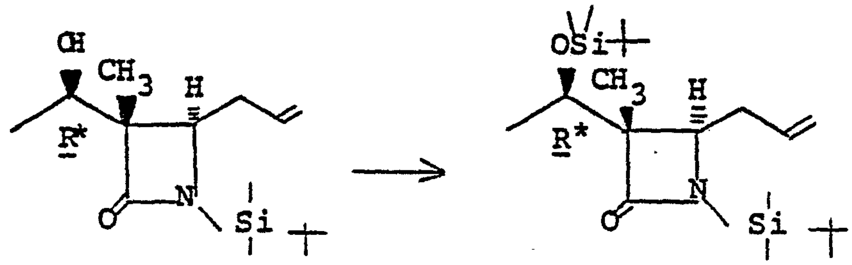

With stirring under $N_2$, 582 mg of 1-(t-butyldimethylsilyl)-3-(1-hydroxyethyl)-3β-methyl-4-(prop-2-en)-azetidin-2-one (2.06 mmol) in 6 ml anhydrous N,N-dimethylformamide is treated with 320μl triethylamine (2.31 mmol) followed by 356 mg t-butyldimethylsilylchloride (2.37 mmol). After stirring over night, the reaction mixture is poured into 1.3 ml, $1MKH_2PO_4$- 50 ml brine-50 ml methylene chloride. After phase separation, the aqueous layer is again extracted with methylene chloride, The combined organic layers are washed 2 x brine, dried over magnesium sulfate, filtered and concentrated to 699 mg crude material. Chromatography on silica gel (0-50% ethylacetate:hexane) provides 473 mg of the title compound and 211 mg recovered starting material.

IR ($CHCl_3$)μ 5.78

MS m/e 397 ($M^+$), 382, 340

NMR (300MHz, $CDCl_3$)δ 0.05, 0.08, 0.21 and 0.26 (4S, $Si(CH_3)_2$'s), 0.90 and 0.96 (2S, $Si-C(CH_3)_3$'s), 1.11(d, J=6Hz, $C\underline{H}_3CHOSi-$), 1.14(S, $CH_3$), 2.30-2.56 (m, $C\underline{H}_2CH=CH_2$), 3.66(dd, J=4 and 10Hz, $H_4$), 3.91 (q, J=6Hz, CH $C\underline{H}OSi-$), 5.08-5.94 (m, $-C\underline{H}=C\underline{H}_2$).

1-(t-Butyldimethylsilyl)-3-(1-t-butyldimethylsilyloxyethyl)-3β-methyl-4-(2-oxoethyl)-azetidin-2-one

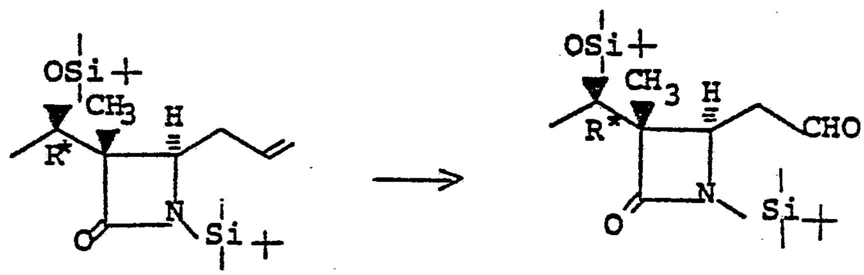

A solution of 1.05g (1-(t-butyldimethylsilyl)-3-(1-t-butyldimethylsilyloxyethyl)-3β-methyl-4-(prop-2-en)-azetidin-2-one (2.6 mmol) in 30 ml anhydrous methylene chloride is cooled to -78°C, and a stream of dried ozone in oxygen is bubbled through until the blue color disappears. The cooling bath is removed, and the reaction mixture is concentrated under a stream of $N_2$ and then *in vacuo*. The crude ozonide is dissolved in 5 ml methylene chloride and is treated with 580μl dimethylsulfide (7.9 mmol) and placed under $N_2$. After 6 hr, 580μl additional dimethylsulfide is added, and the reaction is allowed to stir overnight under $N_2$. The reaction is then concentrated under high vacuum followed by chromatography on silica gel (methylene chloride). Forward fractions, containing unreacted ozonide as well as the desired aldehyde, are re-treated with dimethylsulfide to increase the yield. Later fractions contain 531 mg of the desired aldehyde.

IR($CHCl_3$)μ 5.75, 5.80 (sh)

M.S. m/e 400($M^+$ + 1), 384, 342

NMR (300 MHz, $CDCl_3$)δ 0.06, 0.09, 0.19 and 0.24 (4S, $Si(CH_3)_2$'s), 0.88 and 0.95 (2S, $Si(CH_3)_3$'s), 1.09(s, $CH_3$), 1.15(d, J=6Hz, $C\underline{H}_3CHOSi$-), 2.75-2.80 (m, $CH_2CHO$), 3.98(q, J=6Hz, $CH_3CHOSi$-), 4.16(dd, J=5.5 and 7.5 Hz, $H_4$), 9.84(t, CHO).

1-(t-Butyldimethylsilyl)-3-(1-t-butyldimethylsilyloxyethyl)-3β-methyl-4-(carboxymethyl)-azetidin-2-one

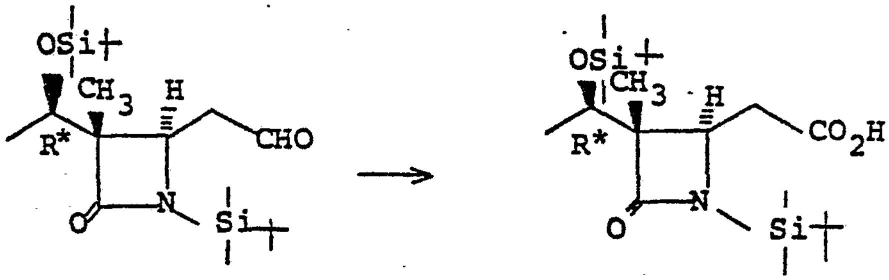

A solution of 620 mg 1-(t-Butyldimethylsilyl)-3-(t-butyldimethylsilyloxyethyl)-3β-methyl-4-(2-oxoethyl)-azetidin-2-one (1.55 mmol) in 22 ml distilled acetone is cooled in an ice bath and treated with 447μl of Jones reagent (2.6M in $CuO_3$, 1.16 mmol). After stirring for 15 min., 409μl absolute ethanol is added, and stirring is continued for 5 min. The cooling bath is removed, and the reaction mixture is quickly concentrated under a $N_2$ stream to a small volume. Ethylacetate and water are added to the concentrate. After phase separation, the organic layer is washed 3 x brine, dried over magnesium sulfate, filtered, and concentrated to 629 mg crude product. Chromatography on silica gel (0-1% acetic acid:methylene chloride) provides 498 mg of the desired product.

IR ($CHCl_3$)μ 5.76br

M.S. m/e(silylated)- NoM+ at 487, 472, 430 (487-t-butyl)

NMR (300MHz, $CDCl_3$) $\delta$ 0.04, 0.07, 0.20 and 0.24 (4S, $Si(CH_3)_2$'s), 0.86 and 0.93(2s, $Si-C(CH_3)_3$'s), 1.09(d, J=6Hz, $C\underline{H}_3CHOSi$), 1.11(s, $CH_3$), 2.63 and 2.72 (2dd, J=9 and 16Hz and J=5 and 16Hz respectively, $C\underline{H}_2CO_2H$), 3.96(q, J=6Hz, $CH_3C\underline{H}OSi-$), 4.11(dd, J=5 and 9Hz, $H_4$).

1-(t-Butyldimethylsilyl)-3-(1-t-Butyldimethylsilyloxyethyl)-3β-methyl-4-(3-p-nitrobenzyloxycarbonyl-2-oxopropyl)azetidin-2-one

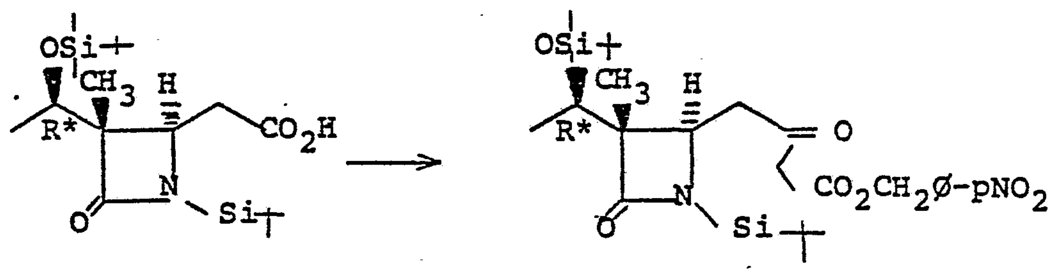

1,1'-Carbonyldiimidazole (1.09mg, 0.67mmol) is added in one portion to a solution of 251 mg 1-(t-butyldimethylsilyl)-3-(1-t-butyldimethylsilyloxyethyl)-3β-methyl-4-(carboxymethyl)-azetidin-2-one (0.60 mmol) in anhydrous tetrahydrofuran (3 ml) at room temperature under $N_2$. The resulting solution is stirred at room temperature for 3.5 hours. In a second flask, magnesium ethoxide (5 mmol) is added in one portion to a solution of the mono-p-nitrobenzyl ester of malonic acid (10 mmol) in anhydrous tetrahydrofuran (25 ml). The resulting mixture is stirred at room temperature for 1 hr, then the tetrahydrofuran is removed at the pump, and the gummy residue is triturated with ether to yield the magnesium salt as an off-white solid. The magnesium salt (339 mg, 0.678 mmol) is then added to the first reaction flask and the resulting mixture is stirred at room temperature overnight. The reaction mixture is then poured into 50 ml of ether, washed with 0.5N hydrochloric acid solution (12.3 ml), water (12.3 ml), saturated aqueous sodium bicarbonate solution (12.3 ml), brine and dried over magnesium sulfate. Removal of solvents *in vacuo* gives 356mg of an oil which is chromatographed on 6-1000μ silica

gel GF plates (25% acetone/hexane). The desired UV band is immediately scraped and extracted with 70% acetone/hexane. Concentration *in vacuo* gives 235 mg of the title compound.

$IR(CHCl_3)\mu$ 5.75

M.S. m/e NoM$^+$ at 592, 577, 535 (M$^+$-*t*-butyl)

NMR (300MHz, $CDCl_3$) $\delta$ 0.04, 0.08, 0.15 and 0.20 (4S, $Si(CH_3)_2$'s), 0.86 and 0.92 (2S, $Si\text{-}C(CH_3)_3$'s), 1.02 (s, $CH_3$), 1.12 (d, J=6Hz, $C\underline{H}_3CHOSi\text{-}$) 2.87 (brd, J=6Hz, $C\underline{H}_2\overset{O}{\parallel}{C}\text{-}CH_2CO_2\text{-}$), 3.58 (S, $\overset{O}{\parallel}{C}\text{-}CH_2CO_2\text{-}$), 3.95(q, J=6Hz, $CH_3C\underline{H}OSi\text{-}$), 4.17(brt, J=6Hz, $H_4$) 5.29 (s, $CO_2C\underline{H}_2\text{Ø}pNO_2$), 7.56 and 8.27 (2d, aromatic protons).

3-(1-hydroxyethyl)-3β-methyl-4-(3-p-nitrobenzyloxycarbonyl-2-oxopropyl)azetidin-2-one

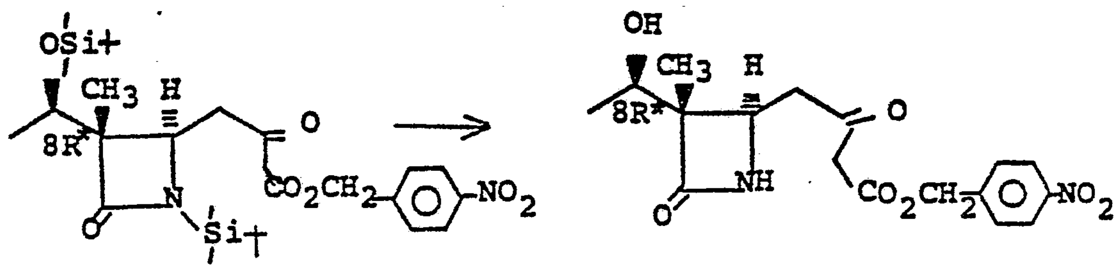

Concentrated hydrochloric acid (0.68 ml) is brought to a volume of 40 ml with 9:1 methanol:water, and 22ml of the solution (4.5 mmol) is added to 319 mg of 1-(t-butyldimethylsilyl)-3-(1-t-butyldimethyl-silyloxyethyl)-3β-methyl-4-(3-p-nitrobenzyloxycarbonyl-2-oxopropyl)-azetidin-2-one (0.54 mmol) with good stirring under $N_2$. After 8 hr, the reaction is added to 8 ml $1MK_2HPO_4$- 150 ml $H_2O$ - 150 ml ethylacetate. After phase separtion, the organic layer is washed with brine containing 8 ml $1MK_2HPO_4$ and then brine alone (2x). After drying over magnesium sulfate, filtration, and concentration *in vacuo*, 241 mg of an oil is obtained. Chromatography on 6-1000μ silica gel GF plates (50% acetone/hexane) followed by extraction of the desired UV band with 70% acetone/hexane provides 129 mg of the title compound. A less polar UV band contains 57 mg of material still having the O-silyl group intact.

Data for the compound:

IR($CHCl_3$)μ 5.70(br), 5.81(sh)

M.S. m/e

NMR(300MHz, $CDCl_3$)δ 1.14(s, $CH_3$), 1.23(d, J=6Hz, $C\underline{H}_3CHOH$), 2.81-2.98(m, $C\underline{H}_2\overset{O}{C}CH_2CO_2$-), 3.62(s, $C\underline{H}_2CO_2$-$CH_2$ØP$NO_2$), 4.05(center of m, $H_4$ and $CH_3CHOH$), 5.30 (s, $CO_2C\underline{H}_2$Øp$NO_2$), 6.01(br, s, NH), 7.57 and 8.30 (2d, aromatics).

3-(1-Hydroxyethyl)-3β-methyl-4-(3-diazo-3-p-nitrobenzyloxycarbonyl-2-oxopropyl)-azetidin-2-one

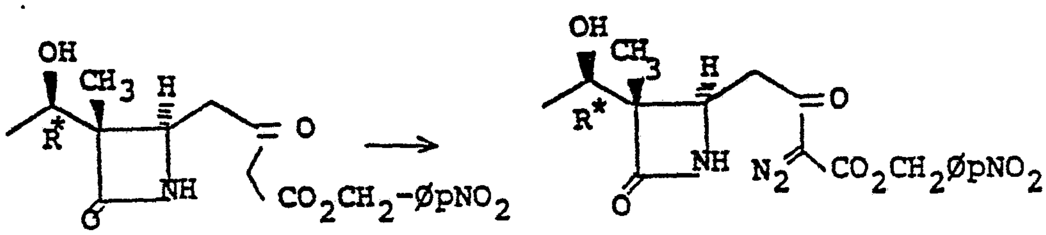

Triethylamine (55μl, 0.397 mmol) is added by syringe to a mixture of 3-(1-hydroxyethyl)-3β-methyl-4-[3-(p-nitrobenzyl)oxycarbonyl-2-oxopropyl]-azetidin-2-one (40 mg, 0.11 mmol) and p-carboxybenzene sulfonylazide (30 mg, 0.13 mmol) in dry acetonitrile (1 ml) at 0°C. When addition is complete, the cooling bath is removed, and the reaction mixture is stirred at room temperature for 1.5 hour. The mixture is then diluted with ethyl acetate (10 ml) and filtered. The filtrate is concentrated *in vacuo*, and the residue is dissolved in methylene chloride, filtered, and concentrated *in vacuo* to 53 mg white foam. A fast filtration through a short silica gel column (ethylacetate) provides 41 mg of the title compound as a slightly off white solid. On a larger scale, the product may crystallize out of the reaction mixture along with by-products of the reaction. Extraction of the original, insoluble materials with methylene chloride, followed by chromatography of the contents of the filtrate, yields the product in these instances.

mp 162-170°C dec.
IR($CHCl_3$)μ 4.70, 5.70, 5.81
NMR (300MHz, di-DMSO)δ 0.95(s, $CH_3$), 1.03(d, J=6Hz, $\underline{CH_3}CHOH$), 3.05 (d, J=6Hz, $\underline{CH_2}\overset{O}{C}CH_2CO_2-$), 3.68-3.76 (m, $CH_3\underline{CH}OH$), 3.90(t, J=6Hz, $H_4$), 4.85(d, OH), 5.46(s, $CH_2$-Ø-p-$NO_2$), 7.76 and 8.31(2d, aromatic protons), 7.93 (s, NH).

p-Nitrobenzyl 6-(1-hydroxyethyl)-6β-methyl-3-[2-p-nitrobenzyloxycarbonyl)amino]ethylthio-1-azabicyclo-[3.2.0]hept-2-en-7-one-2-carboxylate

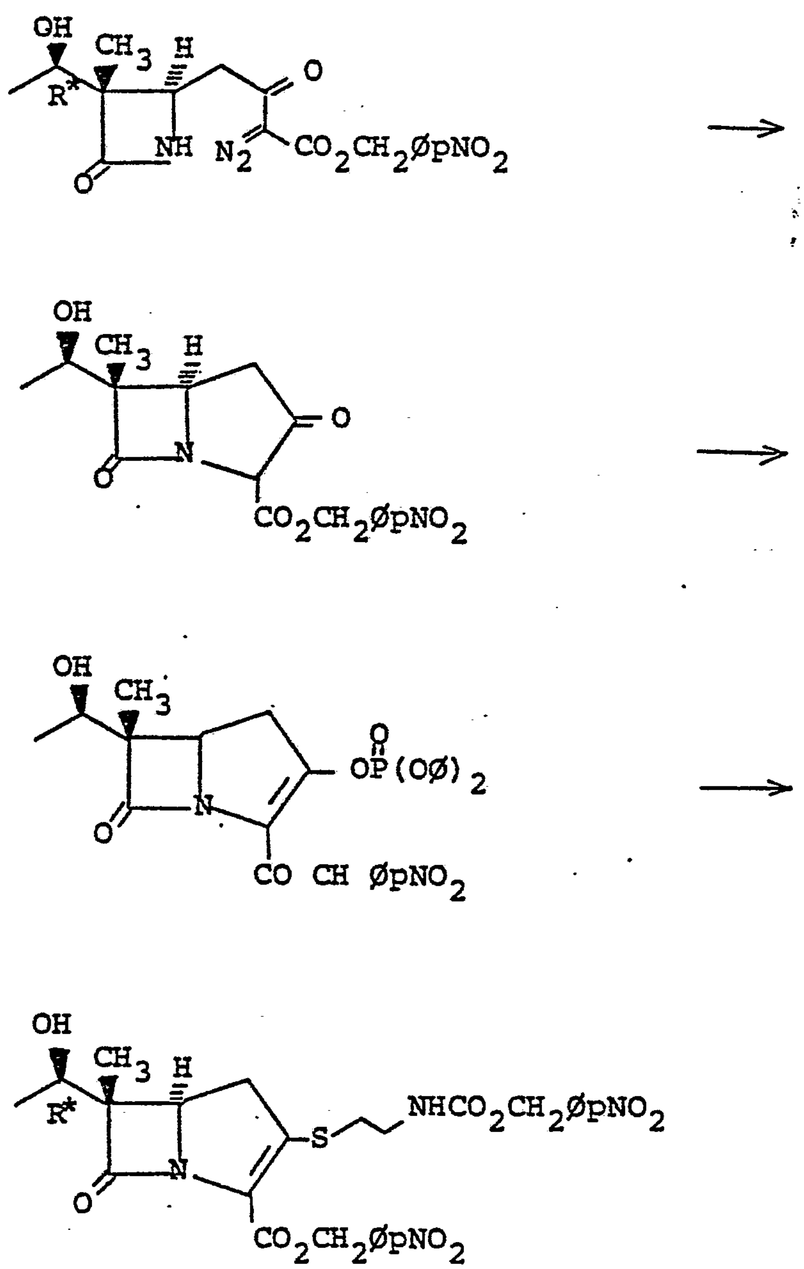

A suspension of 3-(1-hydroxyethyl]-3β-methyl-4-[3-diazo-3-p-nitrobenzyloxycarbonyl-2-oxopropyl]-azetidin-2-one (35 mg, 0.09 mmol) and rhodium (II) acetate (0.1 mg) in dry toluene (3.2 ml) is deoxygenated by evacuating and treating with nitrogen alternately (3X). The mixture is then heated to ca. 100°C for 1 hour with stirring under $N_2$. During heating the solid starting material gradually goes into solution. The mixture is then cooled, filtered to remove the catalyst, and the filtrate is concentrated in vacuo to yield the above bicycloketone as a white foam. The crude bicycloketone (32.5 mg, 0.09 mmol) is dissolved in anhydrous acetonitrile (1.8 ml), and the resulting solution is cooled to 0°C under $N_2$. Diisopropylethylamine (18.9μl, 0.11 mmol) is added followed by diphenylchlorophosphate (19.9μl, 0.10 mmol), and the resulting solution is stirred at 0°C for 55 min. Diisopropylethylamine (17.7μl, 0.10 mmol) is added by syringe followed by p-nitrobenzyloxycarbonylaminoethanethiol (26.6 mg, 0.104 mmol), and the reaction is stirred for 2.5 hr. The reaction is then added to 20 ml ethylacetate-10 ml water. After separation of the layers, the organic layer is extracted with 8 ml 0.1M $KH_2PO_4$, 8ml 0.1M $K_2HPO_4$, 2xbrine, dried over magnesium sulfate, filtered and concentrated in vacuo to 65 mg yellow foam. Chromatography on 3-1000μ silica gel GF plates (ethylacetate) provides upon extraction (ethylacetate) of the desired UV band, concentration to an oil, addition of methylene chloride (drying over magnesium sulfate), filtration, and reconcentration in vacuo, 31 mg of the title compound as a pale yellow foam.

IR ($CHCl_3$)μ 5.64, 5.81(br)

M.S. m/e 600($M^+$), 556, 301

NMR (300MHz, $CDCl_3$) δ 1.24(S, $CH_3$), 1.27(d, J=6Hz, $C\underline{H}_3CHOH$), 3.15 (d for H's with J=9Hz on top of m from 2.94-3.2 for $-SC\underline{H}_2CH_2NH-$), 3.39 - 3.52 (m, $-SCH_2C\underline{H}_2-NH-$), 4.15(center of m, $CH_3C\underline{H}OH$), 4.31 (brt, J=9Hz, $H_5$), 5.20 (s, $NHCO_2C\underline{H}_2$ arom), 5.38(center of two widely spaced d, noneq. methylene protons of ester), 7.50-8.27 (series of peaks, aromatics).

(±)-6-methylthienamycin

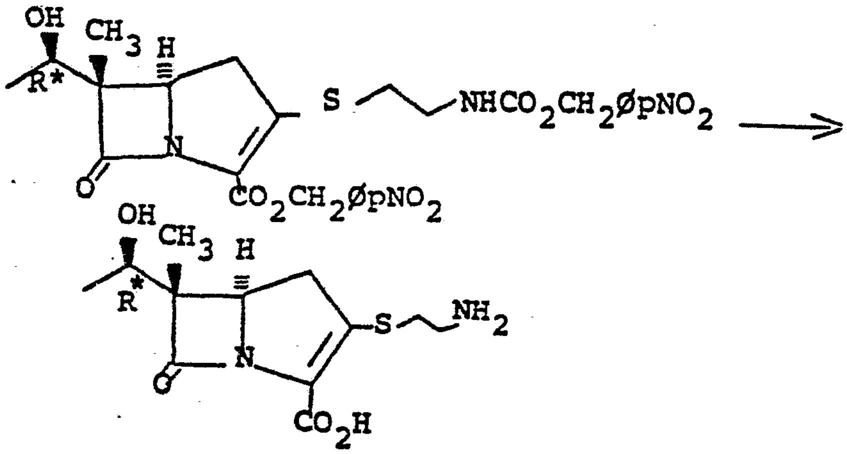

To 5.3mg p-nitrobenzyl 6-(1-hydroxyethyl)-6β-methyl-3-[2-p-nitrobenzyloxycarbonyl)amino]ethylthio-1-azabicyclo[3.2.0]hept-2-en-7-one-2-carboxylate is added 500μl distilled THF, 500μl absolute EtOH, 330μl DI-water, 33μl MOPS buffer, 6.9mg platinum oxide, and two glass beads. The reaction mixture is placed in a Parr shaker, evacuated and covered with $N_2$ alternately (3X), evacuated and covered with 50psi $H_2$. After cooling in an ice bath, the reaction mixture is centrifuged, and the supernatant is filtered through a small plug of cotton into a cold centrifuge tube. The residual catalyst is washed (3X) with 15 drops DI-$H_2O$ and centrifuged; the supernatants are added to the original filtrate which is finally extracted with ethylacetate (3 x 1 ml). The aqueous layer is briefly placed on an aspirator to remove residual organic solvents and then applied to a small column of XAD-2 resin (~7ml vol) packed and eluted with DI-$H_2O$. After the first 2.5 ml, the next 30 ml eluant contains the product. The above sequence is repeated on 5.4mg and 5.0 mg additional starting material.

The combined aqueous solutions are concentrated <u>in vacuo</u> without heat to 3 ml volume. The solution is passed portionwise through a semi-prep (9.5ml void volume) μ-Bondapak-$C_{18}$ HPLC column (3% THF/DI-$H_2O$, flow rate-2 ml/min, 254mμ filter), and the major peak is collected. Concentration, as above, afforded a soltuion of 2.1mg of the title compound having a hydroxylamine-quenchable $UV_{max}$ at 297mμ, essentially no electrophoretics mobility in pH7 phosphate buffer (1500V-30 min), and a clean HPLC trace (retention time ~ 6.5 min).

EXAMPLE 14

Following the procedure of Example 2, the azetidinones of Table I are obtained when the basic procedure of Example 2 is modified according to the remarks entered in Table I.

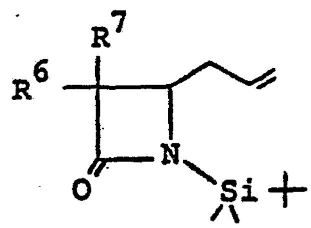

TABLE I

| Compound | $R^6$ | $R^7$ | Remarks |
|---|---|---|---|
| 1.) | $(CH_3)_2CH$ | H | As in Example 2, but substitute equivalent amount of isopropyl iodide for acetaldehyde. |
| 2.) | $CH_3$ | H | As in Example 2 ,but using an equivalent amount of methyl iodide for acetaldehyde. |
| 3.) | $HOCH_2$ | $CH_3$ | As in Example 2, but use compound 2., and excess formaldehyde introduced as a gas just above surface of stirred solution. |
| 4.) | $ØCH_2CH(OH)$ | H | As in Example 2, but using an equivalent amount of phenyl acetaldehyde for acetaldehyde. |

Ø = phenyl

| Compound | $R^6$ | $R^7$ | Remarks |
|---|---|---|---|
| 5.) | $CH_3\overset{OH}{C}H$ | $CH_3$ | Using the procedure of Example 2 upon compound 2 of Table I. |
| 6.) | ØCH$_2$ | H | As in Example 2, but substitute benzylbromide for acetaldehyde. |
| 7.) | $CH_3\overset{OH}{C}H$ | ØCH$_2$ | As in Example 2, but using compound 6 as substrate. |
| 8.) | $CH_3\overset{OMs}{C}H$<br>Ms=mesyl | H | Obtained from the product of Example 2 and methanesulfonyl chloride and triethylamine in methylene chloride at 0°. |
| 9.) | $CH_3\overset{N_3}{C}H$ | H | Obtained from compound 8 on treatment with $LiN_3$ in DMF at 60°. |
| 10.) | $CH_3\overset{NH_2}{C}H$ | H | Obtained from compound 9 by reduction with $H_2S$ and $Et_3N$ in $CH_2Cl_2$. |
| 10a.) | $CH_3\overset{NHCO_2PNB}{C}H$ | H | Obtained from compound 9 on treatment with $ClCO_2PNB$ and DMPA in $CH_2Cl_2$ at 0°. |
| 11.) | $(CH_3)_2CH\overset{OH}{C}H$ | H | As in Example 2, but substituting isobutyraldehyde for acetaldehyde. |

| Compound | $R^6$ | $R^7$ | Remarks |
|---|---|---|---|
| 12.) | 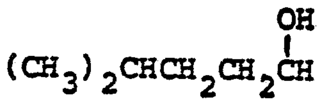 | H | As in Example 2, but substitute 5-methyl valeraldehyde for acetaldehyde |
| 13.) | 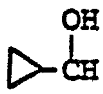 | H | As in Example 2, but substitute cyclopropane carboxaldehyde for acetaldehyde. |
| 14.) | 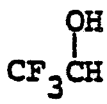 | H | As in Example 2, but substitute trifluoroacetaldehyde for acetaldehyde. |
| 15.) | 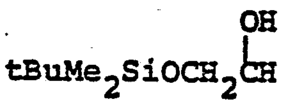 | H | As in Example 2, but substitute t-butyldimethylsilyloxyacetaldehyde for acetaldehyde. |
| 16.) | $HOCH_2CH_2$ | H | As in Example 2, but substitute oxirane for acetaldehyde. |
| 17.) | 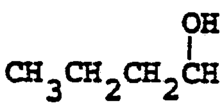 | H | As in Example 2, but substitute butyraldehyde for acetaldehyde. |
| 18.) | 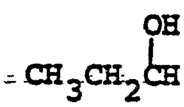 | H | As in Example 2, but substitute propionaldehyde for acetaldehyde. |
| 19.) | 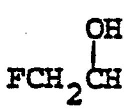 | H | As in Example 2, but substitute fluoroacetaldehyde for acetaldehyde. |

| Compound | $R^6$ | $R^7$ | Remarks |
|---|---|---|---|
| 20.) | 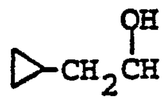 | H | As in Example 2, but substitute cyclopropylacetaldehyde for acetaldehyde. |
| 21.) | $CH_3CH_2$ | H | As in Example 2, but substitute ethyliodide for acetaldehyde. |
| 22.) | $CH_3$ | $CH_3$ | As in Example 2, but use compound 2 and substitute methyliodide for acetaldehyde. |
| 23.) | 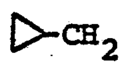 | H | As in Example 2, but substitute cyclopropylmethylbromide for acetaldehyde. |
| 24.) | $HOCH_2CH_2$ | $CH_3$ | As in Example 2, but use compound 2 and substitute oxirane for acetaldehyde. |
| 25.) | 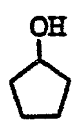 | H | As in Example 2, but use cyclopentanone instead of acetaldehyde. |
| 25a.) | $ClCH_2$-CH-<br>OH | H | As in Example 3B& 4, but use an equivalent amount of chloroacetylimidazole instead of acetylimidazole. |
| 25b.) | $ClCH_2$-CH-<br>OH | $CH_3$ | As in Example 14, Table I, No. 25a, but starting with azetidinone of Example 14, Table I, No.3. |

| Compound | $R^6$ | $R^7$ | Remarks |
|---|---|---|---|
| 26.) | $CF_3\overset{O}{\overset{\|}{C}}-$ | H | As in Example 3B, but use ethyl trifluorothiolacetate instead of N-acetylimidazole. |
| 27.) | $CF_3\overset{OH}{\overset{\|}{C}}H-$ | H | As in Example 4, but substitute product No. 26, Table I, Example 14, and use sodium borohydride as reductant. |
| 28.) | $N_3CH_2\overset{OH}{\overset{\|}{C}}H$ | H | As in Example 2, but use azidoacetaldehyde instead of acetaldehyde. |
| 29.) | $PNBO\overset{O}{\overset{\|}{C}}CH_2$ | H | As in Example 2 but substituted p-nitrobenzyl bromoacetate for acetaldehyde. |
| 30.) | $MeOCH_2\overset{O}{\overset{\|}{C}}-$ | H | As in Example 3B, but substituted N-methoxyacetyl imidazole for N-acetyl imidazole. |
| 31.) | $MeOCH_2\overset{OH}{\overset{\|}{C}}H$ | H | Obtained by employing the procedure of Example 4 on compound No. 30, Table I, Example 14. |
| 32.) | $CF_2CH\overset{O}{\overset{\|}{C}}H$ | H | As in Example 3B, but substitute ethyl difluorothiolacetate for N-acetylimidazole. |
| 33.) | $CF_2CH\overset{OH}{\overset{\|}{C}}H$ | H | Obtained from No. 32, above, using the procedure of Example 4, but substituting sodium borohydride as the reducing agent. |

| Compound | $R^6$ | $R^7$ | Remarks |
|---|---|---|---|
| 34.) | $PNBO\underset{O}{\underset{\|}{C}}OCH2$ | $CH_3$ | Obtained from No. 3, Table I, Example 14 with p-nitrobenzyl chloroformate and 4-dimethylaminepyridine in methylenechloride. |
| 35.) | $PNBO\overset{O}{\overset{\|}{C}}OCH_2CH_2$ | H | Obtained from No. 16 Table I, Example 14, by reaction with p-nitrobenzyl chloroformate and triethylamine in methylene chloride. |
| 36.) | $PNBO\overset{O}{\overset{\|}{C}}OCH_2CH_2$ | $CH_3$ | Obtained from No. 24 Table I, Example 14 as described for the preceeding compound. No.35. |
| 37.) | $HOCH_2$ | H | As in Example 2, but use excess formaldehyde instead of acetaldehyde. |
| 38.) | $PNBO\underset{O}{\underset{\|}{C}}OCH_2$ | H | Obtained from compound 36, above, and p-nitrobenzyl-chloroformate in methylene chloride containing 4-dimethylaminopyridine. |

EXAMPLE 15

Following the foregoing Examples and text, particularly Example 9, the representative intermediates of the present invention are obtained when the indicated substitution from Example 14 is made into the scheme of Example 9.

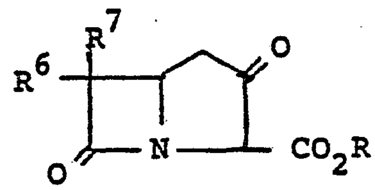

R = PNB (p-nitrobenzyl)

TABLE II

| Compound | $R^6$ | $R^7$ | Remarks |
|---|---|---|---|
| 1.) | $(CH_3)_2CH$ | H | |
| 2.) | $CH_3$ | H | |
| 3.) | $PNBO\overset{O}{\overset{\|}{C}}OCH_2$ | $CH_3$ | The primary alcohol, No.3 Table I, Example 14 is protected as shown by reacting with an equivalent amount of $ClCO_2PNB$ in the presence of DMAP (dimethylaminopropane) in methylene chloride. |
| 4.) | $ØCH_2\overset{OH}{\overset{\|}{C}H}$ | H | |

Ø = phenyl

| Compound | $R^6$ | $R^7$ | Remarks |
|---|---|---|---|
| 5.) | $CH_3\overset{OH}{C}H$ | $CH_3$ | |
| 6.) | $\emptyset CH_2$ | H | |
| 7.) | $CH_3\overset{OH}{C}H$ | $\emptyset CH_2$ | |
| 8.) | $CH_3\overset{N_3}{C}H$ | H | |
| 9.) | $CH_3\overset{NHCO_2PNB}{C}H$ | H | |
| 10.) | $(CH_3)_2CH\overset{OH}{C}H$ | H | |
| 11.) | $(CH_3)_2CHCH_2CH_2\overset{OH}{C}H$ | H | |
| 12.) | 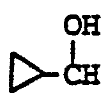 | H | |

| Compound | $R^6$ | $R^7$ | Remarks |
|---|---|---|---|
| 13.) | OH<br>$CF_3CH$ | H | |
| 14.) | OH<br>$HOCH_2-CH-$ | H | |
| 15.) | O<br>$PNBOCOCH_2CH_2$ | H | Protected as described for No. 3, Table II, Example 15. |
| 16.) | OH<br>$CH_3CH_2CH_2CH$ | H | |
| 17.) | OH<br>$CH_3CH_2CH$ | H | |
| 18.) | OH<br>$FCH_2CH$ | H | |

| Compound | $R^6$ | $R^7$ | Remarks |
|---|---|---|---|
| 19.) | 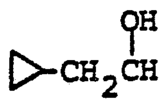 | H | |
| 20.) | $CH_3CH_2$ | H | |
| 21.) | $CH_3$ | $CH_3$ | |
| 22.) | 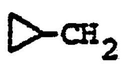 | H | |
| 23.) | $PNBOC(=O)OCH_2CH_2$ | $CH_3$ | Protected as described for No. 3, Table II, Example 15. |
| 24.) | 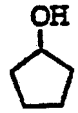 | H | |
| 25.) | $N_3CH_2CH(OH)$ | H | |
| 25a.) | $ClCH_2CH(OH)-$ | H | |
| 25b.) | $ClCH_2CH(OH)-$ | $CH_3$ | |

| Compound | $R^6$ | $R^7$ | Remarks |
|---|---|---|---|
| 26.) | $PNBO\overset{O}{\overset{\|}{C}}CH_2$ | H | |
| 27.) | $MeOCH_2\overset{OH}{\overset{\|}{C}}H$ | H | |
| 28.) | $CF_2CH\overset{OH}{\overset{\|}{C}}H$ | H | |
| 29.) | $PNBO\overset{O}{\overset{\|}{C}}OCH_2$ | H | |

EXAMPLE 16

Following the foregoing Examples and text, the following compounds are prepared in representative demonstration of the disclosed process. In the following Table, the resulting compounds are taken from starting materials which are made available by the foregoing text and examples -- particularly Table II of Example 15. The column labelled "Remarks and Reagents" annotates the established procedure where necessary to obtain the indicated compound. In most instances the compounds are deblocked according to the procedure described in Example 12. However, when the $SR^8$ side chain does not contain a basic function, the final product I is more conveniently isolated as the sodium salt (M=Na); which result is facilitated by conducting the deblocking in a slight excess of $NaHCO_3$. In any event, when either $R^6$ or $R^7$ bears a basic group, the final product I is most conveniently isolated as the free acid (M=H), rather than the sodium salt. It should be noted that compounds designated as "free acids" in reality are isolated as inner salts as a consequence of their zwitterionic nature.

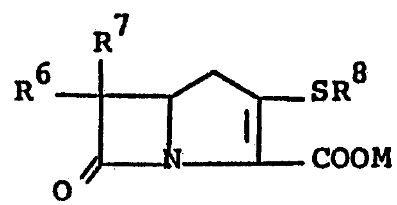

M = H, Na

TABLE III

| Compound | $R^6$ | $R^7$ | $R^8$ | Remarks, Reagents |
|---|---|---|---|---|
| 1.) | $(CH_3)_2CH$ | H | Ø | As in Example 11 ,but substitute HSØ for $HSCH_2CH_2NHCO_2PNB$. Deblock as described in Example 12 and isolate product as Na salt. M=Na. |
| 2.) | $CH_3$ | H | $CH_2$Ø | $HSCH_2$Ø; M=Na |
| 3.) | $HOCH_2$ | $CH_3$ | $CH_2CH_2CH_2NH_2$ | $HSCH_2CH_2CH_2NHCO_2PNB$; M = H. |
| 4.) | Ø$CH_2CH(OH)$ | H | $CH_2C(CH_3)_2NH_2$ | $HSCH_2C(CH_3)_2NHCO_2PNB$ ; M=H. |

Ø = phenyl

| Compound | $R^6$ | $R^7$ | $R^8$ | Remarks, Reagents |
|---|---|---|---|---|
| 5.) | OH<br>$CH_3CH$ | $CH_3$ | $CH_2CH_2NH_2$ | M = H |
| 6.) | OH<br>$CH_3CH$ | OH<br>$CH_3CH$ | $CH_2CH_2NH_2$ | M = H |
| 7.) | OH<br>$CH_3CH$ | $\emptyset CH_2$ | $CH_2CH_2N(CH_3)_2$ | $HSCH_2CH_2N(CH_3)_2$; M = H |
| 8.) | $NH_2$<br>$CH_3CH$ | H | $CH_2CH_2NH_2$ | M = H |
| 9.) | OH<br>$(CH_3)_2CHCH$ | H | 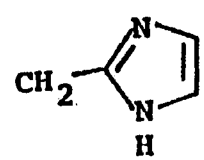 | 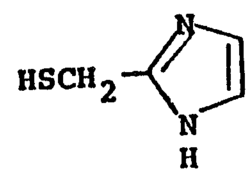 ; M = Na |

| Compound | $R^6$ | $R^7$ | $R^8$ | Remarks, Reagents |
| --- | --- | --- | --- | --- |
| 10.) | 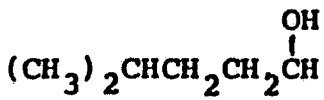 | H | 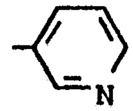 | 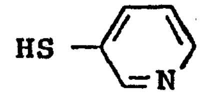 ; M = H |
| 11.) | 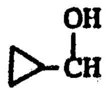 | H | 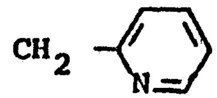 | 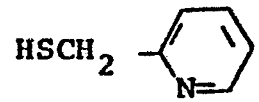 ; M = H |
| 12.) | 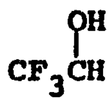 | H | $CH_2CH_2NH_2$ | M = H |
| 13.) | 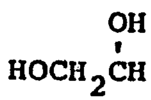 | H | $CH_2CH_2NH_2$ | M = H |
| 14.) | $HOCH_2CH_2$ | H | 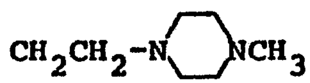 | 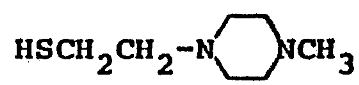 ; M = H |

| Compound | $R^6$ | $R^7$ | $R^8$ | Remarks, Reagents |
|---|---|---|---|---|
| 15.) | 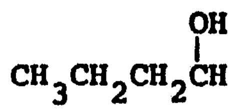 | H | 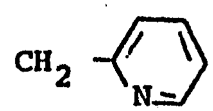 | 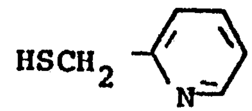 ; M = H |
| 16.) | 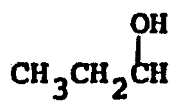 | H | 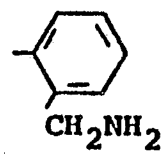 | 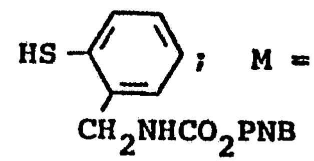 ; M = H |
| 17.) | 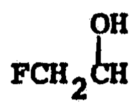 | H | $CH_2CH_2NH_2$ | |
| 18.) | 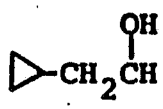 | H | $CH_2CH_2CO_2H$ | $HSCH_2CH_2CO_2PNB$; Product isolated as disodium salt. |
| 19.) | $CH_3CH_2$ | H | $CH_2CH_2NH_2$ | |

| Compound | $R^6$ | $R^7$ | $R^8$ | Remarks, Reagents |
| --- | --- | --- | --- | --- |
| 20.) | $CH_3$ | $CH_3$ | 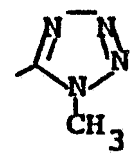 | 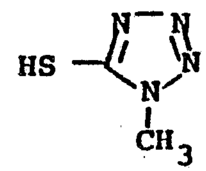 ; M = Na |
| 21.) | 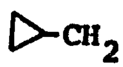 | H | $CH_2CH_2OH$ | $HSCH_2CH_2OH$; M = Na |
| 22.) | $HOCH_2CH_2$ | $CH_3$ | $CH_2CH_2CH_2CH_2NH_2$ | $HSCH_2CH_2CH_2CH_2NHO_2PNB$; M = H |
| 23.) | 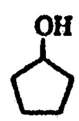 | H | $CH_2C(CH_3)_2CH_2NH_2$ | $CH_2C(CH_3)_2CH_2NHCO_2PNB$; M = H |

| Compound | $R^6$ | $R^7$ | $R^8$ | Remarks, Reagents |
|---|---|---|---|---|
| 24.) | 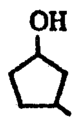 | H | $CH_2CH_2NH_2$ | M = Na |
| 25.) | 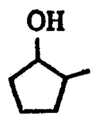 | H | $CH_2CH_2NH_2$ | M = Na |
| 26.) | 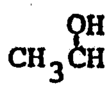 | H | 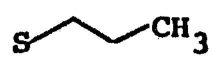 | 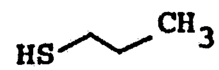 ; M=Na |
| 27.) | " | H | 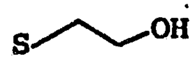 | 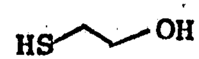 ; M=Na |
| 28.) | " | H | 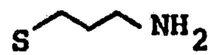 | 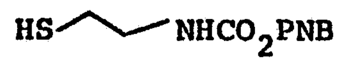 ; M=H |
| 29.) | " | H | 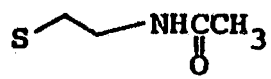 | 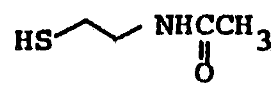 ; M=Na |

| Compound | $R^6$ | $R^7$ | $R^8$ | Remarks, Reagents |
|---|---|---|---|---|
| 30.) | $CH_3\overset{OH}{CH}$ | H | 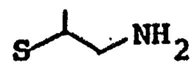 | 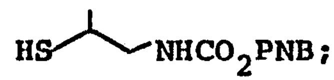; M=H |
| 31.) | " | H | 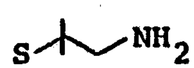 | 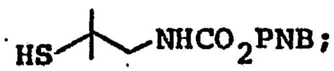; M=H |
| 32.) | " | H | 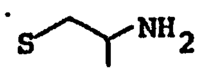 | 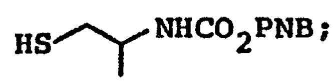; M=H |
| 33.) | " | H | 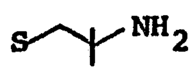 | 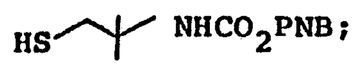; M=H |
| 34.) | " | H | 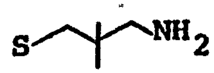 | 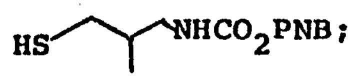; M=H |
| 35.) | " | H | 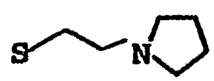 | 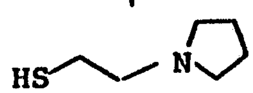; M=H |

| Compound | $R^6$ | $R^7$ | $R^8$ | Remarks, Reagents |
| --- | --- | --- | --- | --- |
| 36.) | $CH_3\overset{OH}{CH}$ | H | 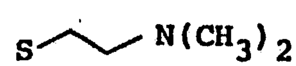 | 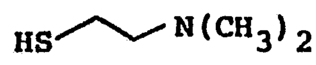; M=H |
| 37.) | " | H | 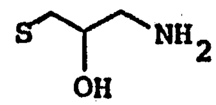 | 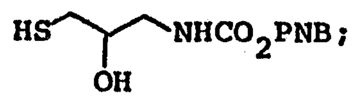; M=H |
| 38.) | " | H | 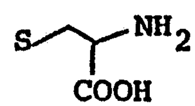 | 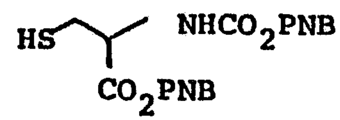; M=H |
| 39.) | " | H | 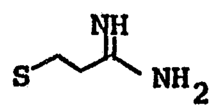 | 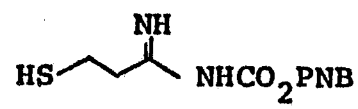; M=H |
| 40.) | " | H | 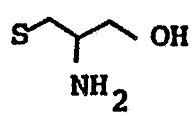 | 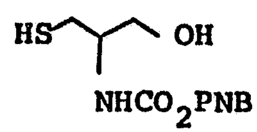; M=H |

| Compound | $R^6$ | $R^7$ | $R^8$ | Remarks, Reagents |
|---|---|---|---|---|
| 41.) | 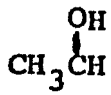 | H | 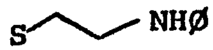 | M=H |
| 42.) | " | H | 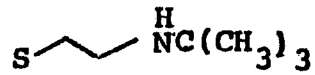 | 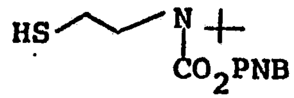 M=H |
| 43.) | " | H | SØ | M=Na |
| 44.) | " | H | 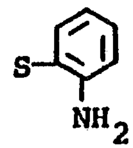 | 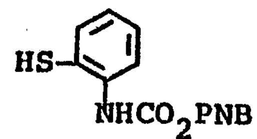 M=H |
| 45.) | " | H | 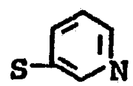 ; | 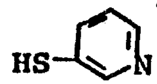 ; M=H |

| Compound | $R^6$ | $R^7$ | $R^8$ | Remarks, Reagents |
|---|---|---|---|---|
| 46.) | $CH_3CH(OH)$ | H | 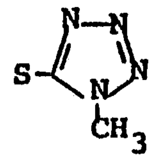 | M=Na |
| 47.) | " | H | 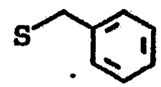 | M=Na |
| 48.) | " | H | 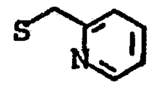 | M=H |
| 49.) | " | H | 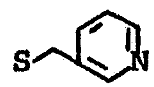 | M=H |
| 50.) | " | H | 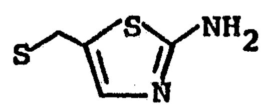 | 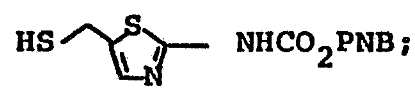 ; M=H |
| 51.) | " | H | 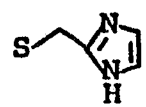 | M=Na |
| 52.) | " | H | 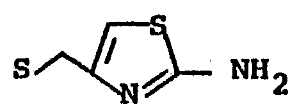 | 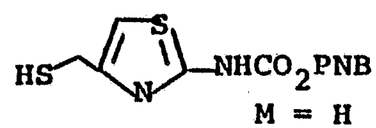 M = H |

| Compound | $R^6$ | $R^7$ | $R^8$ | Remarks, Reagents |
|---|---|---|---|---|
| 53.) | $CH_3\overset{OH}{CH}$ | H | 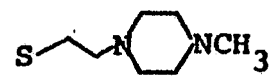 | M=H |
| 54.) | " | H | 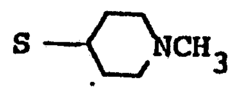 | M=H |
| 55.) | " | H | 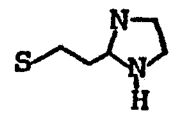 | 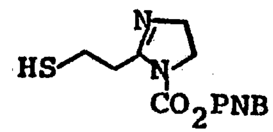 ; M=H |
| 56.) | " | H | 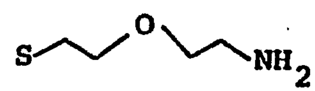 | 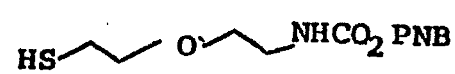 M=H |
| 57.) | " | H | 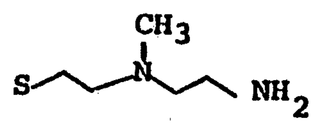 | 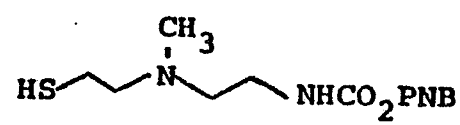 ; M=H |

| Compounds | |
|---|---|
| 58-89 | Compounds 58-89 correspond sequentially to compounds 26-57, above, except that the value for $R^6$ is taken as $CH_3CH_2$ rather than the $CH_3C(OH)H$ of Compounds 26-57. |
| 90-121 | Compounds 90-121 correspond sequentially to compounds 26-57, above, except that the value for $R^6$ is taken as $Cl_2CH\overset{OH}{C}H$ rather than the $CH_3C(OH)H$ of Compounds 26-57. |
| 122-153 | Compounds 122-153 correspond sequentially to compounds 26-57, above, except that the value for $R^6$ is taken as $CF_3\overset{OH}{C}H$ rather than the $CH_3C(OH)H$ of Compounds 26-57. |
| 154-185 | Compounds 154-185 correspond sequentially to compounds 26-57, above, except that the value for $R^6$ is taken as $HOCH_2\overset{OH}{C}H$ rather than the $CH_3C(OH)H$ of Compounds 26-57. |
| 186-217 | Compounds 186-217 correspond sequentially to compounds 26-57, above, except that the value for $R^6$ is taken as $ClCH_2\overset{OH}{C}H$ rather than the $CH_3C(OH)H$ of Compounds 26-57. |

| Compounds | |
|---|---|
| 218-249 | Compounds 218-249 correspond sequentially to compounds 26-57, above, except that the value for $R^6$ is taken as $CH_3CH_2\overset{OH}{C}H$ rather than the $CH_3C(OH)H$ of Compounds 26-57. |
| 250-281 | Compounds 250-281 correspond sequentially to compounds 26-57, above, except that the value for $R^6$ is taken as 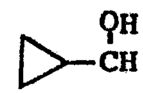 rather than the $CH_3C(OH)H$ of Compounds 26-57. |
| 282-313 | Compounds 282-313 correspond sequentially to compounds 26-57, above, except that the value for $R^6$ is taken as $H_2NCH_2\overset{OH}{C}H$ rather than the $CH_3C(OH)H$ of Compounds 26-57. |
| 314-345 | Compounds 314-345 correspond sequentially to compounds 26-57, above, except that the value for $R^6$ is taken as $CF_2H\overset{OH}{C}-$ rather than the $CH_3C(OH)H$ of Compounds 26-57. |

| Compounds | |
|---|---|
| 346-377 | Compounds 346-377 correspond sequentially to compounds 26-57, above, except that the value for $R^6$ is taken as $HOCH_2$ rather than the $CH_3C(OH)H$ of Compounds 26-57. |
| 378-409 | Compounds 378-409 correspond sequentially to compounds 26-57, above, except that the value for $R^6$ is taken as $HO_2CCH_2$ rather than the $CH_3C(OH)H$ of Compounds 26-57. |
| 410-441 | Compounds 410-441 correspond sequentially to compounds 26-57, above, except that the value for $R^6$ is taken as $CH_3OCH_2\overset{OH}{\overset{\vert}{CH}}$ rather than the $CH_3C(OH)H$ of Compounds 26-57. |
| 442-473 | Compounds 442-473 correspond sequentially to compounds 26-57, above, except that the value for $R^6$ is taken as $(CH_3)_3CCH_2\overset{OH}{\overset{\vert}{CH}}$ rather than the $CH_3C(OH)H$ of Compounds 26-57. |

Incorporation by Reference

The final antibiotic compounds of the present invention Structure I:

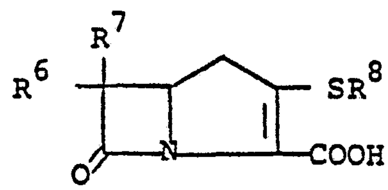

wherein $R^6$, $R^7$ and $R^8$ are defined above are fully disclosed and claimed in co-pending European Patent Application Serial Number 80 102 076 filed April 18, 1980; thus, to the extent that this co-pending U.S. Patent Application defines the compounds of Structure I, their synthesis and their utility as antibiotics, it is incorporated herein by reference.

The compounds of the present invention may also be prepared by the processes disclosed and claimed in the three (3) following, co-pending, commonly assigned U.S. Patent Applications of Christensen, Ratcliffe and Salzmann. To the extent that these applications define $R^6$, $R^7$, and $R^8$ of Structure I and to the extent that they describe processes for the synthesis of intermediates III and IV (defined above) they are hereby incorporated by reference.

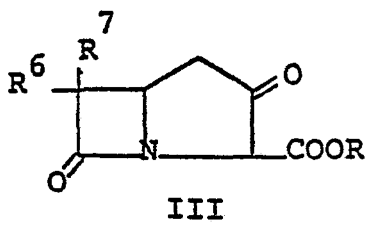

III

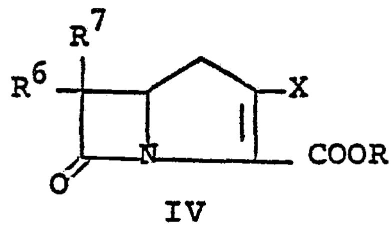

IV

wherein $R^6$, $R^7$ and X are defined above.

1.) <u>Process for the Preparation of 1-Carbapenems and Intermediates via 4-Allylazetidinone;</u> U.S. Patent Application Serial Number , filed 3-27-80 [Merck & Co., Inc. Attorney's Docket Case 16479].

2.) <u>Process for the Preparation of 1-Carbapenems and Intermediates via Trithioorthoacetates;</u> U.S. Patent Application Serail Number , filed 3-27-80 [Merck & Co., Inc. Attorney's Docket Case 16485].

3.) <u>Process for the Preparation of 1-Carbapenems and Intermediates via Silyl-Substituted Dithioacetals;</u> U.S. Patent Application Serial Number , filed 3-27-80 [Merck & Co., Inc. Attorney's Docket Case 16478].

Also incorporated by reference is published European Patent Application 0007614 (Application Number 79102615.6, filed 24 July 1979). This application discloses certain dipeptidase inhibitors which, on co-administration to mammalian subjects, enhance the efficacy of certain 1-carbadethiapenem antibiotics. Thus, to the extent that the cited application: 1.)defines the manner by which susceptible carbadethiapenems substrates of the present invention may be identified; and 2.) discloses suitable inhibitors, compositions, and methods of treatment, it is incorporated herein by reference. A particularly preferred inhibitor is 6-(L-2-Amino-2-carboxyethylthio)-2-(2,2-DCC)-2-hexenoic acid.

Intermediates IV wherein X is halo are conveniently prepared from intermediates III by the procedures disclosed in co-pending, commonly assigned U.S. Patent Application Serial Number 26,979 filed April 4, 1979, which is incorporated herein by reference.

WHAT IS CLAIMED IS:

1. A process for preparing a compound having the structural formula:

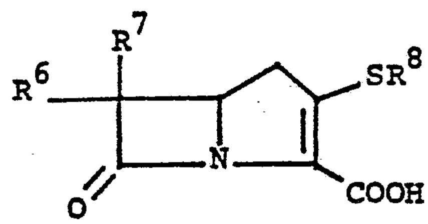

and the pharmaceutically acceptable salt, ester and amide derivatives thereof; wherein $R^6$, $R^7$ and $R^8$ are independently selected from the group consisting of: hydrogen; substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl, aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl; wherein the substituent or substituents relative to the above-named radicals are selected from the group consisting of: chloro, bromo, fluoro, $R^1$,

-OH

$-OR^1$

$-O\overset{O}{\overset{\|}{C}}NR^1R^2$

$-\overset{O}{\overset{\|}{C}}NR^1R^2$

$-NR^1R^2$

$-NH_2$

$-NHR^1$

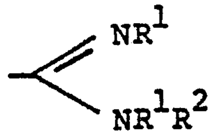

$-SO_2NR^1R^2$

$-NH\overset{O}{\overset{\|}{C}}NR^1R^2$

$-R^2N\overset{O}{\overset{\|}{C}}R^1$

$-CO_2H$

$-CO_2R^1$

$-\overset{O}{\overset{\|}{C}}R^1$

$-O\overset{O}{\overset{\|}{C}}R^1$

$-SH$

$-\overset{O}{\overset{\|}{S}}R^1$

$-\underset{O}{\underset{\|}{\overset{O}{\overset{\|}{S}}}}R^1$

$-CN$

$-N_3$

$-NO_2$

$-\overset{\oplus}{N}(R^1)_3$

$-\overset{R^1}{\overset{|}{C}}=NOR^2$

$-SR^1$ ;

wherein, relative to the above listed substituents on $R^6$, $R^7$ and $R^8$, the groups $R^1$ and $R^2$ are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl; and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms;

comprising activating and treating with $HSR^8$ a compound of the structure:

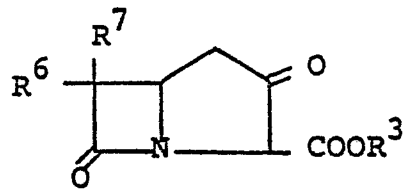

wherein $R^3$ is a protecting group or a pharmaceutically acceptable ester moiety; when $R^6/R^7$ is hydrogen and $R^7/R^6$ is $CH_3CH(OH)$, then $R^8$ is not $-CH_2CH_2NH_2$.

2. A process according to Claim 1 comprising treating:

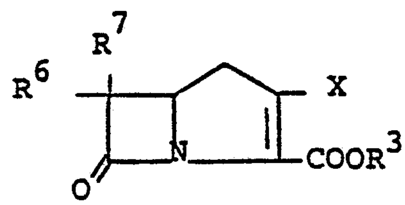

with $HSR^8$ wherein X is a leaving group.

3. A compound selected from:

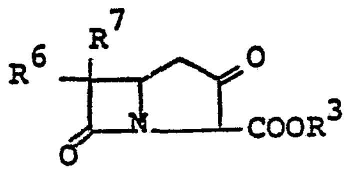 , and 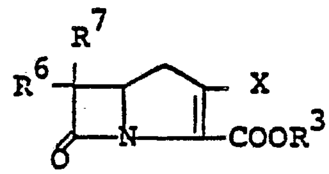

wherein X is a leaving group, $R^3$ is hydrogen, a salt cation, a protecting group, or a pharmaceutically acceptable ester moiety; and wherein $R^6$, $R^7$ and $R^8$ are independently selected from the group consisting of: hydrogen; substituted and unsubstituted: alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl, aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl; wherein the substituent or substituents relative to the above-named radicals are selected from the group consisting of:
chloro, bromo, fluoro, $R^1$,

$-OH$

$-OR^1$

$-O\overset{O}{\overset{\|}{C}}NR^1R^2$

$-\overset{O}{\overset{\|}{C}}NR^1R^2$

$-NR^1R^2$

$-NH_2$

$-NHR^1$

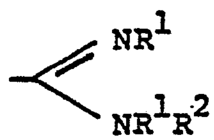

$-SO_2NR^1R^2$

$-NH\overset{O}{\overset{\|}{C}}NR^1R^2$

$-R^2N\overset{O}{\overset{\|}{C}}R^1$

$-CO_2H$

$-CO_2R^1$

$-\overset{O}{\overset{\|}{C}}R^1$

$-O\overset{O}{\overset{\|}{C}}R^1$

$-SH$

$-\overset{O}{\overset{\|}{S}}R^1$

$-\underset{\underset{O}{\|}}{\overset{O}{\overset{\|}{S}}}R^1$

$-CN$

$-N_3$

$-NO_2$

$-\overset{\oplus}{N}(R^1)_3$

$-\overset{R^1}{\overset{|}{C}}=NOR^2$

$-SR^1$ ;

wherein, relative to the above listed substituents on $R^6$, $R^7$ and $R^8$, the groups $R^1$ and $R^2$ are independently selected from: hydrogen, alkyl, alkenyl, and alkynyl, having from 1-10 carbon atoms; cycloalkyl, cycloalkylalkyl, and alkylcycloalkyl, having 3-6 carbon atoms in the cycloalkyl ring and 1-6 carbon atoms in the alkyl moieties; phenyl; aralkyl, aralkenyl, and aralkynyl wherein the aryl moiety is phenyl and the aliphatic portion has 1-6 carbon atoms; heteroaryl, heteroaralkyl, heterocyclyl and heterocyclylalkyl; and wherein the hetero atom or atoms in the above-named heterocyclic moieties are selected from the group consisting of 1-4 oxygen, nitrogen or sulphur atoms and wherein the alkyl moieties associated with said heterocyclic moieties have 1-6 carbon atoms; when $R^6/R^7$ is hydrogen, then $R^7/R^6$ is not $CH_3CH(OH)$.

4. A compound according to Claim 3, wherein $R^7$ is selected from H, $OCH_3$ and $CH_3$.

5. A compound according to Claim 4 wherein $R^6$ is selected from the group consisting of: substituted and unsubstituted: alkyl, alkenyl and cycloalkylalkyl wherein the substituent or substituents are selected from hydroxyl, alkoxyl having from 1-6 carbon atoms, phenoxy, amino, and carboxy.

6. A compound according to Claims 4 or 5 wherein $R^6$ is selected from the group consisting of alkyl, cycloalkylalkyl, alkyl substituted by one or more hydroxyl groups, or cycloalkylalkyl substituted by one or more hydroxyl groups.

7. A compound according to Claims 4, 5 or 6 wherein $R^7$ is hydrogen.

8. A compound according to Claims 3, 4, 5, 6, or 7 wherein $R^6$ is selected from:

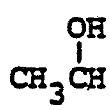

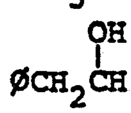 Ø = phenyl

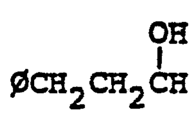

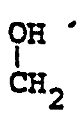

$ØCH_2$

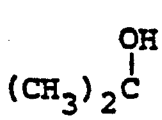

$CH_2{=}CHCH_2$

$(CH_3)_2C{=}CHCH_2$

OH
$CH_3OCH_2CH$

$CH_3$

$CH_3CH_2$

$(CH_3)_2CH$

OH
$N_3CH_2CH$

OH
$(CH_3)_2NCH_2CH$

$HO_2CCH_2$

OH
$CF_3CF_2CH$

OH
$HO_2CCH_2CH$

OH
$CH_3CH(CH_3)CH$

OH
$(CH_3)_2CHCH_2CH$

OH
$CH_3CH_2CH$

$HOCH_2CH_2$

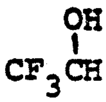

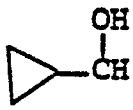

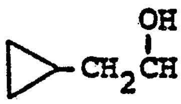

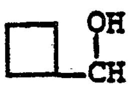

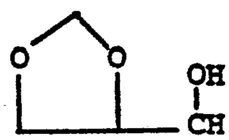

$HOCH_2CH_2CH_2$

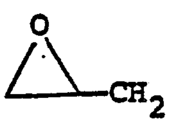

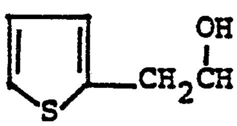

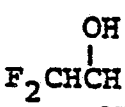

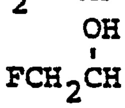

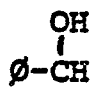

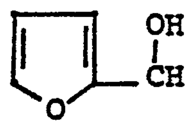

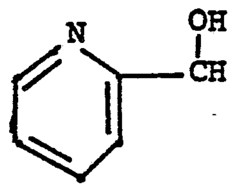

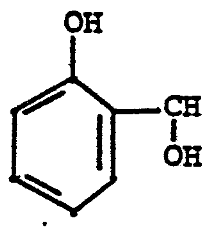

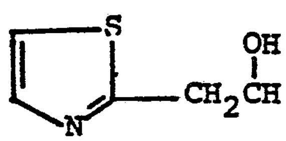

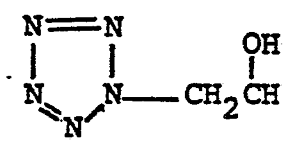

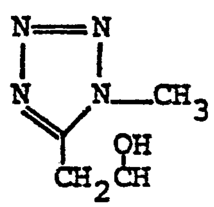

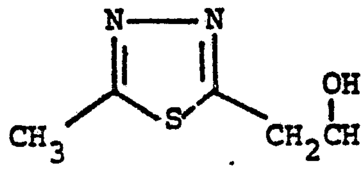

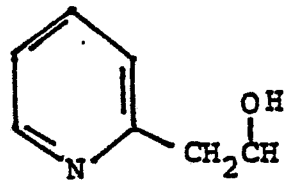

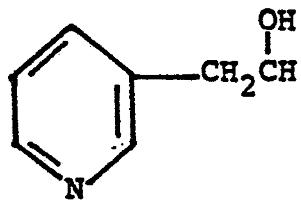

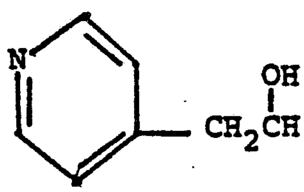

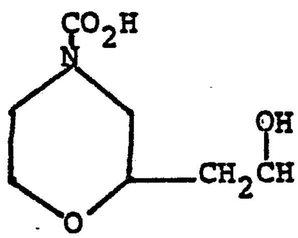

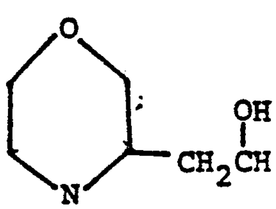

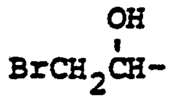

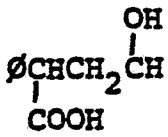

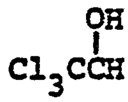

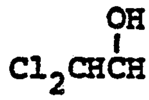

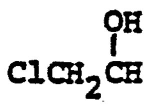

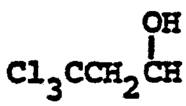

9. A compound according to Claim 3 wherein X is

chloro,
bromo,
iodo,
phenylsulfonyloxy,
p-tôluylsulfonyloxy,
p-nitrophenylsulfonyloxy,
methylsulfonyloxy,
trifluoromethylsulfonyloxy,
diphenylphosphoryl,
bis(trîchloroethyl)phosphoryl.

10. A compound according to Claims 3, 4 or 7 selected from: H, $-\left[\underset{X}{\overset{|}{CH}}\right]_y-(R)_n-R^{1^\circ}$

wherein: y = 0 or 1;
X = OH, $NH_2$; SH;
n = 0 or 1;
R = substituted or unsubstituted: alkyl, alkenyl or alkynyl having 1-6 carbon atoms wherein the substituent is $R^1$;
$R^{1^\circ}$ = alkoxyl, carboxyl, $CF_3$, OH, H, linear or branched alkyl bearing 1 or more hydroxyl groups, amino, aminoalkyl, Cl, F, Br, alkylthio, amidino, guanidino, oximino, phenyloxy, phenylthio,

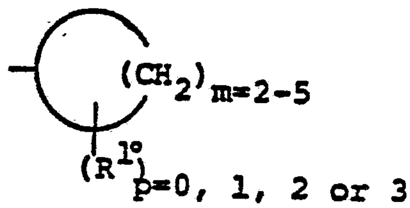

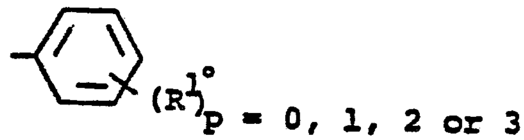

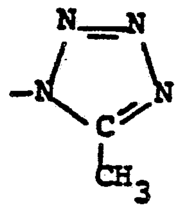

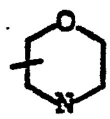

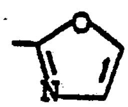

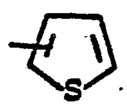

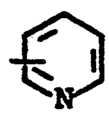

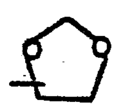

11. A process according to Claims 1 or 2 wherein $R^8$ is selected from:

1.) aliphatic (including carbocyclic) groups having 1-10 carbon atoms selected from: alkyl, cycloalkyl, alkenyl, cycloalkenyl and alkynyl;

2.) substituted aliphatic groups having 1-10 carbon atoms selected from: alkyl, cycloalkyl, alkenyl, cycloalkenyl and alkynyl; wherein the substituents are selected from:

chloro, bromo, fluoro, $R^1$,

$-OH$

$-OR^1$

$-O\overset{O}{\overset{\|}{C}}NR^1R^2$

$-\overset{O}{\overset{\|}{C}}NR^1R^2$

$-NR^1R^2$

$-NH_2$

$-NHR^1$

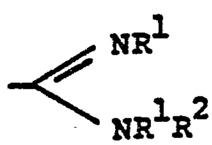

$-SO_2NR^1R^2$

$-NH\overset{O}{\overset{\|}{C}}NR^1R^2$

$-R^2N\overset{O}{\overset{\|}{C}}R^1$

$-CO_2H$

$-CO_2R^1$

$-\overset{O}{\overset{\|}{C}}R^1$

$-O\overset{O}{\overset{\|}{C}}R^1$

$-SH$

$-\overset{O}{\overset{\|}{S}}R^1$

$-\underset{\underset{O}{\|}}{\overset{O}{\overset{\|}{S}}}R^1$

$-CN$

$-N_3$

$-NO_2$

$-\overset{\oplus}{N}(R^1)_3$

$-\overset{R^1}{\overset{|}{C}}=NOR^2$

$-SR^1$

3.) aryl and substituted aryl; wherein the aryl is phenyl and wherein the substituents are defined above under 2.);

4.) heteroaryl and substituted heteroaryl having 1-4 O, N or S atoms; and wherein the substituents are defined above under 2.);

5.) arylaliphatic, wherein aryl is phenyl, which are selected from the aliphatic groups defined under 1.) which are substituted by phenyl or substituted phenyl; wherein such substituents on phenyl are defined under 2.), above;

6.) heteroarylaliphatic and heterocyclylaliphatic; wherein the aliphatic moiety is defined under 1.), above; the substituted and unsubstituted heteroaryl and heterocyclic moieties have 1-4 O, N or S atoms; wherein such substituents are defined under 2.),above;

7.) substituted and unsubstituted alkyl-heteroatom-alkyl having 4-12 carbon atoms; wherein the heteroatom is selected from: O, S, NR° (R° is H, substituted or unsubstituted alkyl); wherein such substituents are defined under 2.),above.

12. A process according to Claim 11, sub-paragraph 1.); wherein $R^8$ is selected from:

$-CH_3$

$-CH_2CH_3$

$-CH_2CH_2CH_3$

$-CH(CH_3)_2$

$-(CH_2)_3CH_3$

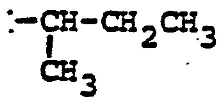

$-CH_2CH(CH_3)_2$

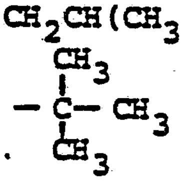

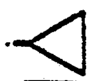

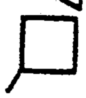

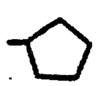

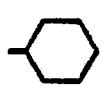

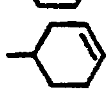

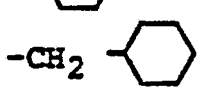

$-CH_2-CH=CH_2$

$-CH_2-CH=C(CH_3)_2$

$-CH_2-C\equiv CH$

$-CH_2-C\equiv C-CH_3$

13. A process according to Claim 11, sub-paragraph 2.); wherein $R^8$ is selected from:

$-(CH_2)_nOR^1$ ; $n = 2\text{-}6$; $R^1 = H$, $\overset{O}{\overset{\|}{C}}CH_3$, $CH_3$

$-(CH_2)_n\overset{O}{\overset{\|}{C}}XR^1$; $n = 1\text{-}6$; $X = O$, $NH$, $NR^1$, $R^1 = H$, $CH_3$

$-(CH_2)_nNH_2$ ; $n = 2\text{-}6$

$-(CH_2)_nNHR^1$ ; $n = 2\text{-}6$; $R^1 = CH_3$, $CH_2CH_3$, $CH_2CH_2CH_3$, $\overset{O}{\overset{\|}{C}}CH_3$

$-(CH_2)_nNR^1R^2$ ; $n = 2\text{-}6$; $R^1/R^2 = CH_3$, $CH_2CH_3$; $CH_3$, $CH_3$; $CH_2CH_3$, $CH_2CH_3$

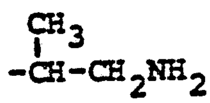

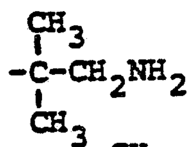

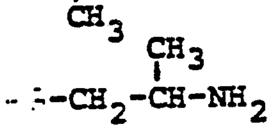

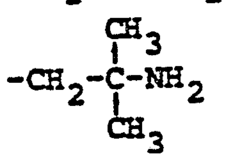

$-CH_2CH_2SCH_3$

$-CH_2CH_2NHC(CH_3)_3$

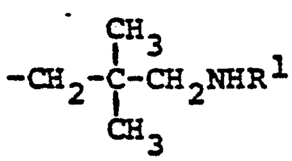 $R^1 = H$, $CH_3$, $\overset{O}{\overset{\|}{C}}CH_3$

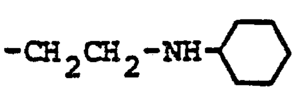

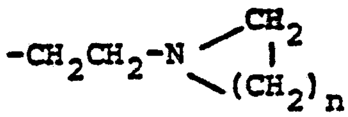 $n=3-5$

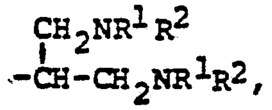 $R^1$ and $R^2$ are independently chosen from H and $CH_3$

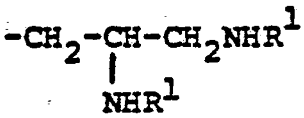 $R^1$=H, $CH_3$

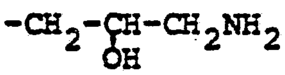

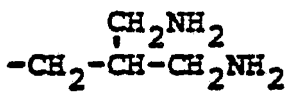

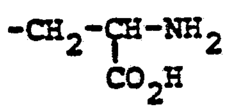

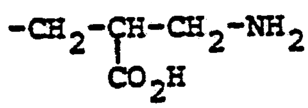

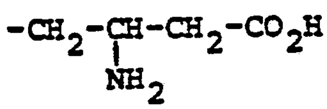

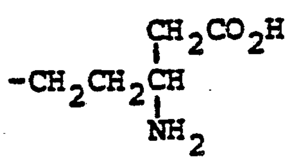

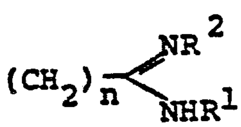 n=1, $R^2/R^1$=H,H; $CH_3$,H

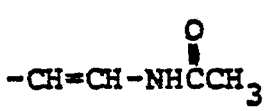

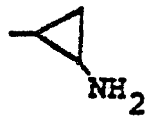

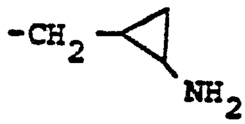

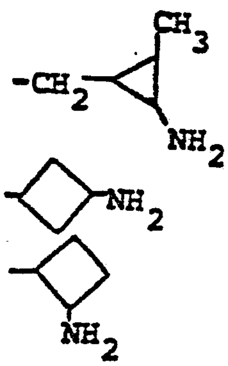

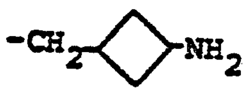

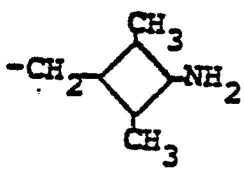

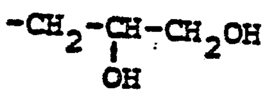

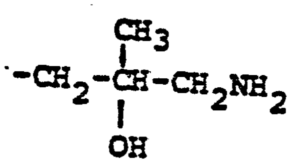

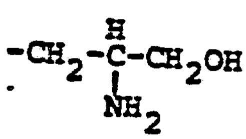

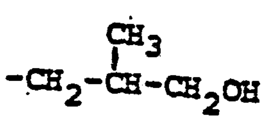

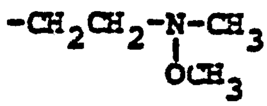

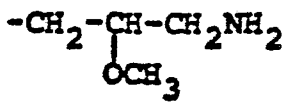

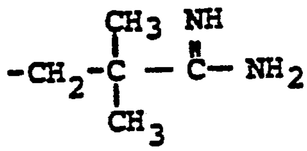

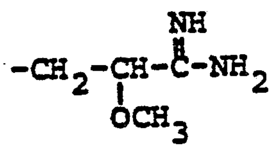

$-CH_2-CH=CH-CH_2NH_2$

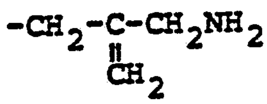

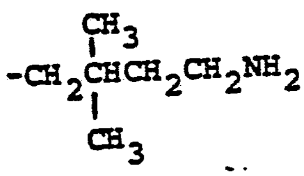

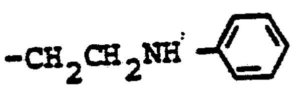

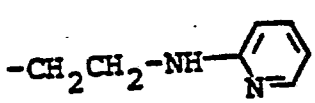

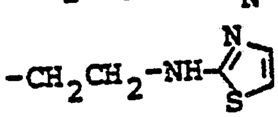

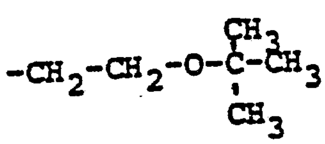

14. A process according to Claim 11, sub-paragraph 3.); wherein $R^8$ is selected from:

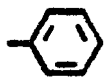

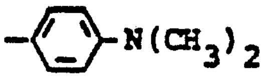

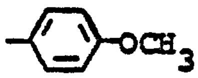

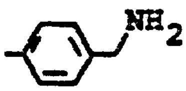

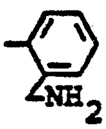

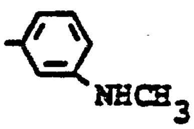

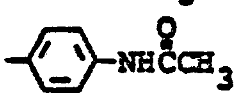

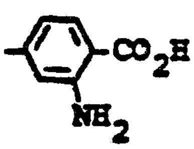

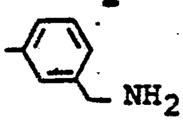

15. A process according to Claim 11, subparagraph 4.), wherein $R^8$ is:

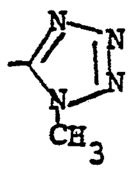

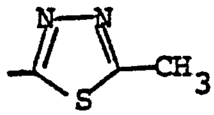

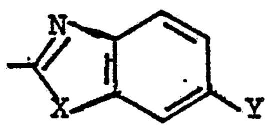

X=N,O Y=H

X=S Y=H, Cl, $OCH_2CH_3$

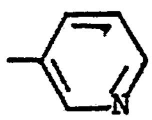

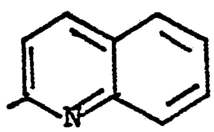

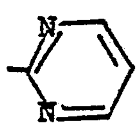

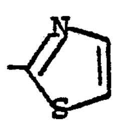

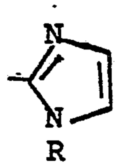

R=H, $CH_3$

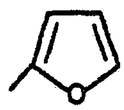

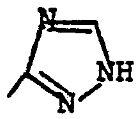

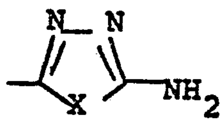 X=NH, S

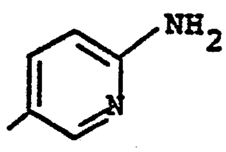

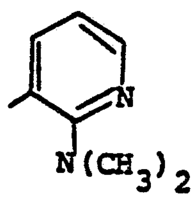

16. A process according to Claim 11, subparagraph 5.); wherein $R^8$ is:

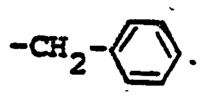

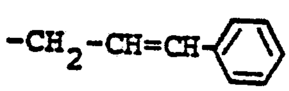

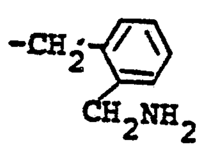

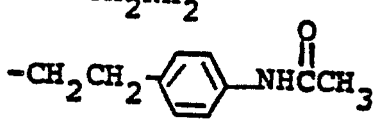

17. A process according to Claim 11, subparagraph 6.); wherein $R^8$ is:

$-(CH_2)_n-$ 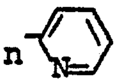

$-(CH_2)_n-$ 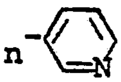

$-(CH_2)_n-$ 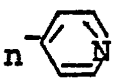

$-(CH_2)_n-$ 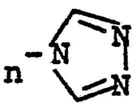

$-(CH_2)_n-$ 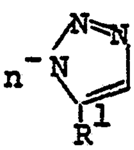 $R^1 = OCH_2CH_3$

$-(CH_2)_n-$ 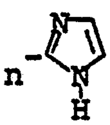

$-(CH_2)_n-$ 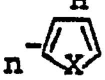

$-(CH_2)_n-$ 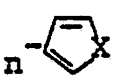 X = O, S, NH

$-(CH_2)_n-$ 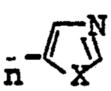 X = O, S, NH

$-(CH_2)_n-$ 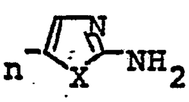 X = O, S, NH

$-(CH_2)_n-$ 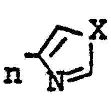 X = O, S, NH

$-(CH_2)_n-$ 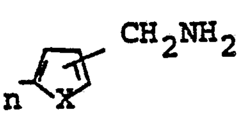 X = O, S, NH

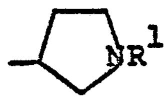

$R^1$=H, $CH_3$

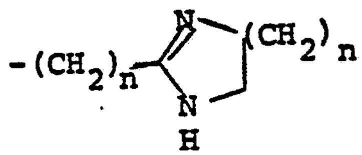

m = 1-3
n = 1-3

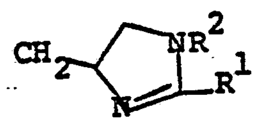

$R^2$=H, $CH_3$, $R^1$=H, $CH_3$, $NH_2$

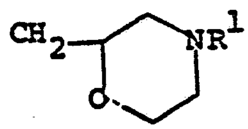

$R^1$= H, $CH_3$

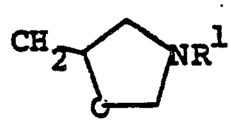

$R^1$=H, $CH_3$

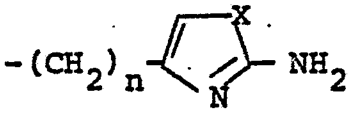 $X = O, S, NH$

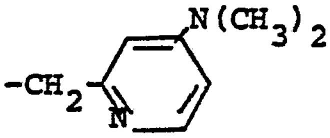

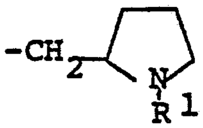 $R^1 = H, CH_3$

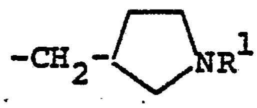 $R^1 = H, CH_3$

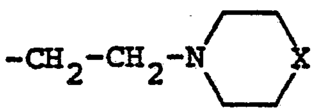 $X = O, NH, NCH_3$

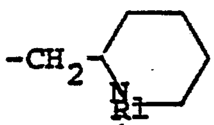 $R^1 = H, CH_3$

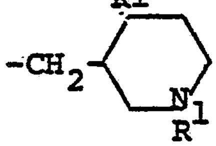 $R^1 = H, CH_3$

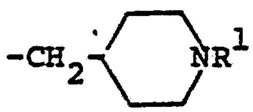 $R^1 = H, CH_3$

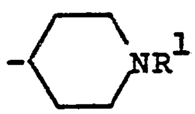 $R^1 = H, CH_3$

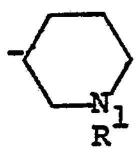 $R^1 = H, CH_3$

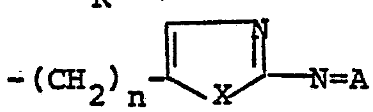 , $X = O, S, NH$

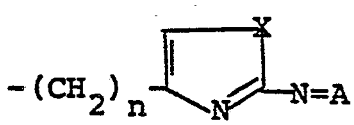 , $X = O, S, NH$

18. A process according to Claim 11, sub-paragraph 7.); wherein $R^8$ is:

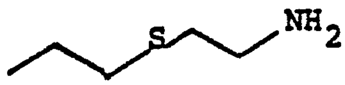

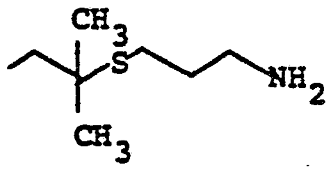

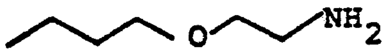

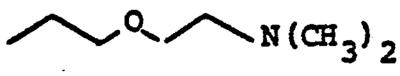

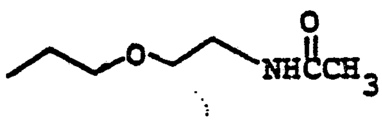

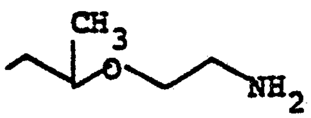

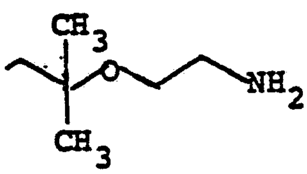

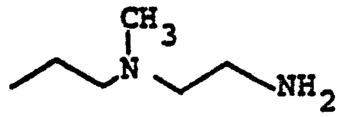

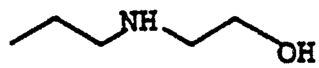

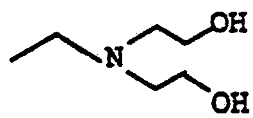

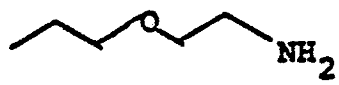

19. A process according to Claim 1 or 2 for preparing:

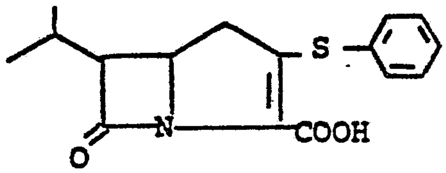

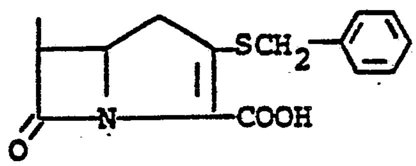

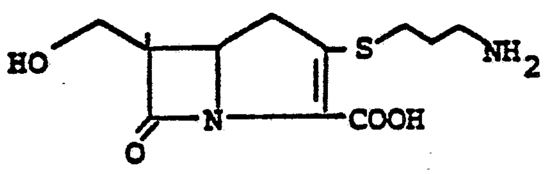

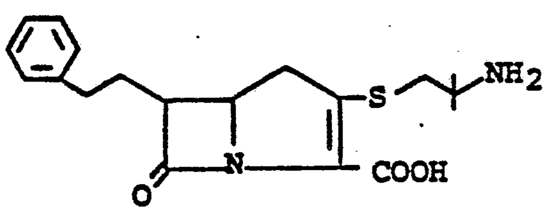

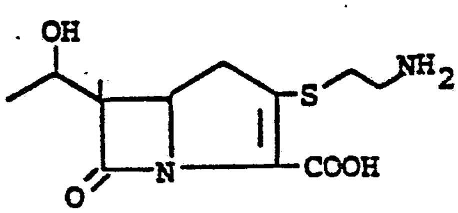

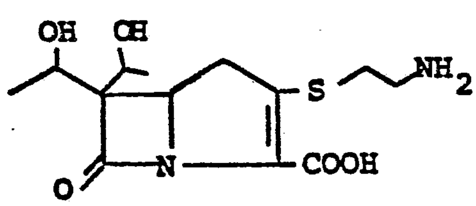

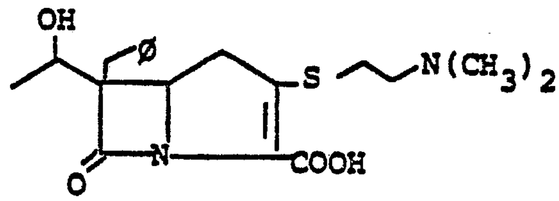

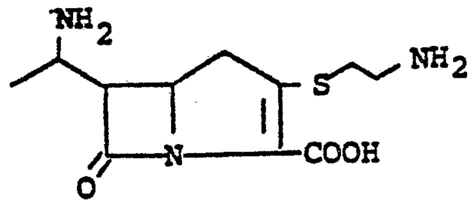

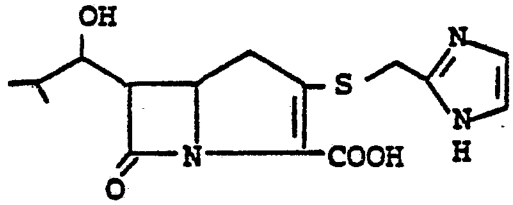

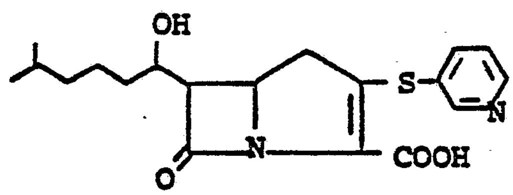

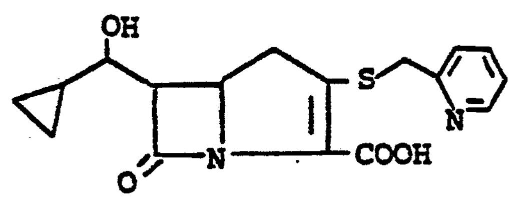

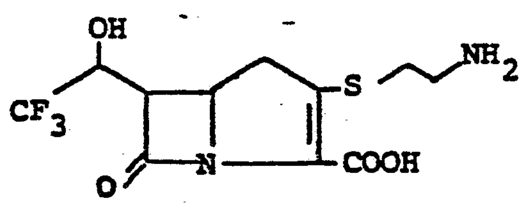

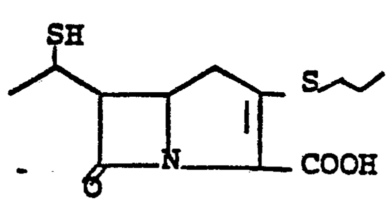

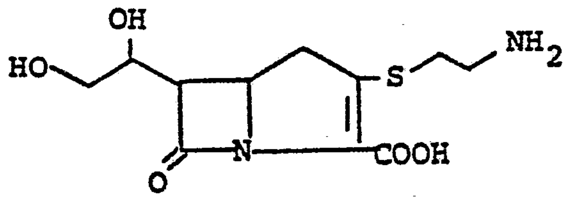

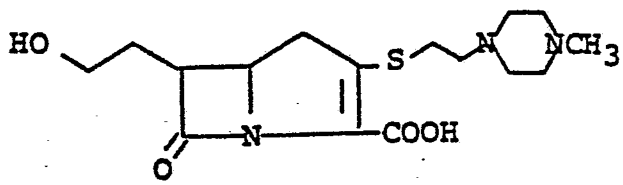

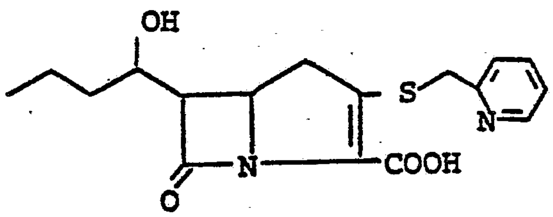

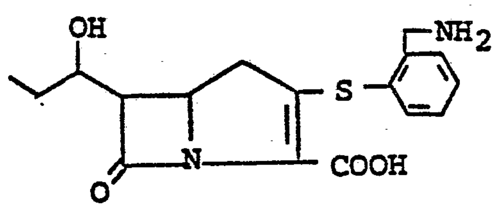

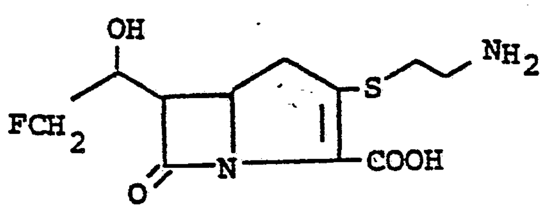

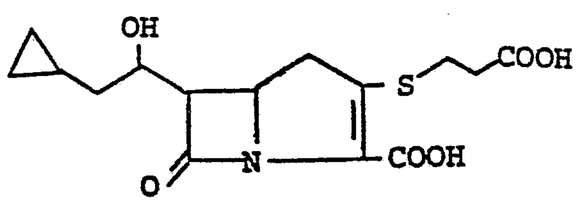

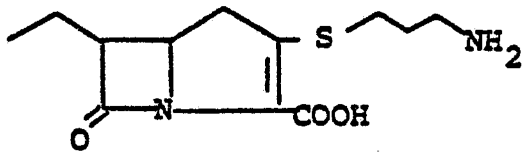

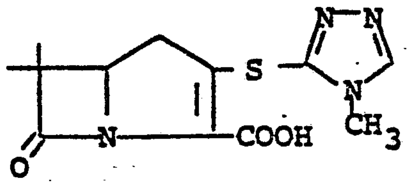

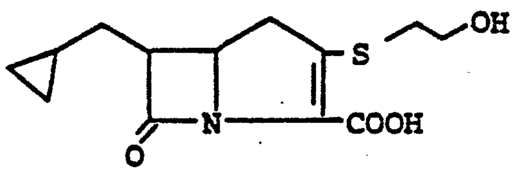

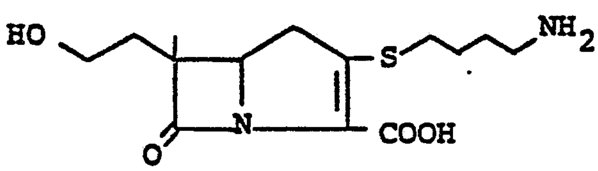

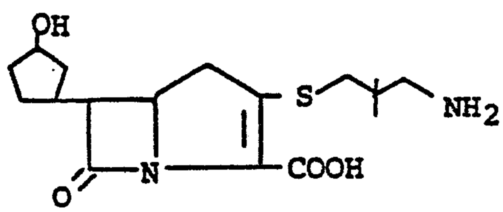

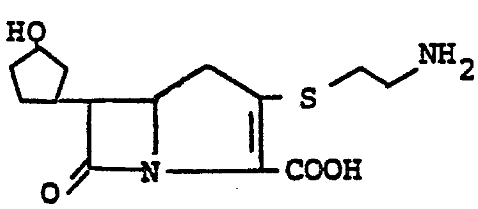

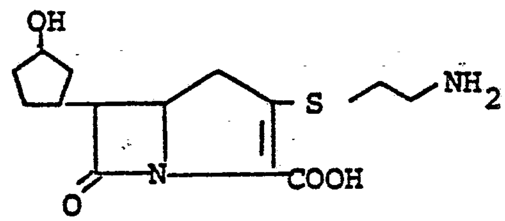

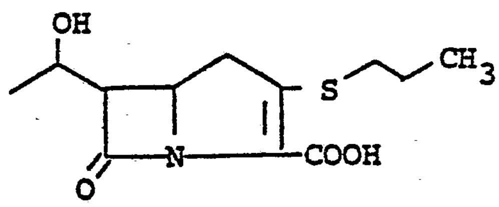

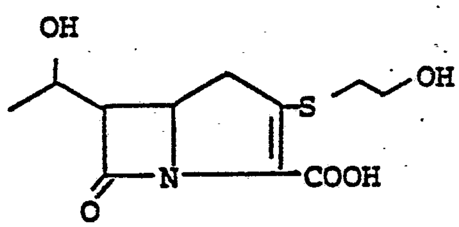

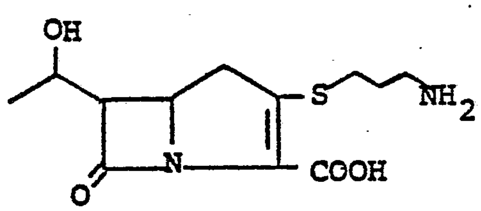

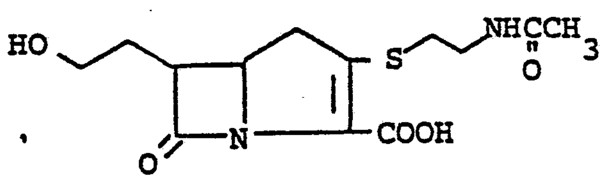

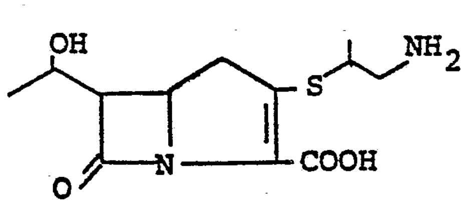

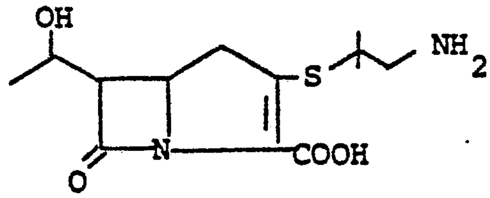

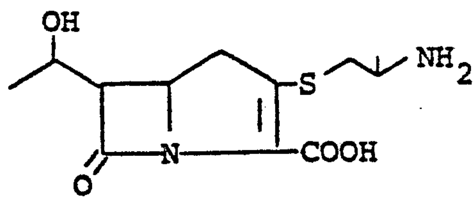

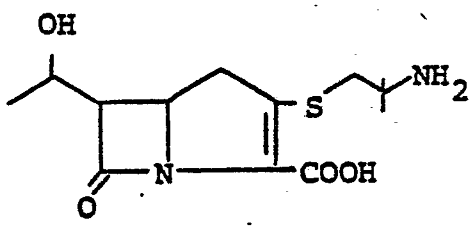

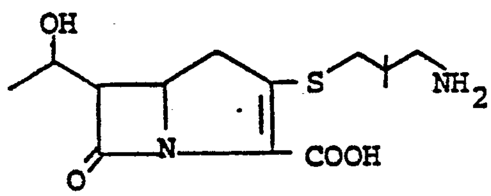

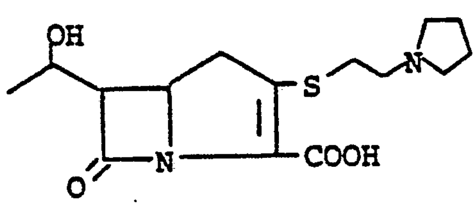

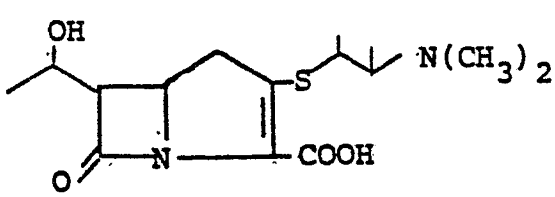

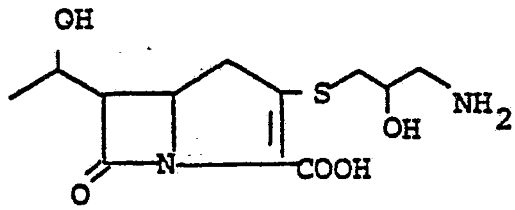

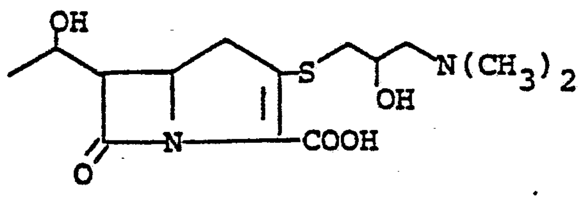

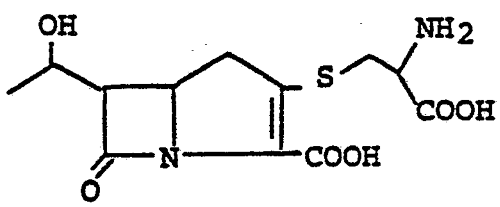

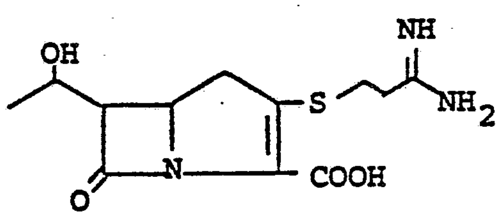

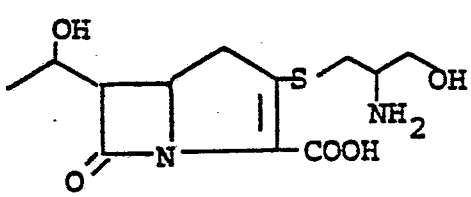

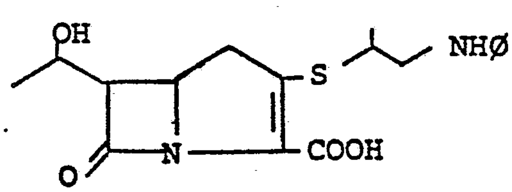

Ø = phenyl

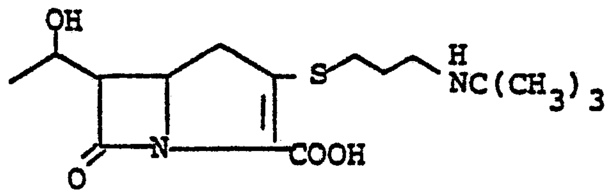

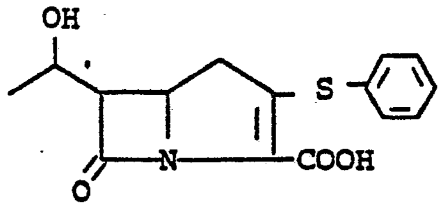

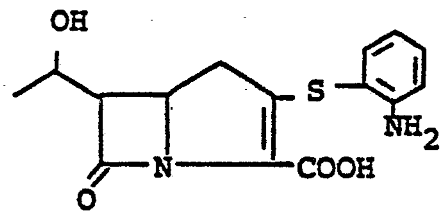

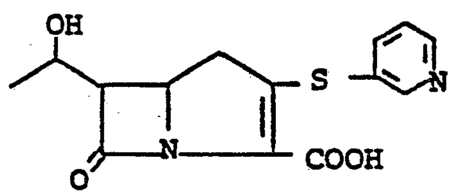

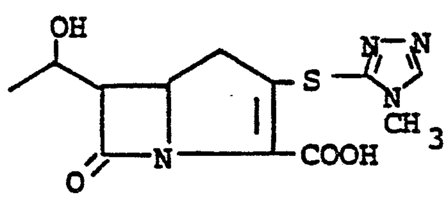

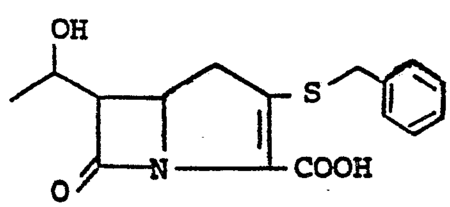

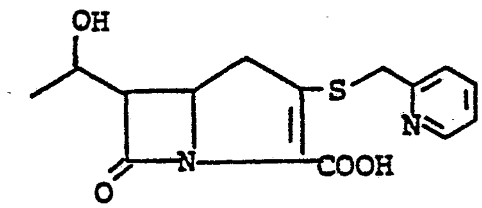

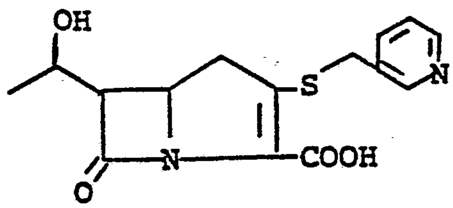

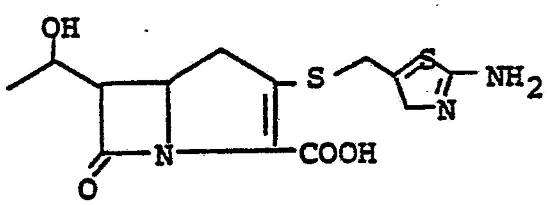

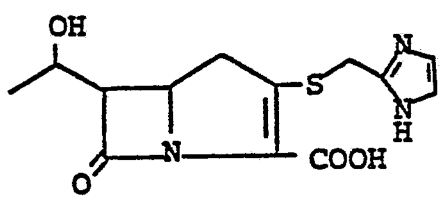

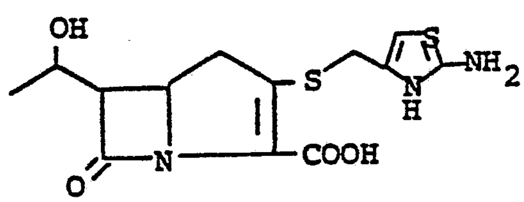

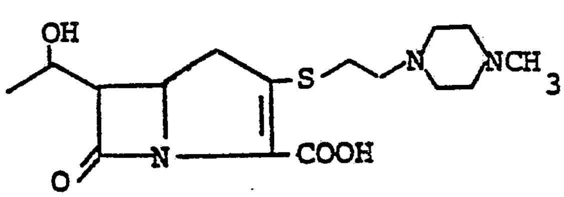

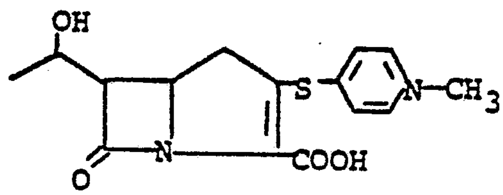

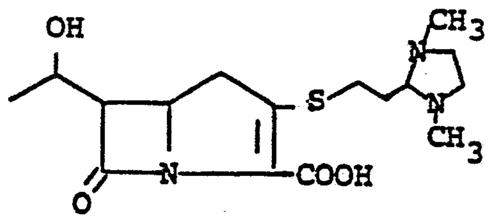

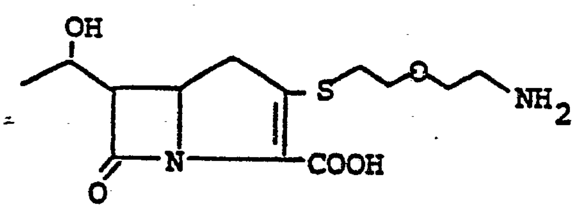

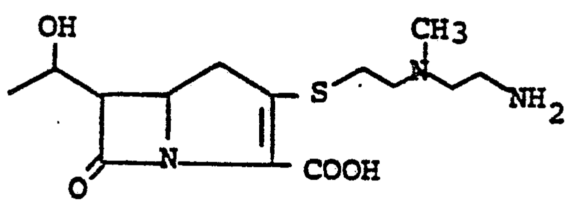

$-CH_2CH_2NH_2$

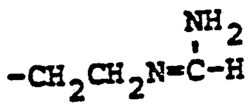

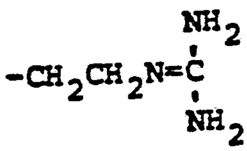

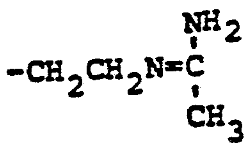

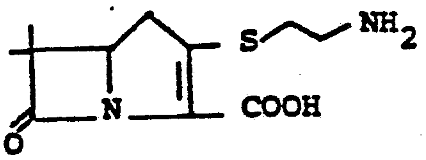

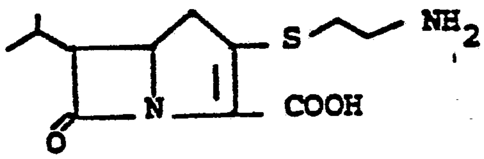

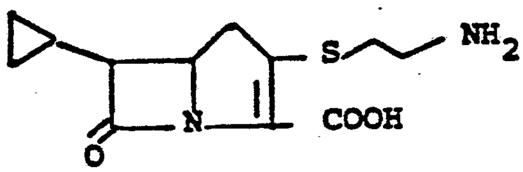

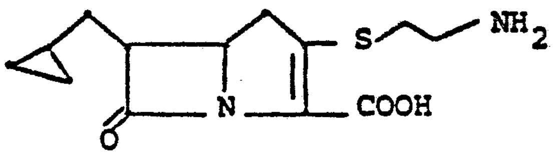

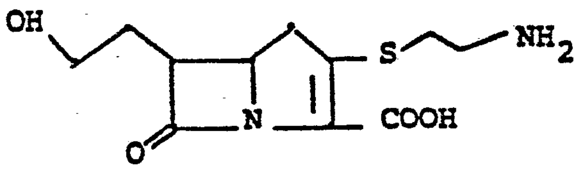

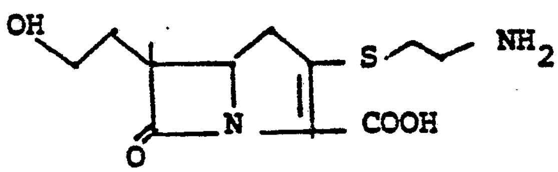

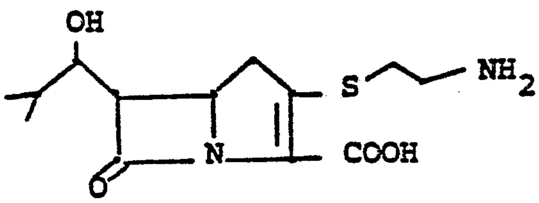

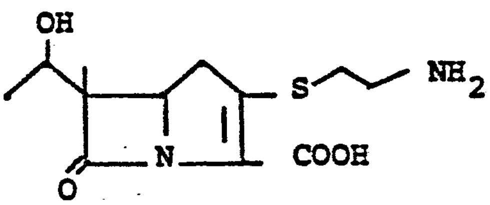

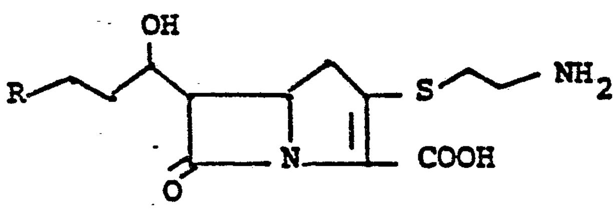

(R = phenyl, m-aminomethylphenyl, o-, p-, m-hydroxyphenyl)

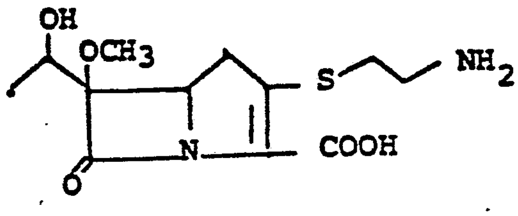

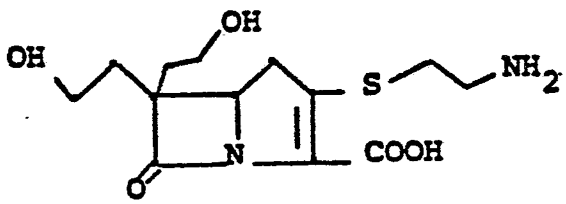

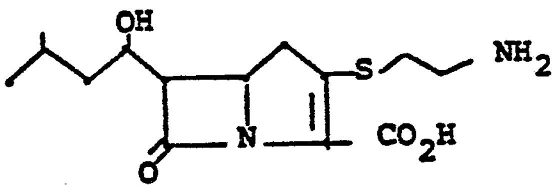

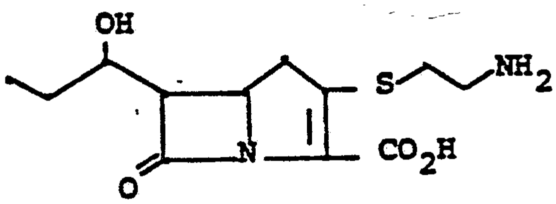

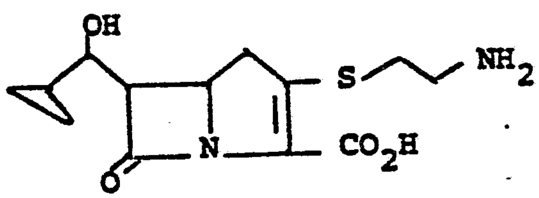

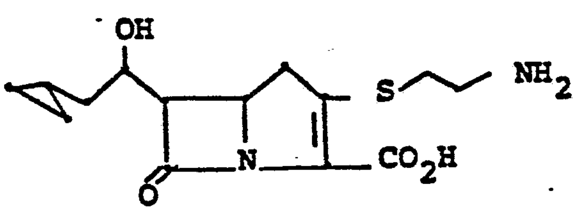

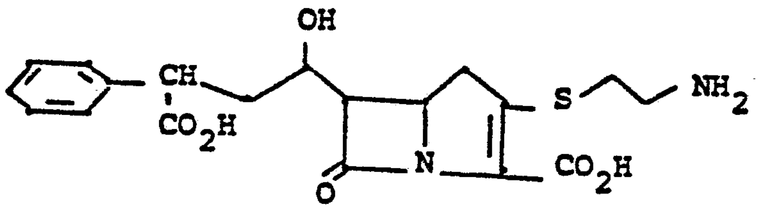

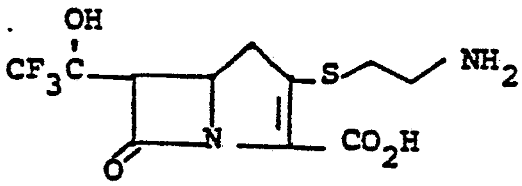

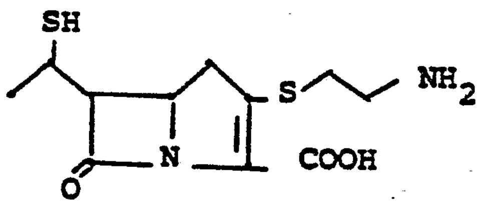

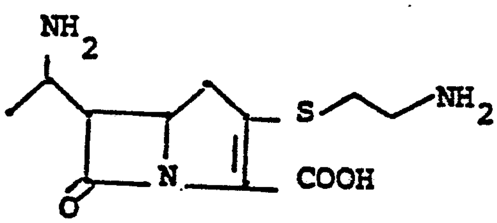

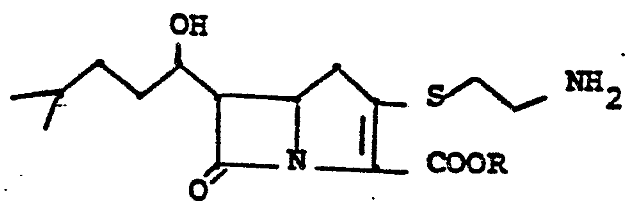

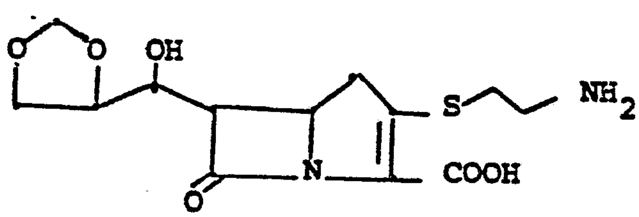

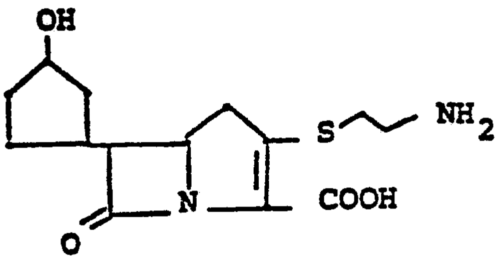

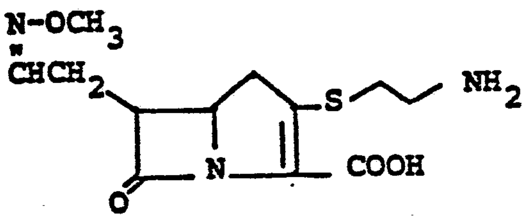

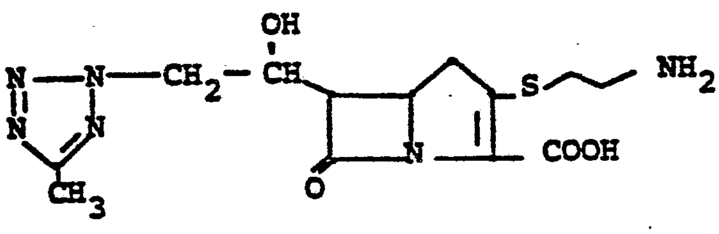

0038869

European Patent Office

# EUROPEAN SEARCH REPORT

Application number EP 80 10 2338

| DOCUMENTS CONSIDERED TO BE RELEVANT | | | CLASSIFICATION OF THE APPLICATION (Int. Cl.3) |
|---|---|---|---|
| Category | Citation of document with indication, where appropriate, of relevant passages | Relevant to claim | |
| | EP - A - 0 007 973 (MERCK)<br>* Claims 1-6 *<br>-- | 1-3 | C 07 D 487/04//<br>C 07 D 205/08<br>C 07 F 7/10<br>C 07 C 125/065<br>(C 07 D 487/04<br>209/00<br>205/00) |
| E | EP - A - 0 017 970 (SANRAKU-OCEAN)<br>* Claims *<br>---- | 1-19 | |
| | | | TECHNICAL FIELDS SEARCHED (Int. Cl.3) |
| | | | C 07 D 487/04 |
| | | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| | The present search report has been drawn up for all claims | | &: member of the same patent family, corresponding document |

| Place of search | Date of completion of the search | Examiner |
|---|---|---|
| The Hague | 13-11-1980 | CHOULY |